# EUROPEAN PATENT APPLICATION

(11) **EP 3 543 349 A1**
(43) Date of publication of application: **25.09.2019**
(21) Application number: 17872098.3
(22) Date of filing: 13.11.2017
(51) Int. Cl.: C12Q 1/37, C12Q 1/02, G01N 33/15, G01N 33/50

(54) **MOLECULAR KINETICS EVALUATION METHOD AND SCREENING METHOD**

(30) Priority: 15.11.2016 JP 2016222682
(71) Applicant: Tokyo University of Science Foundation, Tokyo 162-8601 (JP)
(72) Inventor: MIYAMOTO Etsuko, Tokyo 162-8601 (JP); OZAWA Masaaki, Tokyo 162-8601 (JP)
(74) Representative: Herzog IP Patentanwalts GmbH
(86) International application number: PCT/JP2017/040780
(87) International publication number: WO 2018/092724

(57) **Abstract**

A molecular kinetics evaluation method is provided which involves: a step for dosing a human or non-human animal with a proteolysis-inducing molecule, which is a conjugate of a proteolysis induction tag, i.e., a molecule which has affinity for protease and which does not inhibit proteolysis by protease, and a specific protein affinity molecule having affinity to a specific protein, and inducing proteolysis of the specific protein in vivo in the human or non-human animal; and a step for evaluating the molecular kinetics of the specific protein affinity molecules or the proteolysis-inducing molecules by detecting proteolysis of the specific proteins in a sample which is at least part of the human or non-human animal.

SEQUENCE LISTING

<110>  TOKYO UNIVERSITY OF SCIENCE FOUNDATION

<120>  EVALUATION METHOD OF MOLECULAR DYNAMICS AND SCREENING METHOD

<130>  RKF-050PCT

<150>  JP 2016-222682

<151>  2016-11-15

<160>  2

<210>  1
<211>  49
<212>  DNA
<213>  Artificial Sequence

<220>
<223>  Forward primer for RF cloning

<400>  1
cacgatgata atatggccac aaccatgact gaatataaac ttgtggtag                49


<210>  2
<211>  47
<212>  DNA
<213>  Artificial Sequence

<220>
<223>  Reverse primer for RF cloning

<400>  2
gaacgtcgta cgggtaatcg atcataatta cacactttgt ctttgac                  47

## Description

### TECHNICAL FIELD

The present disclosure relates to a molecular kinetics evaluation method and a screening method.

### BACKGROUND ART

In research and development of drugs, it is essential to evaluate the pharmacokinetics of a test substance (test molecule) (hereinafter, also referred to as "molecular kinetics"), such as the specificity of the test substance to tissues, organs, cells, molecules (including a complex of molecules), and the like. Evaluating the molecular kinetics of a test substance is also useful, for example, when screening for a test substance that is highly specific to a target tissue, organ, cell, molecule, etc.

Conventionally known methods for evaluating molecular kinetics of a test material include a method of analyzing a test material that has been administered to a human or a non-human animal and transferred to each tissue, organ, cell, etc., by high performance liquid chromatograph (HPLC), liquid chromatography-tandem mass spectrometer (LC-MS/MS), and the like; a method of analyzing a test material labeled with a radioisotope that is administered to a human or a non-human animal, and transferred to each tissue, organ, cell, or the like, by, for example, autoradiography; and the like (see, for example, Patent Document 1).

Patent Document 1: Japanese Unexamined Patent Application, Publication No. 2011-22002

### DISCLOSURE OF THE INVENTION

### Problems to be Solved by the Invention

However, analysis methods using HPLC, LC-MS/MS, and the like, are complicated in setting analysis conditions, and an analysis method using autoradiography or the like needs to use a radioisotope, hence all of them were not convenient methods.

In view of the above circumstances, an object of the present disclosure is to provide a new molecular kinetics evaluation method for evaluating molecular kinetics of a test material, and a new screening method for selecting a test material showing specific molecular kinetics.

### Means for Solving the Problems

Specific means for achieving the above object include the following embodiments.
<1> A molecular kinetics evaluation method including:
   a step of administering a protein-degradation inducing molecule to a human or a non-human animal, the protein-degradation inducing molecule being a conjugate of a protein-degradation inducing tag that is a molecule that has an affinity with a protease and does not inhibit degradation of a protein by the protease and a specific protein affinity molecule that has an affinity with a specific protein, and inducing degradation of the specific protein in a living body of the human or the non-human animal; and
   a step of evaluating molecular kinetics of the specific protein affinity molecule or the protein-degradation inducing molecule by detecting degradation of the specific protein in a specimen being at least a portion of the human or the non-human animal.
<2> The molecular kinetics evaluation method according to <1>, in which in the step of inducing degradation of the specific protein, the degradation of the specific protein is induced in a ubiquitin-independent manner.
<3> The molecular kinetics evaluation method according to <1> or <2>, in which the protein-degradation inducing tag has a structure where a protease inhibitory activity of a protease inhibitor is inactivated.
<4> The molecular kinetics evaluation method according to any one of <1> to <3>, in which the protease is a proteasome.
<5> The molecular kinetics evaluation method according to <4>, in which the protein-degradation inducing tag has a structure where a proteasome inhibitory activity of a proteasome inhibitor is inactivated.
<6> The molecular kinetics evaluation method according to <5>, in which the proteasome inhibitory activity is an inhibitory activity against at least one selected from a caspase-like activity, a trypsin-like activity, and a chymotrypsin-like activity.
<7> The molecular kinetics evaluation method according to any one of <1> to <6>, in which the protein-degradation inducing molecule is a drug candidate molecule, and the method further includes a step of evaluating a pharmacological action by inducing the degradation of the specific protein in a living body of the human or the non-human animal.
<8> The molecular kinetics evaluation method according to any one of <1> to <7>, in which the step of inducing degradation of the specific protein includes administering the protein-degradation inducing molecule to a non-human animal, and inducing degradation of the specific protein in a living body of the non-human animal, and the step of evaluating molecular kinetics of the specific protein affinity molecule or the protein-degradation inducing molecule includes detecting degradation of the specific protein in a specimen being at least a portion of the non-human animal.
<9> The molecular kinetics evaluation method according to any one of <1> to <8>, in which the molecular kinetics is specificity to a tissue, an organ, a cell, or a molecule.
<10> A screening method including:
   a step of administering a protein-degradation inducing molecule to a human or a non-human animal, the protein-degradation inducing molecule being a conjugate of a protein-degradation inducing tag that is a molecule which has an affinity with a protease and does not inhibit degradation of a protein by the protease, and a specific protein affinity molecule which has an affinity with a specific protein, and inducing degradation of the specific protein in a living body of the human or the non-human animal; and
   a step of selecting the specific protein affinity molecule or the protein-degradation inducing molecule showing specific molecular kinetics by detecting degradation of the specific protein in a specimen being at least a portion of the human or the non-human animal.
<11> The screening method according to <10>, in which in the step of inducing degradation of the specific protein, the degradation of the specific protein is induced in a ubiquitin-independent manner.
<12> The screening method according to <10> or <11>, in which the step of inducing degradation of the specific protein includes administering the protein-degradation inducing molecule to a non-human animal and inducing degradation of the specific protein in a living body of the non-human animal, and the step of selecting the specific protein affinity molecule or the protein-degradation inducing molecule includes detecting degradation of the specific protein in a specimen being at least a portion of the non-human animal.
<13> The screening method according to any one of <10> to <12>, in which the molecular kinetics is specificity to a tissue, an organ, a cell, or a molecule.

### Effects of the Invention

The present disclosure can provide a new molecular kinetics evaluation method for evaluating molecular kinetics of a test material, and a new screening method for selecting a test material showing specific molecular kinetics.

### BRIEF DESCRIPTION OF THE DRAWINGS

Fig. 1 shows the results of evaluation by FACS (Fluorescence Activated Cell Sorting) analysis of degradation (knockdown) of a wild-type K-Ras protein forcibly expressed in HeLa cells through TUS-007.
Fig. 2 shows the results of evaluation by Western blot analysis of degradation (knockdown) of a wild-type K-Ras protein forcibly expressed in HeLa cells through TUS-007.
Fig. 3 shows the results of evaluation by Western blot analysis of degradation (knockdown) of an endogenous wild-type K-Ras protein and wild-type H-Ras protein in HeLa cells to which TUS-007 was added.
Fig. 4 shows the results of evaluation by Western blot analysis of degradation (knockdown) of the wild type K-Ras protein in each tissue of a mouse when TUS-007 was administered to a mouse individual.
Fig. 5A shows inhibitory activity of TMP-CANDDY_DMT and MG-132 with respect to a catalytic subunit β1 of a proteasome.
Fig. 5B shows inhibitory activity of TMP-CANDDY_DMT and MG-132 with respect to a catalytic subunit β2 of the proteasome.
Fig. 5C shows inhibitory activity of TMP-CANDDY_DMT, and MG-132 with respect to a catalytic subunit β5 of the proteasome.
Fig. 6 shows the results of evaluation by FACS analysis of degradation (knockdown) of an ecDHFR protein forcibly expressed in HeLa cells through TMP-CANDDY_DMT.
Fig. 7A shows the results of evaluation by Western blot analysis of degradation (knockdown) of an ecDHFR protein forcibly expressed in HeLa cells through TMP-CANDDY_DMT.
Fig. 7B shows the results of evaluation by Western blot analysis of degradation (knockdown) of an ecDHFR protein forcibly expressed in HeLa cells through TMP-CANDDY_DMT.
Fig. 8A shows the results of evaluation by Western blot analysis of degradation (knockdown) of an endogenous DHFR protein in HeLa cells to which MTX-CANDDY_MLN was added.
Fig. 8B shows the results of evaluation by Western blot analysis of degradation (knockdown) of an endogenous DHFR protein in HeLa cells to which MTX-CANDDY_MLN was added.
Fig. 9 shows the results of evaluation by Western blot analysis of degradation (knockdown) of the DHFR protein in each tissue of a mouse after MTX-CANDDY_MLN was administered to a mouse individual.
Fig. 10 shows the results of evaluation by Western blot analysis of degradation (knockdown) of an endogenous wild-type p53 protein and MDM2 protein in HCT116 cells to which TIBC-CANDDY_MLN was added.
Fig. 11 shows the results of evaluation by Western blot analysis of degradation (knockdown) of an endogenous wild-type p53 protein in HeLa cells to which TIBC-CANDDY_MLN was added.
Fig. 12 shows the results of evaluation by Western blot analysis of degradation (knockdown) of the wild-type p53 protein in each tissue of a mouse after TIBC-CANDDY_MLN was administered to a mouse individual.
Fig. 13 shows the results of evaluation by Western blot analysis of degradation (knockdown) of the MDM2 protein in each tissue of a mouse after TIBC-CANDDY_MLN was administered to a mouse individual.
Fig. 14A shows inhibitory activity of TMP-CANDDY_ALLN and ALLN with respect to a catalytic subunit β1 of a proteasome.
Fig. 14B shows inhibitory activity of TMP-CANDDY_ALLN and ALLN with respect to a catalytic subunit β2 of the proteasome.
Fig. 14C shows inhibitory activity of TMP-CANDDY_ALLN and ALLN with respect to a catalytic subunit β5 of the proteasome.
Fig. 15 shows the results of evaluation by FACS analysis of degradation (knockdown) of an ecDHFR protein forcibly expressed in HeLa cells through TMP-CANDDY_ALLN.

### PREFERRED MODE FOR CARRYING OUT THE INVENTION

Below, the embodiments of the present invention will be described in detail. However, the present invention shall not be limited to the following embodiments.

A range of numerical values specified using "-" as used herein refers to a range including values indicated before and after "-" as the minimum value and the maximum value, respectively. The term "step" as used herein encompasses a step independent from the other steps as well as a step which cannot be clearly separated from the other steps as long as the purpose of that step can be achieved. Amino acids as used herein are denoted by the single letter notation (for example, "G" for glycine) or the three-letter notation (for example, "Gly" for glycine) as is well known in the art.

### <Protein-degradation inducing molecule>

A protein-degradation inducing molecule of the present disclosure is a conjugate of a protein-degradation inducing tag that is a molecule which has an affinity with a protease and does not inhibit degradation of a protein by the protease, and a specific protein affinity molecule which has an affinity with a specific protein. When this protein-degradation inducing molecule is administered to a human or a non-human animal, the specific protein can be led to degradation (knockdown) by a protease (for example, a proteasome) in a living body of the human or the non-human animal, without ubiquitination of the specific protein (that is, in a ubiquitin-independent manner).

It is noted that a polyubiquitin chain such as a tetraubiquitin chain (Ub₄) or a ubiquitin-like domain (UbL) is likely to function as a protein-degradation inducing tag. However, when a polyubiquitin chain or a ubiquitin-like domain is a protein-degradation inducing tag, the specific protein is indirectly ubiquitinated via the specific protein affinity molecule. In the present specification, such an indirect ubiquitination of the specific protein is also included in the ubiquitination of the specific protein.

In the molecular kinetics evaluation method and screening method described below in the present disclosure, as an indicator for degradation of the specific protein, the molecular kinetics of a test material (a specific protein affinity molecule or a protein-degradation inducing molecule) can be evaluated, and a test material (specific protein affinity molecule or a protein-degradation inducing molecule) showing specific molecular kinetics can be selected.

### (Protein-degradation inducing tag)

The protein-degradation inducing tag is a molecule having an affinity with a protease and that does not inhibit degradation of a protein by the protease. Below, the above protein-degradation inducing tag may also be referred to as a CiKD (Chemical interaction and KnockDown) tag or CANDDY (Chemical AffiNities and Degradation Dynamics) tag.

There is no particular limitation for the protease, and any molecule having a protease activity can be used. For example, it may be a protease complex such as a proteasome, or may be a protease other than the proteasome. Alternatively, it may be a portion of a proteasome as long as the portion has a protease activity.

Examples of the proteasome include 26S proteasome, an immunoproteasome, and a thymus proteasome.
26S proteasome is composed of 20S proteasome and two units of 19S proteasome, the two units of 19S proteasome being attached to the 20S proteasome.
20S proteasome has a cylindrical structure in which an α-ring consisting of 7 subunits of α1 to α7 and a β-ring consisting of 7 subunits of β1 to β7 are stacked in order of αββα, and β1, β2, and β5 show catalytic activities of a caspase-like activity, a trypsin-like activity, and a chymotrypsin-like activity, respectively. In the immunoproteasome, the catalytic subunits β1, β2, and β5 are replaced with β1i, β2i, and β5i, respectively (Science, 1994, 265, 1234-1237). In the thymus proteasome, β5t which is expressed specifically in cortical thymic epithelial cells (cTEC) is incorporated along with β1i and β2i (Science, 2007, 316, 1349-1353).

Examples of a protease other than the proteasome include β-secretase, γ-secretase, aminopeptidase, angiotensin-converting enzyme, bromelain, calpine I, calpine II, carboxypeptidase A, carboxypeptidase B, carboxypeptidase P, carboxypeptidase Y, caspase 1, caspase 2, caspase 3, caspase 5, caspase 6, caspase 7, caspase 8, caspase 9, caspase 13, cathepsin B, cathepsin C, cathepsin D, cathepsin G, cathepsin L, chymotrypsin, clostripain, collagenase, complement C1r, complement C1s, complement factor B, complement factor D, dipeptidyl peptidase I, dipeptidyl peptidase II, dipeptidyl peptidase IV, dispase, elastase, endoproteinase Arg-C, endoproteinase Glu-C, endoproteinase Lys-C, ficin, granzyme B, kallikrein, leucine aminopeptidase, matrix metalloprotease, metalloprotease, papain, pepsin, plasmin, procaspase 3, pronase E, proteinase K, renin, thermolysin, thrombin, trypsin, cytosol alanyl aminopeptidase, enkephalinase, neprilysin, and the like.

As used herein, the phrase "having an affinity with a protease" means the capability of binding to a protease, for example, via a covalent bond, a hydrogen bond, a hydrophobic bond, Van der Waals force, and the like. When the thermal stability of a protease changes in the presence of a certain molecule, the molecule can be determined as having an affinity with that protease.

As used herein, the phrase "without inhibiting degradation of a protein by a protease" means that, for example, the protein-degradation inducing tag does not bind to the degradation active site of the protease via a covalent bonding. When a protein is degraded by a protease in the presence of a certain molecule, and the degradation of the protein is inhibited in the presence of aprotease inhibitor, the molecule can be considered not to inhibit the degradation of the protein by the protease.

Examples of the protein-degradation inducing tag include low molecular weight compounds, natural products, peptides, antibodies, and the like. It is noted that in the present disclosure, the antibody includes a fragment including a variable site of the antibody, for example, a Fab fragment or a F(ab') fragment of Ig (immunoglobulin), in addition to an Ig having two H-chains and two L-chains. The protein-degradation inducing tag preferably has a molecular weight within the range of, for example, 50 to 200000. When the protein-degradation inducing tag is a low molecular weight compound, the molecular weight of the protein-degradation inducing tag is preferably within the range of, for example, 50 to 5000.

There is no particular limitation for the structure of the protein-degradation inducing tag as long as the protein-degradation inducing tag has an affinity with a protease without inhibiting degradation of a protein by the protease. The protein-degradation inducing tag can be obtained by, for example, screening from the candidate molecules. Furthermore, the protein-degradation inducing tag can be produced by inactivating the protease inhibitory activity (for example, proteasome inhibitory activity) of a protease inhibitor (for example, a proteasome inhibitor).

In a certain embodiment, for example, the protein-degradation inducing tag may have a structure represented by the following formula (I). It is demonstrated that the compound represented by the following formula (I) has an affinity with a protease, and does not inhibit the degradation of a protein by the protease (see, for example, the below-mentioned Reference Examples 4 to 6).

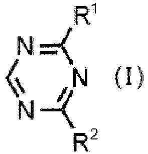

In the formula (I), R¹ and R² each independently represent a hydrocarbon group having 1 to 20 carbon atoms, an alkoxy group having 1 to 20 carbon atoms, an aryloxy group having 6 to 20 carbon atoms, a hydroxy group, a carboxy group, an amino group, or a halogeno group.

Examples of the hydrocarbon group include an alkyl group, an alkenyl group, an aryl group, combinations thereof, and the like. Specific examples include an alkyl group having 1 to 20 carbon atoms such as a methyl group and an ethyl group; an alkenyl group having 2 to 20 carbon atoms such as a vinyl group and an allyl group; an aryl group having 6 to 20 carbon atoms such as a phenyl group and a naphthyl group; an arylalkyl group having 7 to 20 carbon atoms such as a benzyl group and a phenethyl group; an alkylaryl group having 7 to 20 carbon atoms such as a tolyl group and a xylyl group; and the like. Examples of the halogeno group include a fluoro group, a chloro group, a bromo group, and the like.

In another embodiment, the protein-degradation inducing tag may have a structure in which the proteasome inhibitory activity of a proteasome inhibitor is inactivated. More specifically, at least one inhibitory activity selected from a caspase-like activity, a trypsin-like activity, and a chymotrypsin-like activity can be mentioned as the proteasome inhibitory activity.

The term "structure in which a proteasome inhibitory activity is inactivated" as used herein encompasses a structure in which a proteasome inhibitory activity is attenuated in addition to a structure in which a proteasome inhibitory activity is completely eliminated. In a certain embodiment, the protein-degradation inducing tag has a 50% inhibition concentration (IC₅₀) against at least one selected from a caspase-like activity, a trypsin-like activity, and a chymotrypsin-like activity which is 2 times or more of the 50% inhibition concentration (IC₅₀) of the original proteasome inhibitor.

As the proteasome inhibitor, any compound having a proteasome inhibitory activity can be used. A proteasome inhibitor is a compound which has an affinity with a proteasome (a protease complex), and inhibits degradation of a protein by a proteasome. Therefore, a protein-degradation inducing tag may be obtained by replacing the active site of a proteasome inhibitor with another structural moiety to inactivate the proteasome inhibitory activity. Proteasome inhibitors are being studied as anticancer agents, and there are many compounds that have been approved as pharmaceutical products, or are under clinical trials. Moreover, many of proteasome inhibitors have relatively small molecular weights and low hydrophobicity, and are less problematic in terms of cell membrane permeability, cytotoxicity, and the like. For these reasons, synthesizing a protein-degradation inducing tag based on a proteasome inhibitor is quite reasonable and efficient.

Examples of the proteasome inhibitor are shown in the following Tables 1 and 2. The proteasome inhibitors shown in Tables 1 and 2 are each a 20S proteasome inhibitor having an affinity with the active center part of 20S proteasome. Furthermore, the proteasome inhibitors shown in Tables 1 and 2 naturally have affinity with 26S proteasome. However, a proteasome inhibitor which can be used for producing a protein-degradation inducing tag shall not be limited to these examples.

**[Table 1]**

| No. | Generic name / Product name | Structural formula (Circles indicate active sites) | Molecular weight |
|---|---|---|---|
| 1 | Bortezomib | 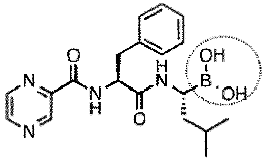 | 384.24 |
| 2 | ALLN (MG-101, Calpain inhibitor I | 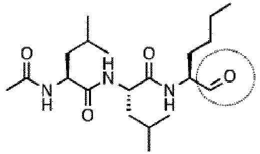 | 383.53 |
| 3 | MLN9708 (Ixazomib) | 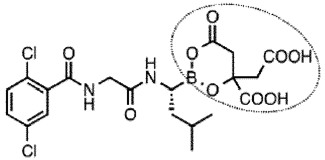 | 517.12 |
| 4 | MLN2238 | 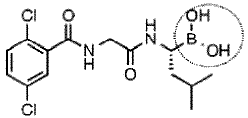 | 361.03 |
| 5 | CEP-18770 | 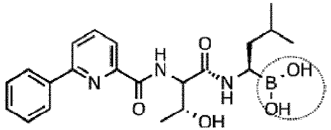 | 413.28 |
| 6 | ONO-7058 (Oprozomib) | 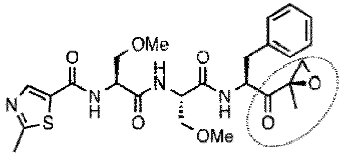 | 532.61 |
| 7 | MG-132 | 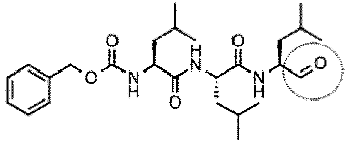 | 475.63 |

**[Table 2]**

| No. | Generic name / Product name | Structural formula (Circles indicate active sites) | Molecular weight |
|---|---|---|---|
| 8 | Carfilzomib | 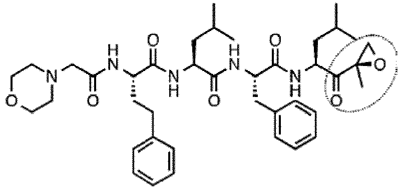 | 719.92 |
| 9 | BSc-2118 | 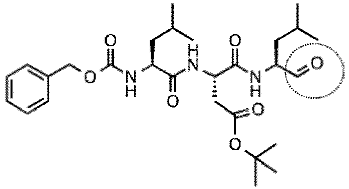 | 533.66 |
| 10 | PSI | 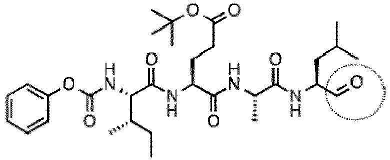 | 604.75 |
| 11 | Epoxomicin | 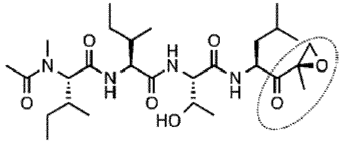 | 554.73 |
| 12 | ONX-0914 | 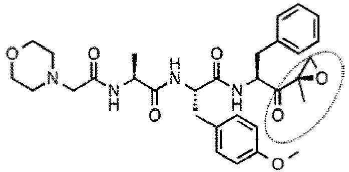 | 580.68 |
| 13 | ¹²⁵I-NIP-L₃VS | 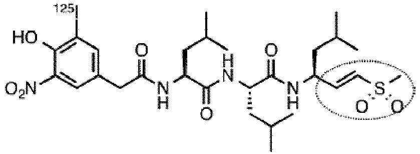 | 720.64 |
| 14 | NPI-0052 (Marizomib) | 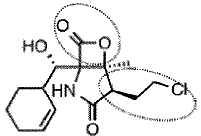 | 313.78 |

For example, bortezomib as a boronic acid-based proteasome inhibitor is known to inhibit a proteasome activity when the boronyl group as an active site covalently binds to the degradation active site of 20S proteasome as shown in the following scheme (Kisselev, A.F. et al., Chemistry & Biology, 2012, 19, 99-115).

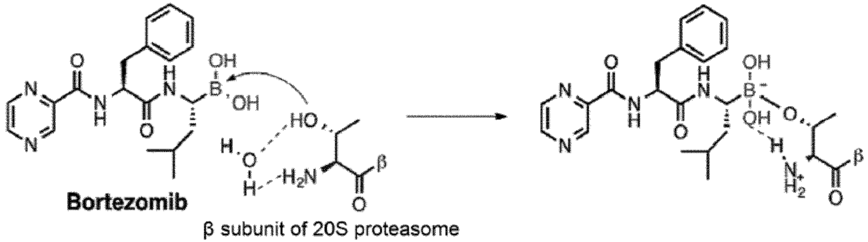

Further, MLN9708 and MLN2238, which are boronic acid-based proteasome inhibitors, are known to inhibit a proteasome activity when the boronic acid ester moiety or the boronyl group as an active site covalently binds to the degradation active site of 20S proteasome as shown in the following scheme (Kisselev, A.F. et al., Chemistry & Biology, 2012, 19, 99-115) .

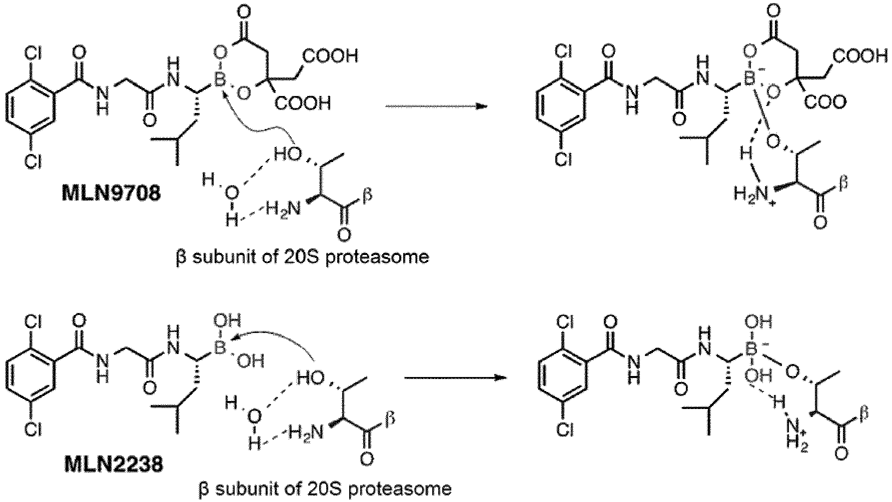

Therefore, a protein-degradation inducing tag may be obtained by replacing the boronyl group or the boronic acid ester moiety as the active sites of bortezomib, MLN9708, and MLN2238 with another structural moiety (a carboxy group, an alkyl group, an aryl group, an amino group, a hydroxy group, and the like) to inactivate the proteasome inhibitory activity.

It is noted that even for other boronic acid-based proteasome inhibitors such as CEP-18770, a protein-degradation inducing tag can be obtained by replacing the active site with another structural moiety (a carboxy group, an alkyl group, an aryl group, an amino group, a hydroxy group, and the like).

Further, ALLN, which is an aldehyde-based proteasome inhibitor, is known to inhibit a proteasome activity when the formyl group as an active site covalently binds to the degradation activity site of 20S proteasome as shown in the following scheme (Kisselev, A.F. et al., Chemistry & Biology, 2012, 19, 99-115).

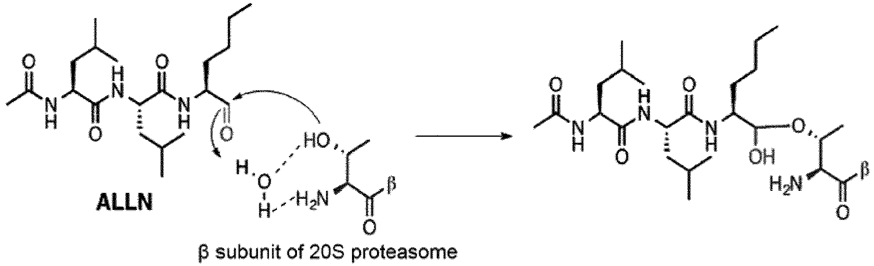

Therefore, a protein-degradation inducing tag can be obtained by replacing the formyl group as the active site of ALLN with another structural moiety (a carboxy group, an alkyl group, an aryl group, an amino group, a hydroxy group, and the like) to inactivate the proteasome inhibitory activity.

It is noted that even for other aldehyde-based proteasome inhibitors such as MG-132, BSc-2118, and PSI, a protein-degradation inducing tag can be obtained by replacing the formyl group as an active site with another structural moiety (a carboxy group, an alkyl group, an aryl group, an amino group, a hydroxy group, and the like).

Examples of the protein-degradation inducing tag having a structure in which the proteasome inhibitory activity of a proteasome inhibitor is inactivated are shown in the following Tables 3 and 4. Examples of the monovalent group represented by R in the tables include a carboxy group, an alkyl group having 1 to 20 carbon atoms, an aryl group having 6 to 20 atoms, an amino group, a hydroxy group, and the like.

**[Table 3]**

| No. | Structural formula | |
|---|---|---|
| 1 | 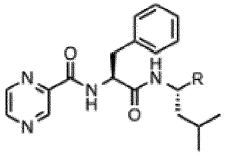 | (In the formula, R represents a monovalent group except for |
| | | 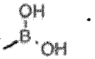 |
| | | ) |
| 2 | 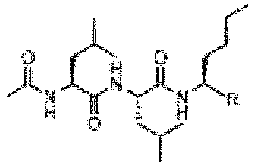 | (In the formula, R represents a monovalent group except for -CHO.) |
| 3 | 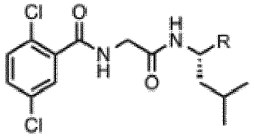 | (In the formula, R represents a monovalent group except for |
| | | 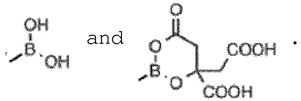 ) |
| 4 | 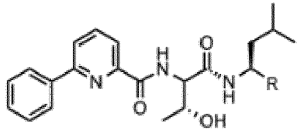 | (In the formula, R represents a monovalent group except for |
| | | 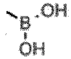 |
| | | .) |
| 5 | 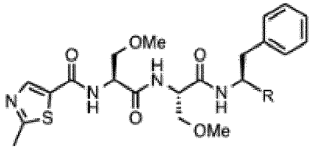 | (In the formula, R represents a monovalent group except for |
| | | 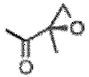 |
| | | .) |
| 6 | 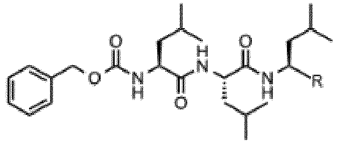 | (In the formula, R represents a monovalent group except for -CHO.) |
| 7 | 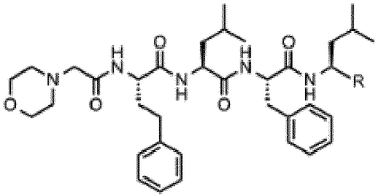 | (In the formula, R represents a monovalent group except for |
| | | 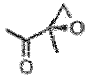 |
| | | .) |

**[Table 4]**

| No. | Structural formula | |
|---|---|---|
| 8 | 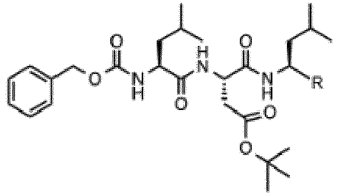 | (In the formula, R represents a monovalent group except for -CHO.) |
| 9 | 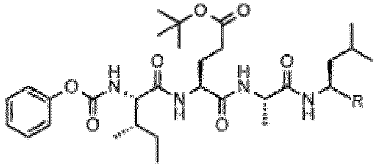 | (In the formula, R represents a monovalent group except for -CHO.) |
| 10 | 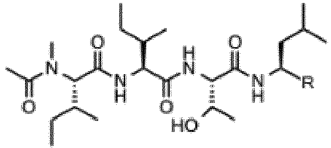 | (In the formula, R represents a monovalent group except for |
| | | 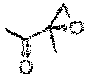 |
| | | .) |
| 11 | 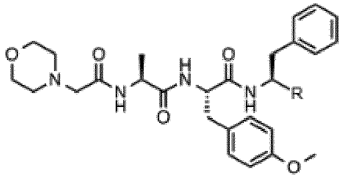 | (In the formula, R represents a monovalent group except for |
| | | 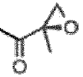 |
| | | .) |
| 12 | 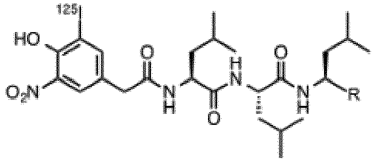 | (In the formula, R represents a monovalent group except for |
| | | 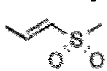 |
| | | .) |
| 13 | 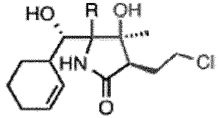 | (In the formula, R represents a monovalent group.) |
| 14 | 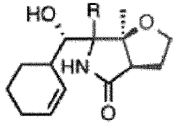 | (In the formula, R represents a monovalent group.) |

Other examples of the proteasome inhibitor are shown in the following Tables 5 to 10. Even for these proteasome inhibitors, a protein-degradation inducing tag can be obtained by inactivating the proteasome inhibitory activity in a similar way as described above.

**[Table 5]**

| **20S proteasome inhibitor** | | | |
|---|---|---|---|
| No. | Generic name / Product name | Structural formula | Molecular weight |
| 15 | Aspirin | 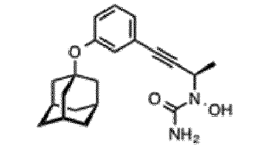 | 180.15 |
| 16 | Hydroxyurea inhibitor | 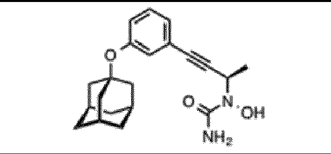 | 354.54 |
| 17 | PI-1840 | 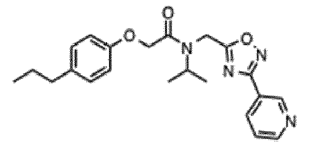 | 394.47 |
| 18 | PI-083 | 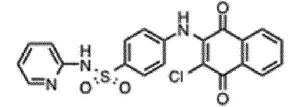 | 439.87 |
| 19 | Cerastol | 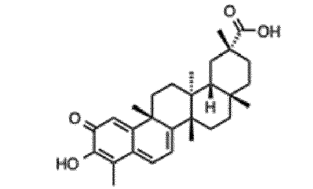 | 450.61 |

**[Table 6]**

| **20S proteasome inhibitor** (Continued) | | | |
|---|---|---|---|
| No. | Generic name / Product name | Structural formula | Molecular weight |
| 20 | CVT-659 | 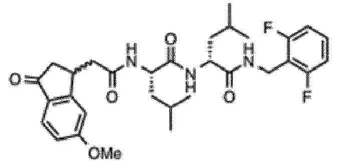 | 571.66 |
| 21 | Capped dipeptide 2 | 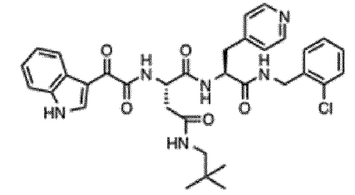 | 645.15 |
| 22 | TMC95A | 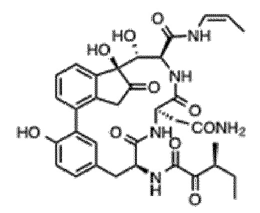 | 677.71 |
| 23 | Capped dipeptide 1 | 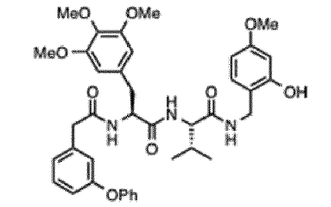 | 699.80 |

**[Table 7]**

| **20S proteasome inhibitor (Continued)** | | | |
|---|---|---|---|
| No. | Generic name / Product name | Structural formula | Molecular weight |
| 24 | Ritonavir | 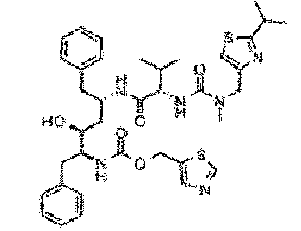 | 720.94 |
| 25 | Scytonemide A | 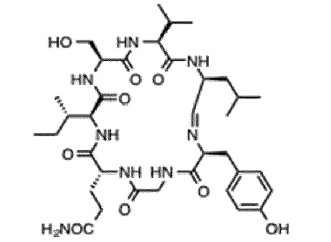 | 744.89 |
| 26 | Argyrin A | 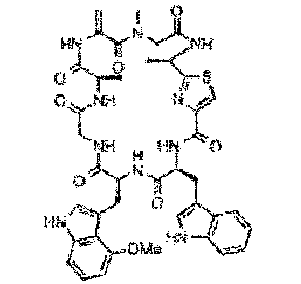 | 824.91 |
| 27 | Benzylstatine peptide 1 | 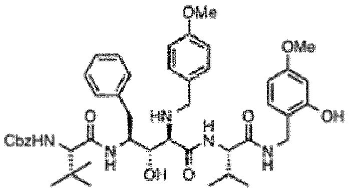 | 826.00 |

**[Table 8]**

| **19S proteasome inhibitor** | | | |
|---|---|---|---|
| No. | Generic name / Product name | Structural formula | Molecular weight |
| 1 | RIP-1 (Rpt4 inhibitor) | 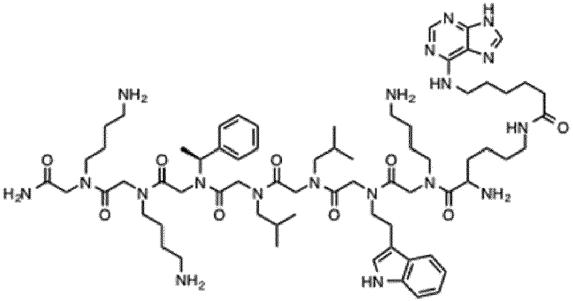 | 1348.76 |

**[Table 9]**

| **Inhibitor for a constituent factor other than 20S/19S** | | | | |
|---|---|---|---|---|
| No. | Generic name / Product name | Structural formula | Molecular weight | Others |
| 1 | JBIR-22 | 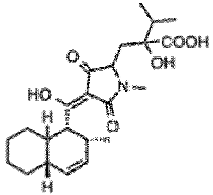 | 419.52 | PAC-3 (molecule assembly factor) inhibition) |

**[Table 10]**

| 20S immunoproteasome inhibitor | | | | |
|---|---|---|---|---|
| No. | Generic name / Product name | Structural formula | Molecular weight | Others |
| 1 | PR-957 | 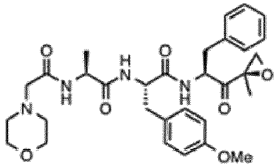 | 580.68 | β5i is inhibited |
| 2 | IPSI-001 | 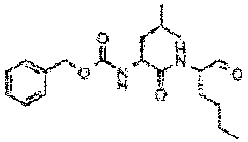 | 362.47 | β2i is inhibited |
| 3 | LMP2-sp-ek | 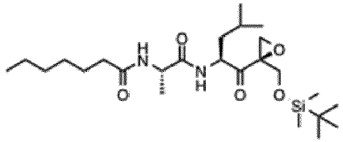 | 484.75 | β2i is inhibited |

In another embodiment, the protein-degradation inducing tag may have a structure in which the protease inhibitory activity of a protease inhibitor (except for the proteasome inhibitors described above) is inactivated.

The term "structure in which a protease inhibitory activity is inactivated" as used herein encompasses a structure in which the protease inhibitory activity is attenuated in addition to a structure in which the protease inhibitory activity is completely eliminated. In a certain embodiment, the protein-degradation inducing tag has a 50% inhibition concentration (IC₅₀) against a protease as an inhibition target of a protease inhibitor which is 2 times or more of the 50% inhibition concentration (IC₅₀) of the original protease inhibitor.

As a protease inhibitor, any compound having a protease inhibitory activity can be used. The protease inhibitor is a compound having an affinity with a protease and inhibiting degradation of a protein by the protease. Therefore, a protein-degradation inducing tag can be obtained by replacing the active site of a protease inhibitor with another structural moiety to inactivate the protease inhibitory activity.

Examples of the protease inhibitor are shown in the following Tables 11 to 78. Protein-degradation inducing tags can be obtained by replacing the active sites of these protease inhibitors with other structural moieties to inactivate the protease inhibitory activities. However, a protease inhibitor which can be used for producing protein-degradation inducing tags shall not be limited to these examples. Existing data bases ("MEROPS-the peptidase database" (http://merops.sanger.ac.uk/index.shtml), and the like) can be consulted for information about proteases and protease inhibitors if needed.

**[Table 11]**

| β-secretase inhibitor | | | | |
|---|---|---|---|---|
| No. | Name | Structural formula | Molecul ar weiqht | Protease to be inhibited |
| 1 | OM99-2 | 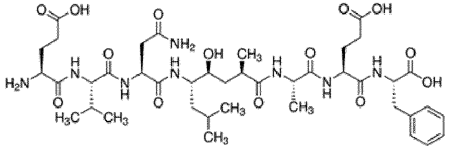 | 892.99 | |

**[Table 12]**

| γ-secretase inhibitor | | | | |
|---|---|---|---|---|
| No. | Name | Structural formula | Molecul ar weiqht | Protease to be inhibited |
| 1 | γ⁻ Secretase inhibitor | 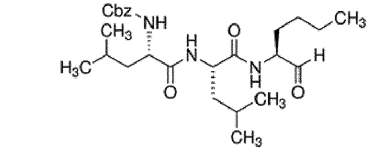 | 705.83 | |
| 2 | L-685,458 | 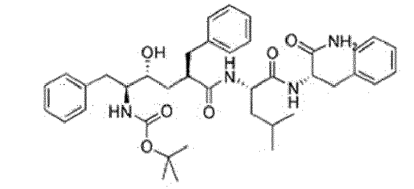 | 672.85 | |

**[Table 13]**

| **Aminopeptidase inhibitor** | | | | |
|---|---|---|---|---|
| No. | Name | Structural formula | Molecul ar weiqht | Protease to be inhibited |
| 1 | Cysteamine | 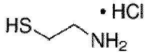 | 113.61 | |
| 2 | Bestatin | 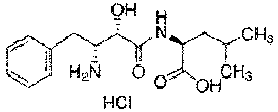 | 344.83 | ▪Aminopeptidase B |
| | | | | ▪Leucine aminopeptidase |

**[Table 14]**

| **Angiotensin** converting enzyme inhibitor | | | | |
|---|---|---|---|---|
| No. | Name | Structural formula | Molecul ar weight | Protease to be inhibited |
| 1 | Captopril | 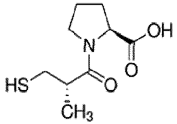 | 217.29 | ▪Formation of angiotensin II is inhibited |
| 2 | Fenoldopam monohydrob romide | 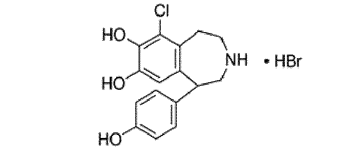 | 386.67 | |
| 3 | Angiotensi n Converting Enzyme Inhibitor | 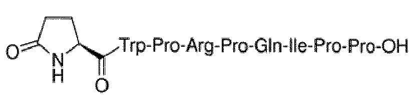 | 1101.26 | |

**[Table 15]**

| **Bromelain inhibitor** | | | | |
|---|---|---|---|---|
| No. | Name | Structural formula | Molecul ar weiqht | Protease to be inhibited |
| 1 | E-64 | 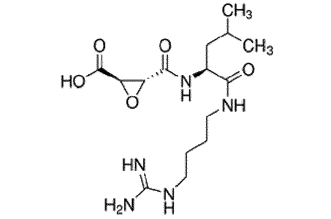 | 357.41 | ▪Cathepsin B |
| | | | | ▪Ficin |
| | | | | ▪Papain |
| | | | | ▪Bromelain |
| 2 | N-Ethylmalei mide | 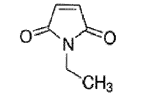 | 125.13 | ▪Calpine |
| | | | | ▪Ficin |
| 3 | N-p-Tosyl-L-phenilalan ine chlorometh yl ketone | 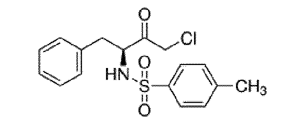 | 351.85 | ▪Papain |
| | | | | ▪Chymotrypsin |
| | | | | ▪Ficin |
| | | | | ▪Bromelain |
| 4 | Sodium iodoacetat e | 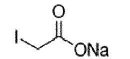 | 207.93 | ▪Carboxypeptidase P |
| | | | | ▪Bromelain |
| | | | | ▪Ficin |
| | | | | ▪Cathepsin |

**[Table 16]**

| **Calpain** inhibitor | | | | |
|---|---|---|---|---|
| No. | Name | Structural formula | Molecul ar weiqht | Protease to be inhibited |
| 1 | E-64c | 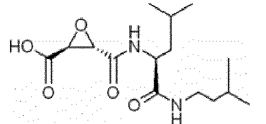 | 314.38 | |
| 2 | E-64d | 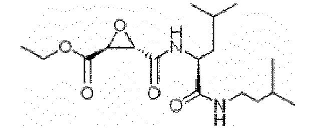 | 342.43 | |
| 3 | Z-Leu-Leu-Leu-fluorometh yl ketone | 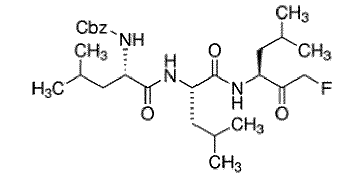 | 507.64 | |
| 4 | N-Ethylmalei mide | 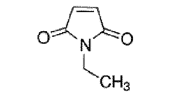 | 125.13 | ▪Ficin ▪Calpine |
| 5 | Antipain dihydrochl oride from microbial source | 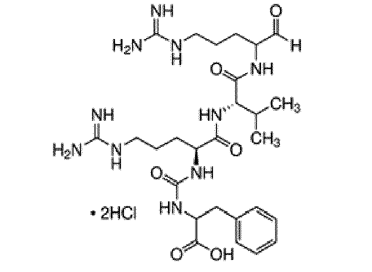 | 677.62 | ▪Calpine |
| | | | | ▪Papain |
| | | | | ▪Trypsin |
| | | | | ▪Cathepsin A |
| | | | | ▪Cathepsin B |
| | | | | ▪Cathepsin D |
| | | | | ▪Plasmin |
| | | | | ▪Chymotrypsin |
| | | | | ▪Pepsin |
| | | | | ▪Granzyme B |
| | | | | ▪Thrombin |
| 6 | 4-Chloromerc uribenzoic acid | 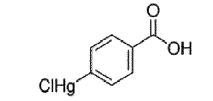 | 357.16 | ▪Calpine |
| | | | | ▪Carboxypeptidase |
| | | | | ▪Clostripain |
| 7 | Leupeptin | 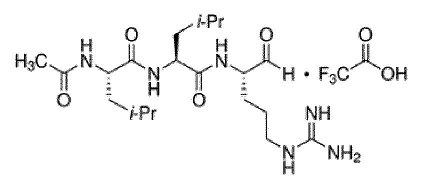 | 426.55 | ▪Plasmin |
| | | | | ▪Trypsin |
| | | | | ▪Papain |
| | | | | ▪Calpine |
| | | | | ▪Cathepsin B |
| | | | | ▪Thrombin |
| | | | | ▪Kallikrein |
| | | | | ▪Endoproteinase |
| | | | | ▪Chymotrypsin |
| | | | | ▪Proteasome (β2) |

**[Table 17]**

| **Calpain** I **inhibitor** | | | | |
|---|---|---|---|---|
| No. | Name | Structural formula | Molecul ar weiqht | Protease to be inhibited |
| 1 | Calpain Inhibitor I (ALLN, Ac-LLnL-CHO, MG-101) | 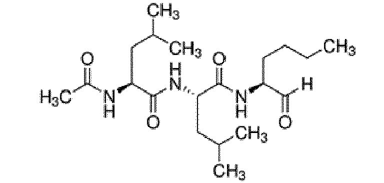 | 383.53 | |
| | | | | ▪Cathepsin B |
| | | | | ▪Cathepsin L |
| | | | | ▪Calpine |
| | | | | ▪Proteasome |
| 2 | Calpain Inhibitor II | 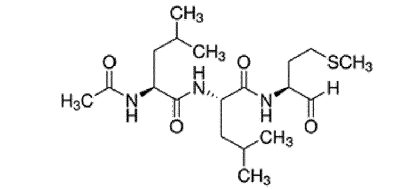 | 401.56 | |
| | | | | ▪Cathepsin B |
| | | | | ▪Calpine |
| | | | | ▪Proteasome |

**[Table 18]**

| **Calpain** II inhibitor | | | | |
|---|---|---|---|---|
| No. | Name | Structural formula | Molecul ar weiqht | Protease to be inhibited |
| 1 | E-64c | 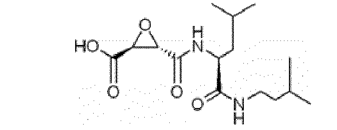 | 314.38 | |
| 2 | Calpain Inhibitor I (ALLN, Ac-LLnL-CHO, MG-101 ) | 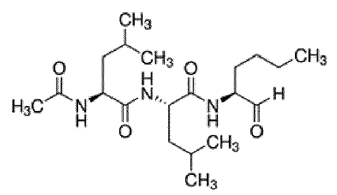 | 383.53 | |
| | | | | ▪Cathepsin B |
| | | | | ▪Cathepsin L |
| | | | | ▪Calpine |
| | | | | ▪Proteasome |
| 3 | Calpain Inhibitor II | 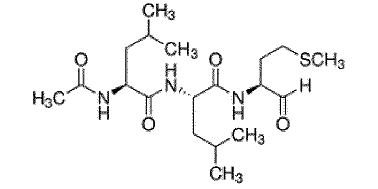 | 401.56 | |
| | | | | ▪Cathepsin B |
| | | | | ▪Calpine |
| | | | | ▪Proteasome |
| 4 | N-Ethylmalei mide | 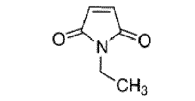 | 125.13 | ▪Ficin |
| | | | | ▪Calpine |
| 5 | Antipain dihydrochl oride from microbial source | 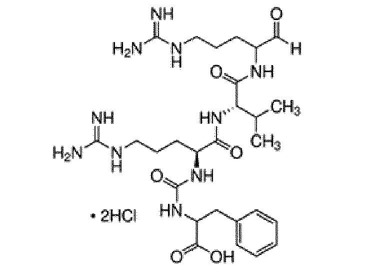 | 677.62 | ▪Calpine |
| | | | | ▪Papain |
| | | | | ▪Trypsin |
| | | | | ▪Cathepsin A |
| | | | | ▪Cathepsin B |
| | | | | ▪Cathepsin D |
| | | | | ▪Plasmin |
| | | | | ▪Chymotrypsin |
| | | | | ▪Pepsin |
| | | | | ▪Granzyme B |
| | | | | ▪Thrombin |
| 6 | 4-Chloromerc uribenzoic acid | 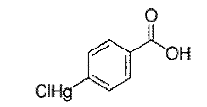 | 357.16 | ▪Calpine |
| | | | | ▪Carboxypeptidase |
| | | | | ▪Clostripain |
| 7 | Leupeptin | 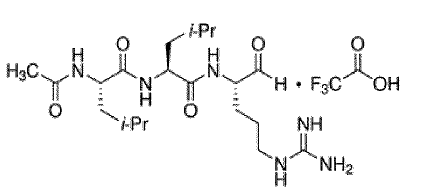 | 426.55 | ▪Plasmin |
| | | | | ▪Trypsin |
| | | | | ▪Papain |
| | | | | ▪Calpine |
| | | | | ▪Cathepsin B |
| | | | | ▪Thrombin |
| | | | | ▪Kallikrein |
| | | | | ▪Endoproteinase |
| | | | | ▪Chymotrypsin |
| | | | | ▪Proteasome (β2) |

**[Table 19]**

| **Carboxypeptidase** A/B inhibitor | | | | |
|---|---|---|---|---|
| No. | Name | Structural formula | Molecul ar weight | Protease to be inhibited |
| 1 | Ethylene glycol-bis(2-aminoethyl ether)-N,N,N',N'-tetraaceti c acid | 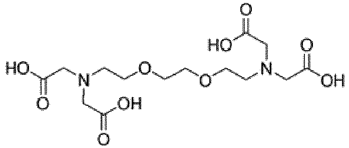 | 380.35 | ▪Carboxypeptidase A |
| | | | | ▪Carboxypeptidase B |
| 2 | EDTA disodium salt | 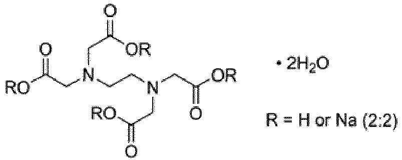 | 372.24 | ▪Carboxypeptidase A |
| | | | | ▪Carboxypeptidase B |
| | | | | ▪Dispase |
| | | | | ▪Collagenase |
| 3 | Pentetic acid (DETAPAC, DTPA) | 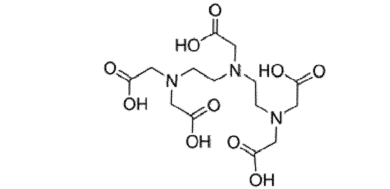 | 393.35 | ▪Carboxypeptidase A |
| | | | | ▪Carboxypeptidase B |
| 4 | 1, 10-Phenanthro line monohydrat e | 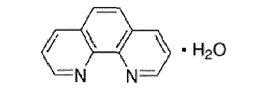 | 198.22 | ▪Carboxypeptidase A |
| | | | | ▪Carboxypeptidase B |
| | | | | ▪Dispase |
| | | | | ▪Leucine aminopeptidase |
| | | | | ▪Thermolysin |

**[Table 20]**

| **Carboxypeptidase** P inhibitor | | | | |
|---|---|---|---|---|
| No. | Name | Structural formula | Molecul ar weight | Protease to be inhibited |
| 1 | Diisopropy lfluoropho sphate | 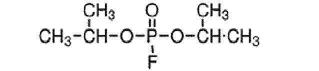 | 184.15 | ▪Carboxypeptidase |
| | | | | ▪Chymotrypsin |
| | | | | ▪Complement |
| | | | | ▪Elastase |
| | | | | ▪Endoproteinase |
| | | | | ▪Kallikrein |
| | | | | ▪Plasmin |
| | | | | ▪Thrombin |
| | | | | ▪Pronase |
| | | | | ▪Proteinase |
| 2 | 4-Chloromerc uribenzoic acid | 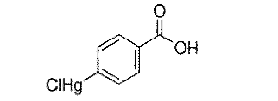 | 357.16 | ▪Calpine |
| | | | | ▪Carboxypeptidase |
| | | | | ▪Clostripain |
| 3 | Diethyl pyrocarbon ate (DEP) | 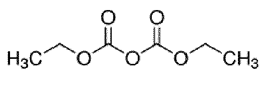 | 162.14 | |
| 4 | Sodium iodoacetat e | 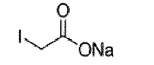 | 207.93 | ▪Carboxypeptidase P |
| | | | | ▪Bromelain |
| | | | | ▪Ficin |
| | | | | ▪Cathepsin |

**[Table 21]**

| **Carboxypeptidase Y** inhibitor | | | | |
|---|---|---|---|---|
| No. | Name | Structural formula | Molecul ar weight | Protease to be inhibited |
| 1 | Diisopropy lfluoropho sphate | 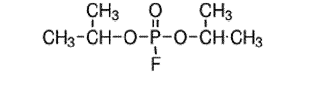 | 184.15 | ▪Carboxypeptidase |
| | | | | ▪Chymotrypsin |
| | | | | ▪Complement |
| | | | | ▪Elastase |
| | | | | ▪Endoproteinase |
| | | | | ▪Kallikrein |
| | | | | ▪Plasmin |
| | | | | ▪Thrombin |
| | | | | ▪Pronase |
| | | | | ▪Proteinase |
| 2 | Phenylmeth anesulfony 1 fluoride | 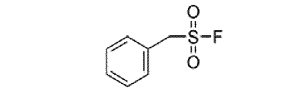 | 174.19 | ▪Thrombin |
| | | | | ▪Elastase |
| | | | | ▪Plasmin |
| | | | | ▪Proteinase |

**[Table 22]**

| **Cathepsin** B inhibitor | | | | |
|---|---|---|---|---|
| No. | Name | Structural formula | Molecul ar weight | Protease to be inhibited |
| 1 | CA-074 | 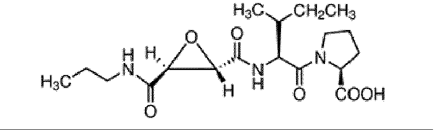 | 383.44 | |
| 2 | CA-074 methyl ester | 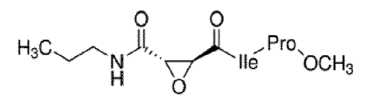 | 397.47 | |
| 3 | E-64 | 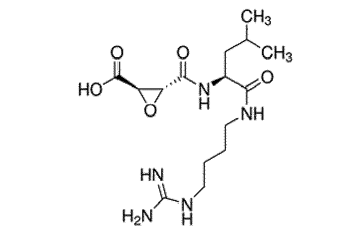 | 357.41 | ▪Cathepsin B |
| | | | | ▪Ficin |
| | | | | ▪Papain |
| | | | | ▪Bromelain |
| 4 | Z-Phe-Phe-fluorometh yl ketone (Z-FF-FMK) | 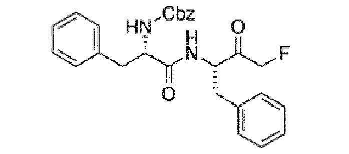 | 462.51 | |
| 5 | Antipain dihydrochl oride from microbial source | 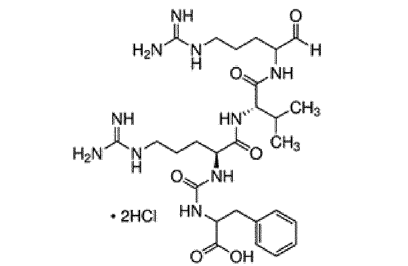 | 677.62 | ▪Calpine |
| | | | | ▪Papain |
| | | | | ▪Trypsin |
| | | | | ▪Cathepsin A |
| | | | | ▪Cathepsin B |
| | | | | ▪Cathepsin D |
| | | | | ▪Plasmin |
| | | | | ▪Chymotrypsin |
| | | | | ▪Pepsin |

**[Table 23]**

| **Cathepsin** B inhibitor (Continued) | | | | |
|---|---|---|---|---|
| No. | Name | Structural formula | Molecul ar weight | Protease to be inhibited |
| 6 | Calpain Inhibitor I (ALLN, Ac-LLnL-CHO, MG-101 ) | 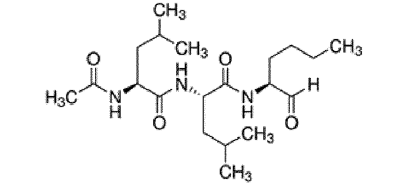 | 383.53 | ▪Cathepsin B |
| | | | | ▪Cathepsin L |
| | | | | ▪Calpine |
| | | | | ▪Proteasome |
| 7 | Calpain Inhibitor II | 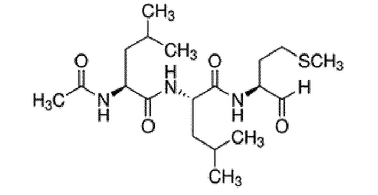 | 401.56 | ▪Cathepsin B |
| | | | | ▪Calpine |
| | | | | ▪Proteasome |
| 8 | Chymostati n | 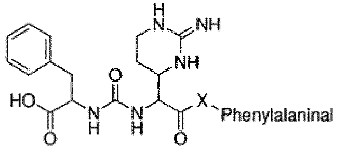 | A: MW = 607.7 | ▪Chymotrypsin |
| | | | | ▪Papain |
| | | | B: MW = 593.7 | ▪Chymotrypsin-like serine proteinase |
| | | Chymostatin A X = Leu | C: MW = 607.7 | ▪Cathepsin A, B, C, B, H, L |
| | | Chymostatin B X = Val | | |
| | | Chymostatin C X = Ile | | |
| 9 | Leupeptin | 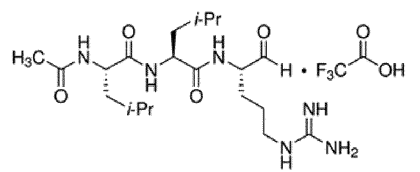 | 426.55 | ▪Plasmin |
| | | | | ▪Trypsin |
| | | | | ▪Papain |
| | | | | ▪Calpine |
| | | | | ▪Cathepsin B |
| | | | | ▪Thrombin |
| | | | | ▪Kallikrein |
| | | | | ▪Endoproteinase |
| | | | | ▪Chymotrypsin |
| | | | | ▪Proteasome (β2) |

**[Table 24]**

| **Cathepsin C** inhibitor | | | | |
|---|---|---|---|---|
| No. | Name | Structural formula | Molecul ar weight | Protease to be inhibited |
| 1 | Sodium iodoacetat e | 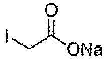 | 207.93 | ▪Carboxypeptidase P |
| | | | | ▪Bromelain |
| | | | | ▪Ficin |
| | | | | ▪Cathepsin |

**[Table 25]**

| **Cathepsin** D inhibitor | | | | |
|---|---|---|---|---|
| No. | Name | Structural formula | Molecul ar weight | Protease to be inhibited |
| 1 | Antipain dihydrochl oride from microbial source | 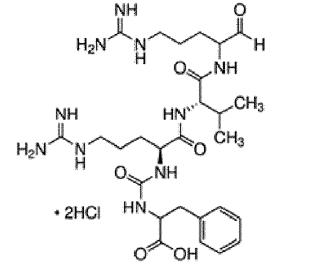 | 677.62 | ▪Calpine |
| | | | | ▪Papain |
| | | | | ▪Trypsin |
| | | | | ▪Cathepsin A |
| | | | | ▪Cathepsin B |
| | | | | ▪Cathepsin D |
| | | | | ▪Plasmin |
| | | | | ▪Chymotrypsin |
| | | | | ▪Pepsin |
| | | | | ▪Granzyme B |
| | | | | ▪Thrombin |
| 2 | Chymostati n | 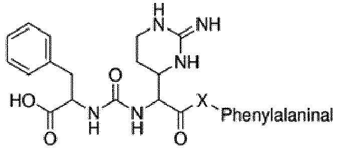 | A: MW = 607.7 | ▪Chymotrypsin |
| | | | | ▪Papain |
| | | | B: MW = 593.7 | ▪Chymotrypsin-like serine proteinase |
| | | Chymostatin A X = Leu | C: MW = 607.7 | ▪Cathepsin A, B, C, B, H, L |
| | | Chymostatin B X = Val | | ▪Proteasome (β5) |
| | | Chymostatin C X = Ile | | |
| 3 | Pepstatin A | 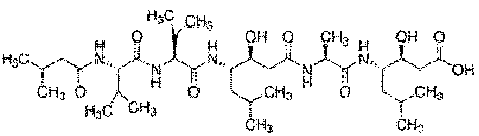 | 685.89 | ▪Pepsin |
| | | | | ▪Cathepsin |

**[Table 26]**

| **Cathepsin L inhibitor** | | | | |
|---|---|---|---|---|
| No. | Name | Structural formula | Molecul ar weight | Protease to be inhibited |
| 1 | Z-Phe-Phe-fluorometh yl ketone (Z-FF-FMK) | 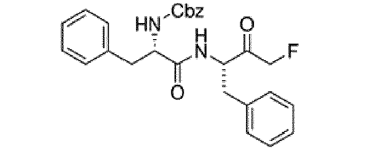 | 462.51 | |
| 2 | Calpain Inhibitor I (ALLN, Ac-LLnL-CHO, MG-101) | 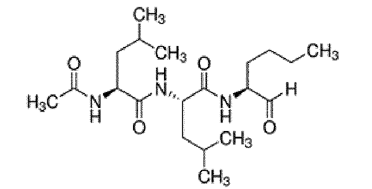 | 383.53 | ▪Cathepsin B |
| | | | | ▪Cathepsin L |
| | | | | ▪Calpine |
| | | | | ▪Proteasome |

**[Table 27]**

| **Chymotrypsin** inhibitor | | | | |
|---|---|---|---|---|
| No. | Name | Structural formula | Molecul ar weight | Protease to be inhibited |
| 1 | Diisopropy lfluoropho sphate | 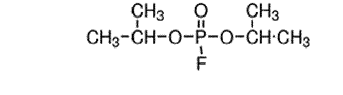 | 184.15 | ▪Carboxypeptidase |
| | | | | ▪Chymotrypsin |
| | | | | ▪Complement |
| | | | | ▪Elastase |
| | | | | ▪Endoproteinase |
| | | | | ▪Kallikrein |
| | | | | ▪Plasmin |
| | | | | ▪Thrombin |
| | | | | ▪Pronase |
| | | | | ▪Proteinase |
| 2 | 4-(2-Aminoethyl )benzenesu lfonyl fluoride hydrochlor ide (AEBSF) | 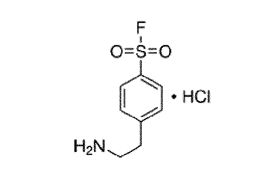 | 239.69 | ▪Plasmin |
| | | | | ▪Trypsin |
| | | | | ▪Chymotrypsin |
| 3 | 6-Aminocapro ic acid | 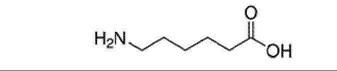 | 131.17 | |
| 4 | Chymostati n | 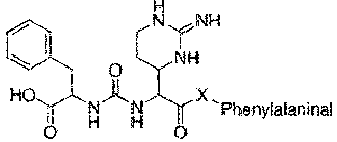 | A: MW = 607.7 | ▪Chymotrypsin |
| | | | | ▪Papain |
| | | Chymostatin A X = Leu | B: MW = 593.7 | ▪Chymotrypsin-like serine proteinase |
| | | Chymostatin B X = Val | C: MW = 607.7 | ▪Cathepsin A, B, C, B, H, L |
| | | Chymostatin C X = Ile | | ▪Proteasome (β5) |

**[Table 28]**

| **Chymotrypsin inhibitor** (Continued) | | | | |
|---|---|---|---|---|
| No. | Name | Structural formula | Molecul ar weiqht | Protease to be inhibited |
| 1 | N-p-Tosyl-L-phenylalan ine chlorometh yl ketone | 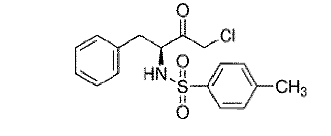 | 351.85 | ▪Papain |
| | | | | ▪Chymotrypsin |
| | | | | ▪Ficin |
| | | | | ▪Bromelain |
| 2 | Bromoenol lactone | 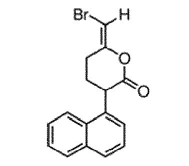 | 317.18 | |
| 3 | Gabexate mesylate | 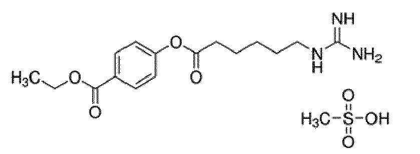 | 417.48 | |
| 4 | Leupeptin | 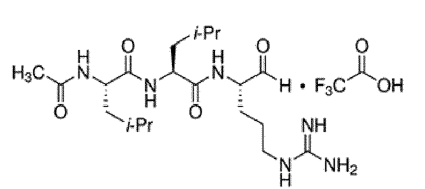 | 426.55 | ▪Plasmin |
| | | | | ▪Trypsin |
| | | | | ▪Papain |
| | | | | ▪Calpine |
| | | | | ▪Cathepsin B |
| | | | | ▪Thrombin |
| | | | | ▪Kallikrein |
| | | | | ▪Endoproteinase |
| | | | | ▪Chymotrypsin |
| | | | | ▪Proteasome (β2) |

**[Table 29]**

| **Clostripain inhibitor** | | | | |
|---|---|---|---|---|
| No. | Name | Structural formula | Molecul ar weiqht | Protease to be inhibited |
| 1 | 4-Chloromerc uribenzoic acid | 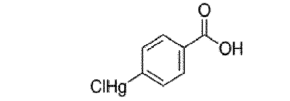 | 357.16 | ▪Calpine |
| | | | | ▪Carboxypeptidase |
| | | | | ▪Clostripain |
| 2 | Nα-Tosyl-L-lysine chlorometh yl ketone hydrochlor ide | 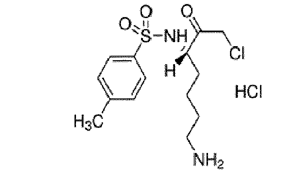 | 369.31 | |

**[Table 30]**

| **Collagenase inhibitor** | | | | |
|---|---|---|---|---|
| No. | Name | Structural formula | Molecul ar weiqht | Protease to be inhibited |
| 1 | EDTA disodium salt | 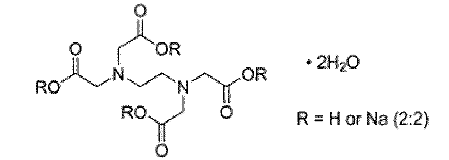 | 372.24 | |
| | | | | ▪Carboxypeptidase A |
| | | | | ▪Carboxypeptidase B |
| | | | | ▪Dispase |
| | | | | ▪Collagenase |
| 2 | Dichlorome thylene diphosphon ic acid disodium salt (DMDP) | 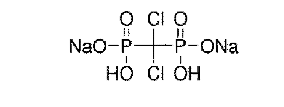 | 288.86 | |

**[Table 31]**

| Complement Clr/Cls inhibitor | | | | |
|---|---|---|---|---|
| No. | Name | Structural formula | Molecul ar weiqht | Protease to be inhibited |
| 1 | Diisopropy lfluoropho sphate | 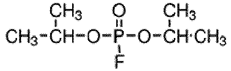 | 184.15 | ▪Carboxypeptidase |
| | | | | ▪Chymotrypsin |
| | | | | ▪Complement |
| | | | | ▪Elastase |
| | | | | ▪Endoproteinase |
| | | | | ▪Kallikrein |
| | | | | ▪Plasmin |
| | | | | ▪Thrombin |
| | | | | ▪Pronase |
| | | | | ▪Proteinase |

**[Table 32]**

| **Complement factor D/B inhibitor** | | | | |
|---|---|---|---|---|
| No. | Name | Structural formula | Molecul ar weiqht | Protease to be inhibited |
| 1 | Diisopropy lfluoropho sphate | 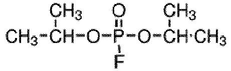 | 184.15 | ▪Carboxypeptidase |
| | | | | ▪Chymotrypsin |
| | | | | ▪Complement |
| | | | | ▪Elastase |
| | | | | ▪Endoproteinase |
| | | | | ▪Kallikrein |
| | | | | ▪Plasmin |
| | | | | ▪Thrombin |
| | | | | ▪Pronase |
| | | | | ▪Proteinase |

**[Table 33]**

| **Dipeptidyl peptidase** II **inhibitor** | | | | |
|---|---|---|---|---|
| No. | Name | Structural formula | Molecul ar weiqht | Protease to be inhibited |
| 1 | Puromycin | 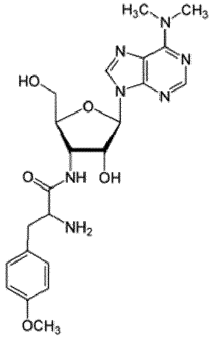 | 471.51 | ▪Dipeptidyl peptidase II |
| | | | | ▪Cytosol alanyl aminopeptidase |

**[Table 34]**

| **Dipeptidyl peptidase III inhibitor** | | | | |
|---|---|---|---|---|
| No. | Name | Structural formula | Molecul ar weiqht | Protease to be inhibited |
| 1 | Opiorphin | 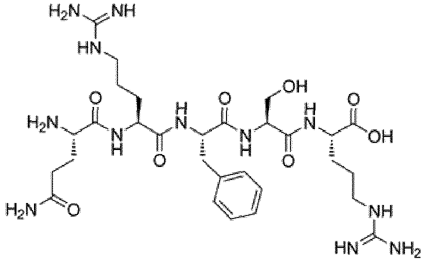 | 692.77 | ▪Enkephalinase |
| | | | | ▪Neprilysin |
| | | | | ▪Dipeptidyl peptidase III |
| | | | | ▪Cytosol alanyl aminopeptidase |

**[Table 35]**

| **Dipeptidyl peptidase IV inhibitor** | | | | |
|---|---|---|---|---|
| No. | Name | Structural formula | Molecul ar weiqht | Protease to be inhibited |
| 1 | Ile-Pro-Ile | 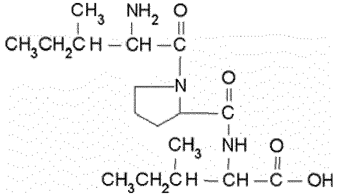 | 341.45 | ▪Dipeptidyl peptidase IV |

**[Table 36]**

| **Dispase inhibitor** | | | | |
|---|---|---|---|---|
| No. | Name | Structural formula | Molecul ar weiqht | Protease to be inhibited |
| 1 | EDTA disodium salt | 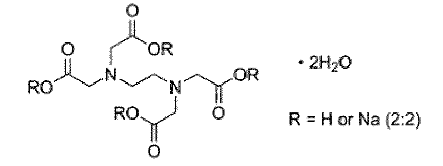 | 372.24 | ▪Carboxypeptidase A |
| | | | | ▪Carboxypeptidase B |
| | | | | ▪Dispase |
| | | | | ▪Collagenase |
| 2 | 1,10-Phenanthro line monohydrat e | 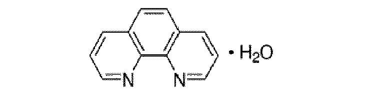 | 198.22 | ▪Carboxypeptidase A |
| | | | | ▪Carboxypeptidase B |
| | | | | ▪Dispase |
| | | | | ▪Leucine aminopeptidase |
| | | | | ▪Thermolysin |

**[Table 37]**

| **Elastase (granulocyte) inhibitor** | | | | |
|---|---|---|---|---|
| No. | Name | Structural formula | Molecul ar weiqht | Protease to be inhibited |
| 1 | N-(Methoxysu ccinyl)-Ala-Ala-Pro-Val-chlorometh yl ketone | 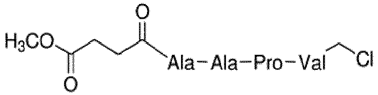 | 502.99 | |

**[Table 38]**

| **Elastase (leukocyte) inhibitor** | | | | |
|---|---|---|---|---|
| No. | Name | Structural formula | Molecul ar weight | Protease to be inhibited |
| 1 | Diisopropy lfluoropho sphate | 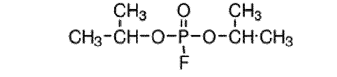 | 184.15 | ▪Carboxypeptidase |
| | | | | ▪Chymotrypsin |
| | | | | ▪Complement |
| | | | | ▪Elastase |
| | | | | ▪Endoproteinase |
| | | | | ▪Kallikrein |
| | | | | ▪Plasmin |
| | | | | ▪Thrombin |
| | | | | ▪Pronase |
| | | | | ▪Proteinase |
| 2 | 3,4-Dichlorois ocoumarin | 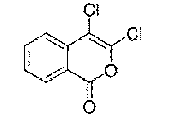 | 215.03 | ▪Thrombin |
| | | | | ▪Papain |
| | | | | ▪Plasmin |
| 3 | Phenylmeth anesulfony 1 fluoride | 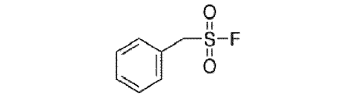 | 174.19 | ▪Thrombin |
| | | | | ▪Elastase |
| | | | | ▪Plasmin |
| | | | | ▪Proteinase |

**[Table 39]**

| **Elastase** (pancreas) inhibitor | | | | |
|---|---|---|---|---|
| No. | Name | Structural formula | Molecul ar weight | Protease to be inhibited |
| 1 | Diisopropy lfluoropho sphate | 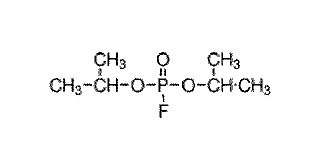 | 184.15 | ▪Carboxypeptidase |
| | | | | ▪Chymotrypsin |
| | | | | ▪Complement |
| | | | | ▪Elastase |
| | | | | ▪Endoproteinase |
| | | | | ▪Kallikrein |
| | | | | ▪Plasmin |
| | | | | ▪Thrombin |
| | | | | ▪Pronase |
| | | | | ▪Proteinase |
| 2 | 3,4-Dichlorois ocoumarin | 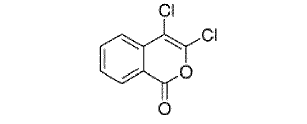 | 215.03 | ▪Thrombin |
| | | | | ▪Papain |
| | | | | ▪Plasmin |

**[Table 40]**

| **Endoproteinase Arg-C inhibitor** | | | | |
|---|---|---|---|---|
| No. | Name | Structural formula | Molecul ar weight | Protease to be inhibited |
| 1 | Diisopropy lfluoropho sphate | 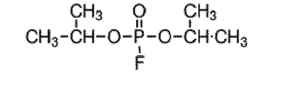 | 184.15 | ▪Carboxypeptidase |
| | | | | ▪Chymotrypsin |
| | | | | ▪Complement |
| | | | | ▪Elastase |
| | | | | ▪Endoproteinase |
| | | | | ▪Kallikrein |
| | | | | ▪Plasmin |
| | | | | ▪Thrombin |
| | | | | ▪Pronase |
| | | | | ▪Proteinase |
| 2 | 3,4-Dichlorois ocoumarin | 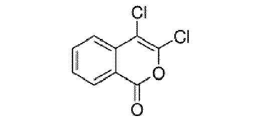 | 215.03 | ▪Thrombin |
| | | | | ▪Papain |
| | | | | ▪Plasmin |

**[Table 41]**

| **Endoproteinase Glu-C** inhibitor | | | | |
|---|---|---|---|---|
| No. | Name | Structural formula | Molecul ar weight | Protease to be inhibited |
| 1 | Diisopropy lfluoropho sphate | 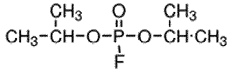 | 184.15 | ▪Carboxypeptidase |
| | | | | ▪Chymotrypsin |
| | | | | ▪Complement |
| | | | | ▪Elastase |
| | | | | ▪Endoproteinase |
| | | | | ▪Kallikrein |
| | | | | ▪Plasmin |
| | | | | ▪Thrombin |
| | | | | ▪Pronase |
| | | | | ▪Proteinase |

**[Table 42]**

| **Endoproteinase Lys-C inhibitor** | | | | |
|---|---|---|---|---|
| No. | Name | Structural formula | Molecul ar weight | Protease to be inhibited |
| 1 | Diisopropy lfluoropho sphate | 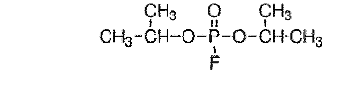 | 184.15 | ▪Carboxypeptidase |
| | | | | ▪Chymotrypsin |
| | | | | ▪Complement |
| | | | | ▪Elastase |
| | | | | ▪Endoproteinase |
| | | | | ▪Kallikrein |
| | | | | ▪Plasmin |
| | | | | ▪Thrombin |
| | | | | ▪Pronase |
| | | | | ▪Proteinase |
| 2 | 3,4-Dichlorois ocoumarin | 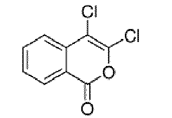 | 215.03 | ▪Thrombin |
| | | | | ▪Papain |
| | | | | ▪Plasmin |
| 3 | Leupeptin | 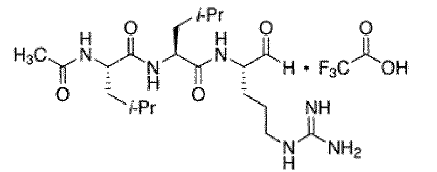 | 426.55 | ▪Plasmin |
| | | | | ▪Trypsin |
| | | | | ▪Papain |
| | | | | ▪Calpine |
| | | | | ▪Cathepsin B |
| | | | | ▪Thrombin |
| | | | | ▪Kallikrein |
| | | | | ▪Endoproteinase |
| | | | | ▪Chymotrypsin |
| | | | | ▪Proteasome (β2) |

**[Table 43]**

| **Ficin inhibitor** | | | | |
|---|---|---|---|---|
| No. | Name | Structural formula | Molecul ar weight | Protease to be inhibited |
| 1 | E-64 | 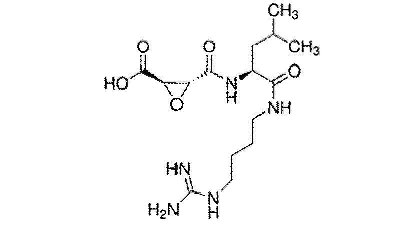 | 357.41 | |
| | | | | ▪Cathepsin B |
| | | | | ▪Ficin |
| | | | | ▪Papain |
| | | | | ▪Bromelain |
| 2 | N-Ethylmalei mide | 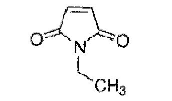 | 125.13 | ▪Calpine |
| | | | | ▪Ficin |
| 3 | N-p-Tosyl-L-phenilalan ine chlorometh yl ketone | 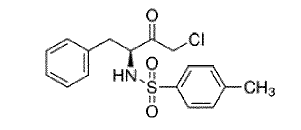 | 351.85 | ▪Papain |
| | | | | ▪Chymotrypsin |
| | | | | ▪Ficin |
| | | | | ▪Bromelain |
| 4 | Sodium iodoacetat e | 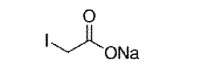 | 207.93 | ▪Carboxypeptidase P |
| | | | | ▪Bromelain |
| | | | | ▪Ficin |
| | | | | ▪Cathepsin |
| 5 | Nα-ToSyl-L-lysine chlorometh yl ketone hydrochlor ide | 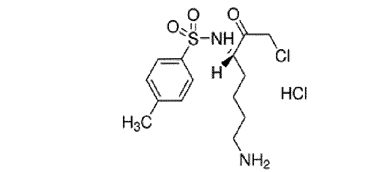 | 369.31 | |

**[Table 44]**

| **Granzyme B inhibitor** | | | | |
|---|---|---|---|---|
| No. | Name | Structural formula | Molecul ar weight | Protease to be inhibited |
| 1 | Antipain dihydrochl oride from microbial source | 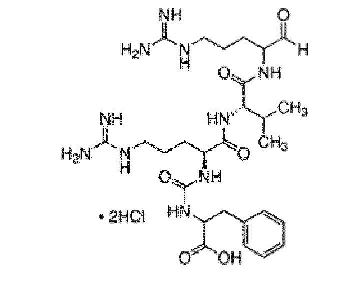 | 677.62 | ▪Calpine |
| | | | | ▪Papain |
| | | | | ▪Trypsin |
| | | | | ▪Cathepsin A |
| | | | | ▪Cathepsin B |
| | | | | ▪Cathepsin D |
| | | | | ▪Plasmin |
| | | | | ▪Chymotrypsin |
| | | | | ▪Pepsin |
| | | | | ▪Granzyme B |
| | | | | ▪Thrombin |
| 2 | 3,4-Dichlorois ocoumarin | 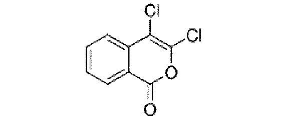 | 215.03 | ▪Thrombin |
| | | | | ▪Papain |
| | | | | ▪Plasmin |

**[Table 45]**

| **Kallikrein (tissue) inhibitor** | | | | |
|---|---|---|---|---|
| No. | Name | Structural formula | Molecul ar weight | Protease to be inhibited |
| 1 | Diisopropy lfluoropho sphate | 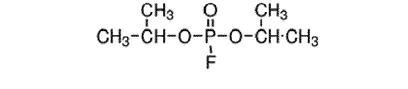 | 184.15 | ▪Carboxypeptidase |
| | | | | ▪Chymotrypsin |
| | | | | ▪Complement |
| | | | | ▪Elastase |
| | | | | ▪Endoproteinase |
| | | | | ▪Kallikrein |
| | | | | ▪Plasmin |
| | | | | ▪Thrombin |
| | | | | ▪Pronase |
| | | | | ▪Proteinase |
| 2 | 3,4-Dichlorois ocoumarin | 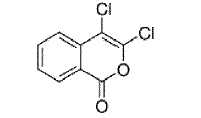 | 215.03 | ▪Thrombin |
| | | | | ▪Papain |
| | | | | ▪Plasmin |
| 3 | Leupeptin | 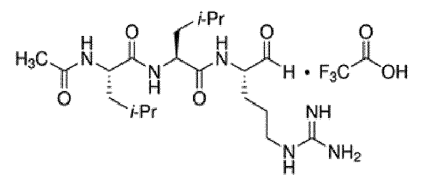 | 426.55 | ▪Plasmin |
| | | | | ▪Trypsin |
| | | | | ▪Papain |
| | | | | ▪Calpine |
| | | | | ▪Cathepsin B |
| | | | | ▪Thrombin |
| | | | | ▪Kallikrein |
| | | | | ▪Endoproteinase |
| | | | | ▪Chymotrypsin |
| | | | | ▪Proteasome (β2) |

**[Table 46]**

| **Kallikrein (plasma) inhibitor** | | | | |
|---|---|---|---|---|
| No. | Name | Structural formula | Molecul ar weight | Protease to be inhibited |
| 1 | Gabexate mesylate | 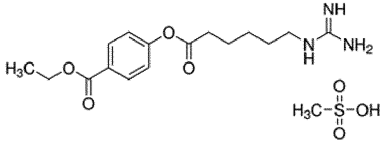 | 417.48 | |

**[Table 47]**

| **Leucine aminopeptidase inhibitor** | | | | |
|---|---|---|---|---|
| No. | Name | Structural formula | Molecul ar weight | Protease to be inhibited |
| 1 | Actinonin | 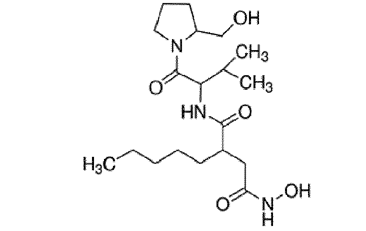 | 385.5 | |
| 2 | Bestatin hydrochlor ide | 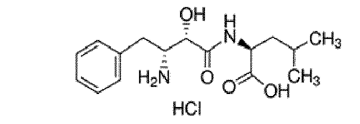 | 344.83 | ▪Aminopeptidase B |

**[Table 48]**

| **Leucine aminopeptidase (cytosol) inhibitor** | | | | |
|---|---|---|---|---|
| No. | Name | Structural formula | Molecul ar weight | Protease to be inhibited |
| 1 | Actinonin | 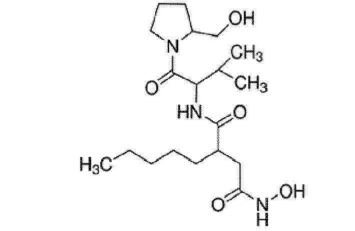 | 385.5 | |
| 2 | Amastatin hydrochlor ide hydrate | 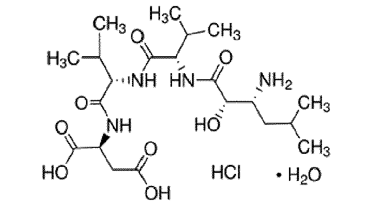 | 511.01 (anhydr ous basis) | |
| 3 | Ethylene glycol-bis (2-aminoethyl ether)-N,N,N',N'-tetraaceti c acid | 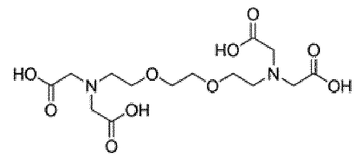 | 380.35 | |
| 4 | Ethylenedi aminetetra acetic acid disodium salt dihydrate | 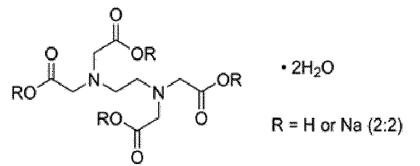 | 372.24 | |

**[Table 49]**

| **Leucine aminopeptidase (cytosol) inhibitor (Continued)** | | | | |
|---|---|---|---|---|
| No. | Name | Structural formula | Molecul ar weiqht | Protease to be inhibited |
| 5 | Diethylene triaminepe ntaacetic acid | 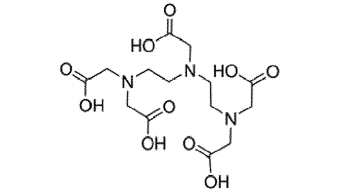 | 393.35 | |
| 6 | 3, 4-Dichlorois ocoumarin | 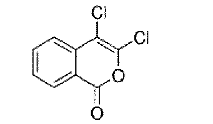 | 215.03 | ▪Thrombin |
| | | | | ▪Papain |
| | | | | ▪Plasmin |
| 7 | 1, 10-Phenanthro line monohydrat e | 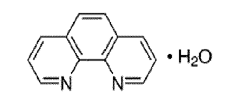 | 198.22 | ▪Carboxypeptidase A |
| | | | | ▪Carboxypeptidase B |
| | | | | ▪Dispase |
| | | | | ▪Leucine aminopeptidase |
| | | | | ▪Thermolysin |
| 8 | Bestatin hydrochlor ide | 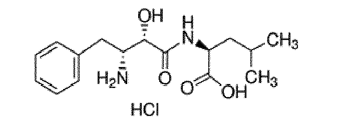 | 344.83 | ▪AminopeptidaseB |

**[Table 50]**

| **Leucine aminopeptidase (microsome) inhibitor** | | | | |
|---|---|---|---|---|
| No. | Name | Structural formula | Molecul ar weight | Protease to be inhibited |
| 1 | Actinonin | 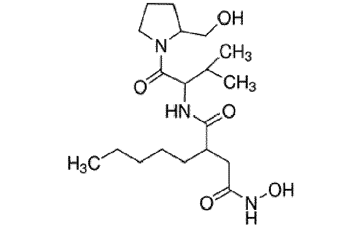 | 385.5 | |
| 2 | Amastatin hydrochlor ide hydrate | 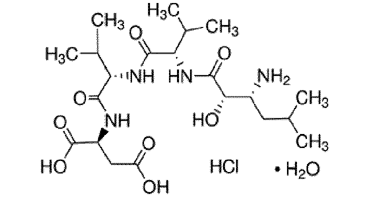 | 511.01 (anhydr ous basis) | |
| 3 | Bestatin hydrochlor ide | 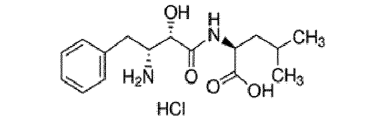 | 344.83 | ▪Aminopeptidase B |

**[Table 51]**

| **Matrix aminopeptidase inhibitor** | | | | |
|---|---|---|---|---|
| No. | Name | Structural formula | Molecul ar weight | Protease to be inhibited |
| 1 | GM6001 | 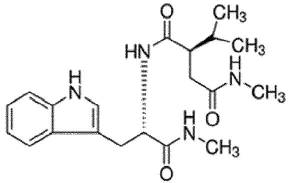 | 388.46 | |

**[Table 52]**

| **Metalloprotease inhibitor** | | | | |
|---|---|---|---|---|
| No. | Name | Structural formula | Molecul ar weight | Protease to be inhibited |
| 1 | Epiamastat in hydrochlor ide | 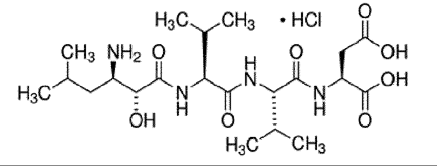 | 474.55 | |

**[Table 53]**

| **Papain inhibitor** | | | | |
|---|---|---|---|---|
| No. | Name | Structural formula | Molecul ar weight | Protease to be inhibited |
| 1 | E-64 | 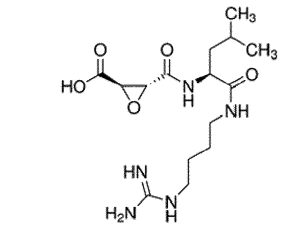 | 357.41 | |
| 2 | Gly-Gly-Tyr-Arg | 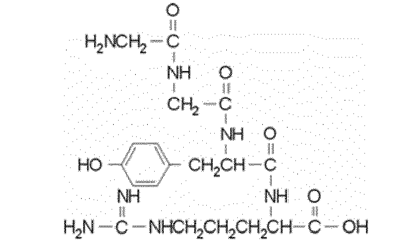 | 451.48 | |
| 3 | Antipain dihydrochl oride from microbial source | 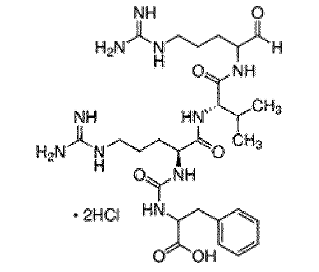 | 677.62 | ▪Calpine |
| | | | | ▪Papain |
| | | | | ▪Trypsin |
| | | | | ▪Cathepsin A |
| | | | | ▪Cathepsin B |
| | | | | ▪Cathepsin D |
| | | | | ▪Plasmin |
| | | | | ▪Chymotrypsin |
| | | | | ▪Pepsin |
| | | | | ▪Granzyme B |
| | | | | ▪Thrombin |
| 4 | Ebselen | 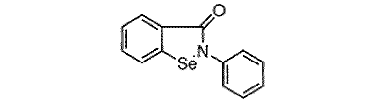 | 274.18 | |

**[Table 54]**

| **Papain inhibitor (Continued)** | | | | |
|---|---|---|---|---|
| No. | Name | Structural formula | Molecul ar weight | Protease to be inhibited |
| 5 | Chymostati n | 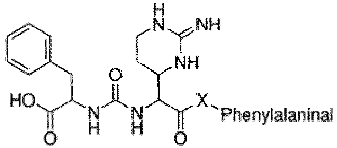 | A: MW = 607.7 | ▪Chymotrypsin |
| | | | | ▪Papain |
| | | | B: MW = 593.7 | ▪Chymotrypsin-like serine proteinase |
| | | Chymostatin A X = Leu | C: MW = 607.7 | ▪Cathepsin A, B, C, B, H, L |
| | | Chymostatin B X = Val | | ▪Proteasome (β5) |
| | | Chymostatin C X = Ile | | |
| 6 | Cystamine dihydrochl oride | 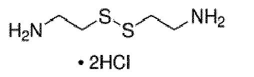 | 225.2 | |
| 7 | 3,4-Dichlorois ocoumarin | 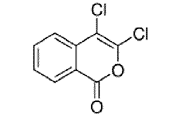 | 215.03 | ▪Thrombin |
| | | | | ▪Papain |
| | | | | ▪Plasmin |
| 8 | N-p-Tosyl-L-phenilalan ine chlorometh yl ketone | 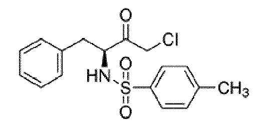 | 351.85 | ▪Papain |
| | | | | ▪Chymotrypsin |
| | | | | ▪Ficin |
| | | | | ▪Bromelain |
| 9 | Leupeptin | 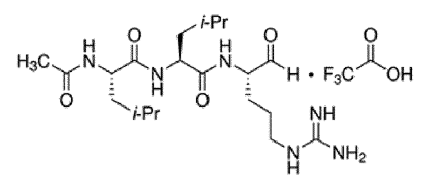 | 426.55 | ▪Plasmin |
| | | | | ▪Trypsin |
| | | | | ▪Papain |
| | | | | ▪Calpine |
| | | | | ▪Cathepsin B |
| | | | | ▪Thrombin |
| | | | | ▪Kallikrein |
| | | | | ▪Endoproteinase |
| | | | | ▪Chymotrypsin |
| | | | | ▪Proteasome (β2) |

**[Table 55]**

| **Pepsin inhibitor** | | | | |
|---|---|---|---|---|
| No. | Name | Structural formula | Molecul ar weight | Protease to be inhibited |
| 1 | Pepstatin A | 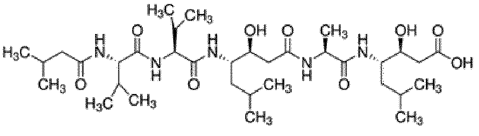 | 685.89 | ▪Cathepsin D |

**[Table 56]**

| **Plasmin inhibitor** | | | | |
|---|---|---|---|---|
| No. | Name | Structural formula | Molecul ar weight | Protease to be inhibited |
| 1 | Diisopropy lfluoropho sphate | 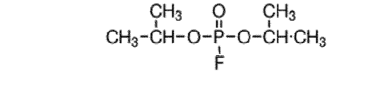 | 184.15 | ▪Carboxypeptidase |
| | | | | ▪Chymotrypsin |
| | | | | ▪Complement |
| | | | | ▪Elastase |
| | | | | ▪Endoproteinase |
| | | | | ▪Kallikrein |
| | | | | ▪Plasmin |
| | | | | ▪Thrombin |
| | | | | ▪Pronase |
| | | | | ▪Proteinase |
| 2 | Elastatina 1 | 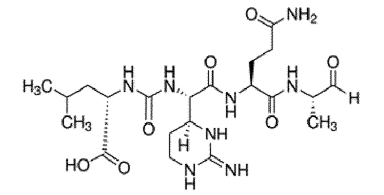 | 512.56 | |
| 3 | 4-(2-Aminoethyl )benzenesu Ifonyl fluoride hydrochlor ide (AEBSF) | 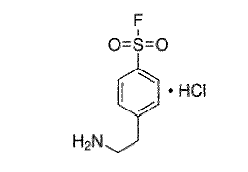 | 239.69 | ▪Plasmin |
| | | | | ▪Trypsin |
| | | | | ▪Chymotrypsin |
| 4 | 6-Aminocapro ic acid | 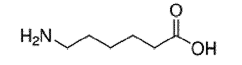 | 131.17 | |
| 5 | Antipain dihydrochl oride from microbial source | 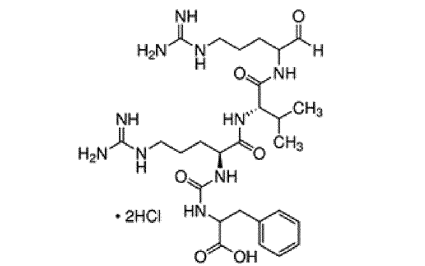 | 677.62 | ▪Calpine |
| | | | | ▪Papain |
| | | | | ▪Trypsin |
| | | | | ▪Cathepsin A |
| | | | | ▪Cathepsin B |
| | | | | ▪Cathepsin D |
| | | | | ▪Plasmin |
| | | | | ▪Chymotrypsin |
| | | | | ▪Pepsin |
| | | | | ▪Granzyme B |
| | | | | ▪Thrombin |

**[Table 57]**

| **Plasmin inhibitor (Continued)** | | | | |
|---|---|---|---|---|
| No. | Name | Structural formula | Molecul ar weight | Protease to be inhibited |
| 6 | 3,4-Dichlorois ocoumarin | 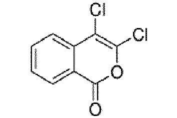 | 215.03 | ▪Thrombin |
| | | | | ▪Papain |
| | | | | ▪Plasmin |
| 7 | Phenylmeth anesulfony 1 fluoride | 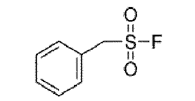 | 174.19 | ▪Thrombin |
| | | | | ▪Elastase |
| | | | | ▪Plasmin |
| | | | | ▪Proteinase |
| 8 | Gabexate mesylate | 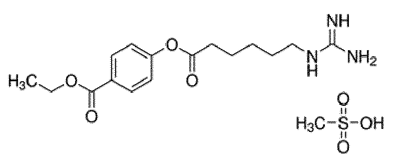 | 417.48 | |
| 9 | Leupeptin | 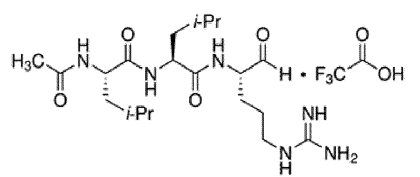 | 426.55 | ▪Plasmin |
| | | | | ▪Trypsin |
| | | | | ▪Papain |
| | | | | ▪Calpine |
| | | | | ▪Cathepsin B |
| | | | | ▪Thrombin |
| | | | | ▪Kallikrein |
| | | | | ▪Endoproteinase |
| | | | | ▪Chymotrypsin |
| | | | | ▪Proteasome (β2) |

**[Table 58]**

| **Thrombin inhibitor** | | | | |
|---|---|---|---|---|
| No. | Name | Structural formula | Molecul ar weight | Protease to be inhibited |
| 1 | Diisopropy lfluoropho sphate | 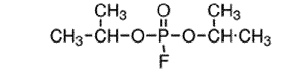 | 184.15 | ▪Carboxypeptidase |
| | | | | ▪Chymotrypsin |
| | | | | ▪Complement |
| | | | | ▪Elastase |
| | | | | ▪Endoproteinase |
| | | | | ▪Kallikrein |
| | | | | ▪Plasmin |
| | | | | ▪Thrombin |
| | | | | ▪Pronase |
| | | | | ▪Proteinase |
| 2 | Nα-Tosyl-L-lysine chlorometh yl ketone hydrochlor ide | 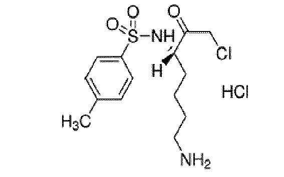 | 369.31 | |
| 3 | 4- (2-Aminoethyl )benzenesu Ifonyl fluoride hydrochlor ide (AEBSF) | 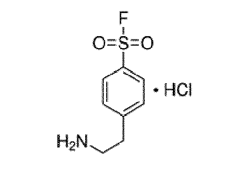 | 239.69 | |
| 4 | Antipain dihydrochl oride from microbial source | 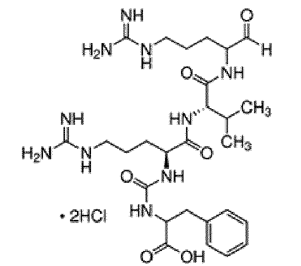 | 677.62 | ▪Calpine |
| | | | | ▪Papain |
| | | | | ▪Trypsin |
| | | | | ▪Cathepsin A |
| | | | | ▪Cathepsin B |
| | | | | ▪Cathepsin D |
| | | | | ▪Plasmin |
| | | | | ▪Chymotrypsin |
| | | | | ▪Pepsin |

**[Table 59]**

| **Thrombin inhibitor (Continued)** | | | | |
|---|---|---|---|---|
| No. | Name | Structural formula | Molecul ar weight | Protease to be inhibited |
| 5 | 3,4-Dichlorois ocoumarin | 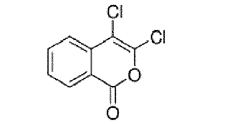 | 215.03 | ▪Thrombin |
| | | | | ▪Papain |
| | | | | ▪Plasmin |
| 6 | Phenylmeth anesulfony 1 fluoride | 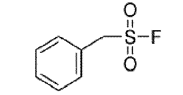 | 174.19 | ▪Thrombin |
| | | | | ▪Elastase |
| | | | | ▪Plasmin |
| | | | | ▪Proteinase |
| 7 | Gabexate mesylate | 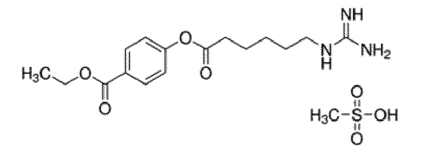 | 417.48 | |
| 8 | Leupeptin | 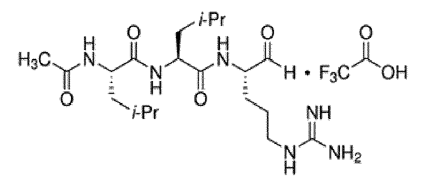 | 426.55 | ▪Plasmin |
| | | | | ▪Trypsin |
| | | | | ▪Papain |
| | | | | ▪Calpine |
| | | | | ▪Cathepsin B |
| | | | | ▪Thrombin |
| | | | | ▪Kallikrein |
| | | | | ▪Endoproteinase |
| | | | | ▪Chymotrypsin |
| | | | | ▪Proteasome (β2) |

**[Table 60]**

| **Thermolysin inhibitor** | | | | |
|---|---|---|---|---|
| No. | Name | Structural formula | Molecul ar weight | Protease to be inhibited |
| 1 | Ethylene glycol-bis(2-aminoethyl ether)-N,N,N',N'-tetraaceti c acid | 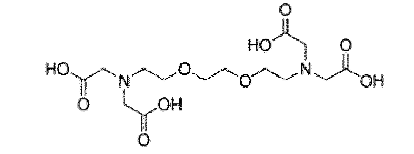 | 380.35 | |
| 2 | Ethylenedi aminetetra acetic acid disodium salt dihydrate | 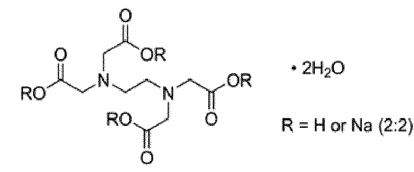 | 372.24 | ▪Carboxypeptidase A |
| | | | | ▪Carboxypeptidase B |
| | | | | ▪Dispase |
| | | | | ▪Collagenase |
| 3 | Diethylene triaminepe ntaacetic acid | 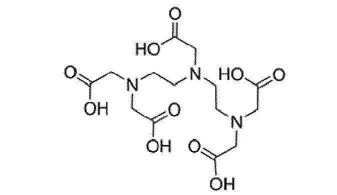 | 393.35 | |
| 4 | 1,10-Phenanthro line monohydrat e | 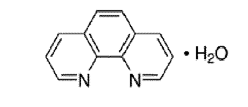 | 198.22 | ▪Carboxypeptidase A |
| | | | | ▪Carboxypeptidase B |
| | | | | ▪Dispase |
| | | | | ▪Leucine aminopeptidase |
| | | | | ▪Thermolysin |
| 5 | Phosphoram idon disodium salt | 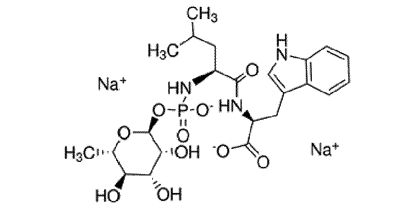 | 587.47 | |

**[Table 61]**

| **Trypsin inhibitor** | | | | |
|---|---|---|---|---|
| No. | Name | Structural formula | Molecul ar weight | Protease to be inhibited |
| 1 | 4- (2-Aminoethyl )benzenesu lfonyl fluoride hydrochlor ide | 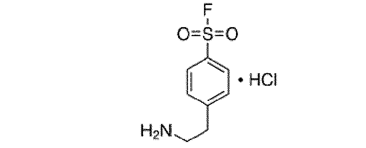 | 239.69 | ▪Plasmin |
| | | | | ▪Trypsin |
| | | | | ▪Chymotrypsin |
| 2 | Antipain dihydrochl oride from microbial source | 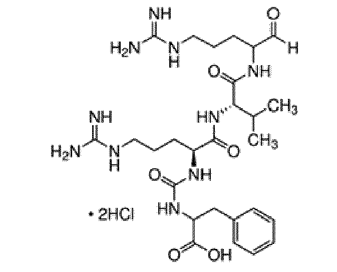 | 677.62 | ▪Calpine |
| | | | | ▪Papain |
| | | | | ▪Trypsin |
| | | | | ▪Cathepsin A |
| | | | | ▪Cathepsin B |
| | | | | ▪Cathepsin D |
| | | | | ▪Plasmin |
| | | | | ▪Chymotrypsin |
| | | | | ▪Pepsin |
| | | | | ▪Granzyme B |
| | | | | ▪Thrombin |
| 3 | Boldine | 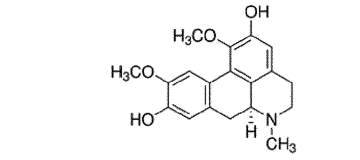 | 327.37 | |

**[Table 62]**

| **Pronase E inhibitor** | | | | |
|---|---|---|---|---|
| No. | Name | Structural formula | Molecul ar weight | Protease to be inhibited |
| 1 | EDTA disodium salt | 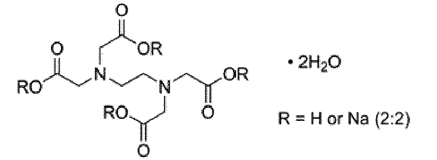 | 372.24 | |
| | | | | ▪Carboxypeptidase A |
| | | | | ▪Carboxypeptidase B |
| | | | | ▪Dispase |
| | | | | ▪Collagenase |
| 2 | Diisopropy lfluoropho sphate | 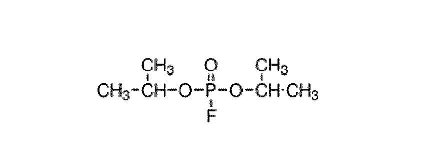 | 184.15 | ▪Carboxypeptidase |
| | | | | ▪Chymotrypsin |
| | | | | ▪Complement |
| | | | | ▪Elastase |
| | | | | ▪Endoproteinase |
| | | | | ▪Kallikrein |
| | | | | ▪Plasmin |
| | | | | ▪Thrombin |
| | | | | ▪Pronase |
| | | | | ▪Proteinase |

**[Table 63]**

| **Procaspase 3 inhibitor** | | | | |
|---|---|---|---|---|
| No. | Name | Structural formula | Molecul ar weight | Protease to be inhibited |
| 1 | N-Acetyl-Glu-Ser-Met-Asp-al (Ac-ESMD-CHO) | 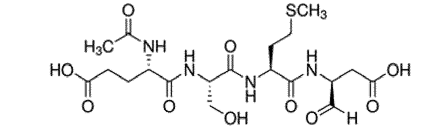 | 506.53 | |
| 2 | N-Acetyl-Ile-Glu-Thr-Asp-al (Ac-IETD-CHO) | 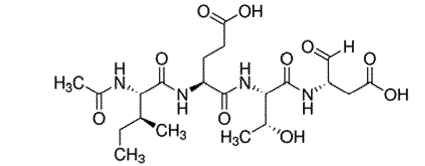 | 502.52 | |

**[Table 64]**

| **Proteinase K inhibitor** | | | | |
|---|---|---|---|---|
| No. | Name | Structural formula | Molecul ar weight | Protease to be inhibited |
| 1 | Phenylmeth anesulfony 1 fluoride | 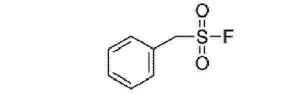 | 174.19 | ▪Thrombin |
| | | | | ▪Elastase |
| | | | | ▪Plasmin |
| | | | | ▪Proteinase |
| 2 | Diisopropy lfluoropho sphate | 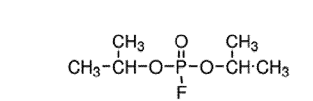 | 184.15 | ▪Carboxypeptidase |
| | | | | ▪Chymotrypsin |
| | | | | ▪Complement |
| | | | | ▪Elastase |
| | | | | ▪Endoproteinase |
| | | | | ▪Kallikrein |
| | | | | ▪Plasmin |
| | | | | ▪Thrombin |
| | | | | ▪Pronase |
| | | | | ▪Proteinase |

**[Table 65]**

| **Renin inhibitor** | | | | |
|---|---|---|---|---|
| No. | Name | Structural formula | Molecul ar weight | Protease to be inhibited |
| 1 | Pepstatin A | 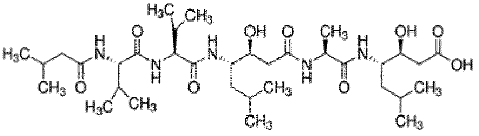 | 685.89 | ▪Cathepsin D |

**[Table 66]**

| **Caspase inhibitor** | | | | |
|---|---|---|---|---|
| No. | Name | Structural formula | Molecul ar weiqht | Protease to be inhibited |
| 1 | BOC-Asp (OMe)-fluorometh yl ketone (Boc-D- | 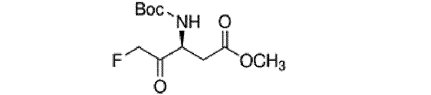 | 263.26 | |
| 2 | Z-Ala-Glu(OMe)-Val-Asp(OMe)-fluorometh yl ketone (Z-AEVD-FMK) | 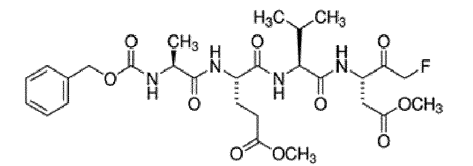 | 610.63 | |

**[Table 67]**

| **Caspase 1 inhibitor** | | | | |
|---|---|---|---|---|
| No. | Name | Structural formula | Molecul ar weiqht | Protease to be inhibited |
| 1 | N-Acetyl-Trp-Glu-His-Asp-al (Ac-WEHD-CHO) | 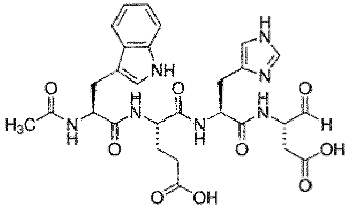 | 611.6 | |

**[Table 68]**

| **Caspase 2 inhibitor** | | | | |
|---|---|---|---|---|
| No. | Name | Structural formula | Molecul ar weiqht | Protease to be inhibited |
| 1 | N-Acetyl-Val-Asp-Val-Ala-Asp-CHO (Ac-VDVAD-CHO) | 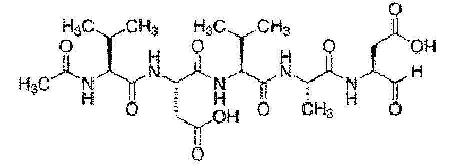 | 543.52 | |
| 2 | Z-Val-Asp(O-Me)-Val-Ala-Asp (O-Me)fluorom ethyl ketone (Z-VDVAD-FMK) | 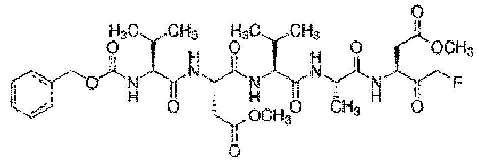 | 695.73 | |

**[Table 69]**

| **Caspase 3 inhibitor** | | | | |
|---|---|---|---|---|
| No. | Name | Structural formula | Molecul ar weiqht | Protease to be inhibited |
| 1 | N-Acetyl-Glu-Ser-Met-Asp-al (Ac-ESMD-CHO) | 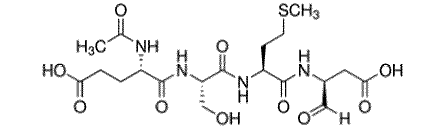 | 506.53 | |
| 2 | Z-Asp(OMe)-Gln-Met-Asp (OMe) fl uoromethyl ketone | 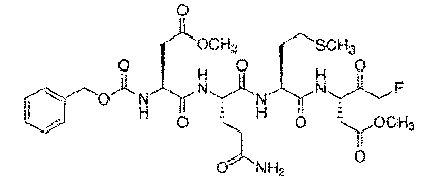 | 685.72 | |
| 3 | N-Acetyl-Asp-Glu-Val-Asp-al (Ac-DEVD-CHO) | 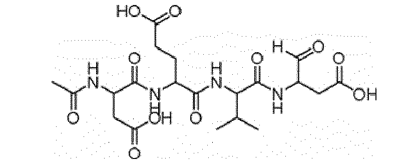 | 502.47 | |
| 4 | N-Acetyl-Ile-Glu-Thr-Asp-al (Ac-IETD-CHO) | 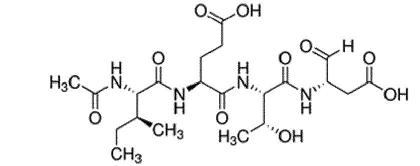 | 502.52 | |

**[Table 70]**

| **Caspase 5 inhibitor** | | | | |
|---|---|---|---|---|
| No. | Name | Structural formula | Molecul ar weiqht | Protease to be inhibited |
| 1 | N-Acetyl-Trp-Glu-His-Asp-al (Ac-WEHD-CHO) | 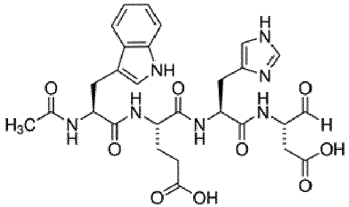 | 611.6 | |

**[Table 71]**

| **Caspase 6 inhibitor** | | | | |
|---|---|---|---|---|
| No. | Name | Structural formula | Molecul ar weiqht | Protease to be inhibited |
| 1 | N-Acetyl-Val-Glu-Ile-Asp-al | 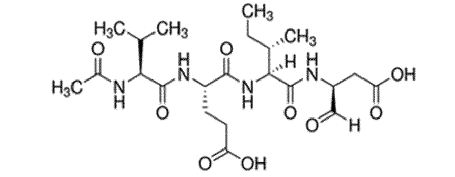 | 500.54 | |
| 2 | Z-Asp(OMe)-Gln-Met-Asp (OMe) fl uoromethyl ketone | 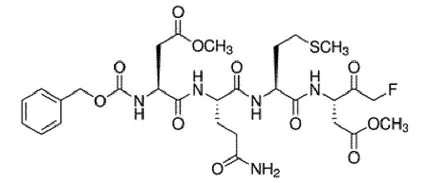 | 685.72 | |
| 3 | Z-Val-Glu(O-Me) - Ile-Asp(O-Me)fluorom ethyl ketone | 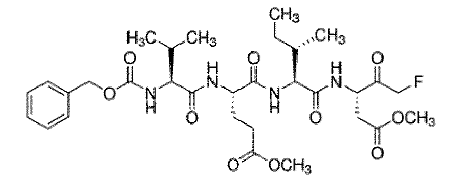 | 652.71 | |

**[Table 72]**

| **Caspase 7 inhibitor** | | | | |
|---|---|---|---|---|
| No. | Name | Structural formula | Molecul ar weiqht | Protease to be inhibited |
| 1 | Z-Asp(O-Me)-Glu(O-Me)-Val-Asp(O-Me) fluoeom ethyl ketone (Z-DEVD-FMK) | 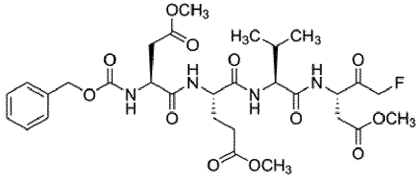 | 668.66 | |

**[Table 73]**

| **Caspase 8 inhibitor** | | | | |
|---|---|---|---|---|
| No. | Name | Structural formula | Molecul ar weight | Protease to be inhibited |
| 1 | Z-Ile-Glu(O-Me)- Thr-Asp(O-Me)fluorom ethyl ketone (Z-IETD-FMK) | 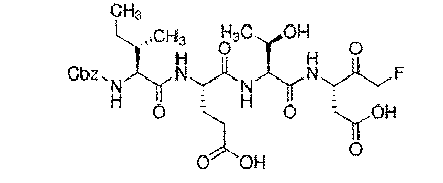 | 654.68 | |
| 2 | Z-Leu-Glu(OMe)-Thr-Asp(OMe)-fluorometh yl ketone (Z-LETD-FMK) | 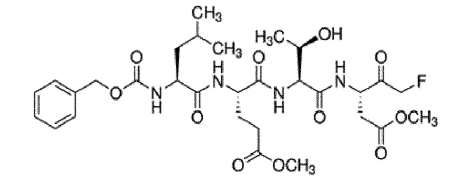 | 655.69 | |
| 3 | N-Acetyl-Ile-Glu-Thr-Asp-al (Ac-IETD-CHO) | 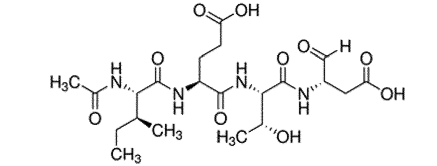 | 502.52 | |

**[Table 74]**

| **Caspase 9 inhibitor** | | | | |
|---|---|---|---|---|
| No. | Name | Structural formula | Molecul ar weight | Protease to be inhibited |
| 1 | Z-Leu-Glu(O-Me)-His-Asp(O-Me)fluorom ethyl ketone (Z-LE (OMe) HD ( OMe) -FMK, Z-LEHD-FMK) | 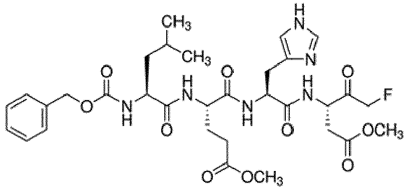 | 690.72 | |

**[Table 75]**

| **Caspase 13 inhibitor** | | | | |
|---|---|---|---|---|
| No. | Name | Structural formula | Molecul ar weight | Protease to be inhibited |
| 1 | Z-Leu-Glu(OMe)-Glu(OMe)-Asp(OMe)-fluorometh yl ketone (Z-LEED-FMK) | 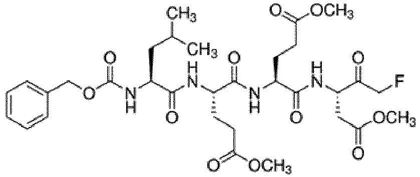 | 696.72 | |

**[Table 76]**

| **Cytosol alanyl aminopeptidase inhibitor** | | | | |
|---|---|---|---|---|
| No. | Name | Structural formula | Molecul ar weight | Protease to be inhibited |
| 1 | Puromycin | 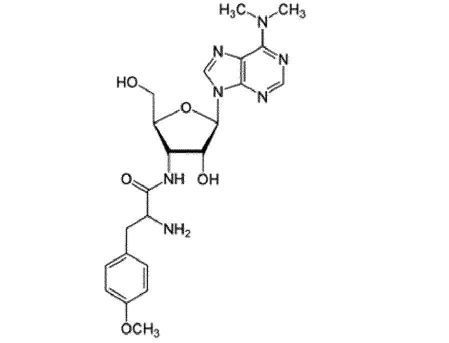 | 471.51 | ▪Dipeptidyl peptidase II |
| | | | | ▪Cytosol alanyl aminopeptidase |
| 2 | Opiorphin | 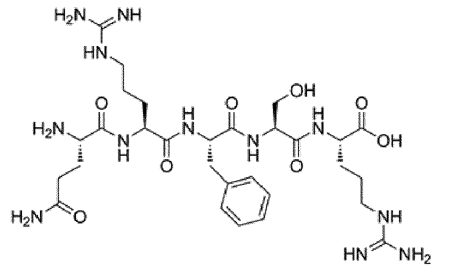 | 692.77 | ▪Enkephalinase |
| | | | | ▪Neprilysin |
| | | | | ▪Dipeptidyl peptidase III |
| | | | | ▪Cytosol alanyl aminopeptidase |

**[Table 77]**

| **Enkephalinase inhibitor** | | | | |
|---|---|---|---|---|
| No. | Name | Structural formula | Molecul ar weight | Protease to be inhibited |
| 1 | Opiorphin | 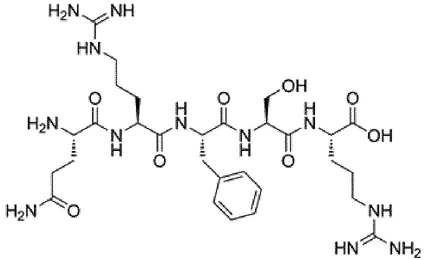 | 692.77 | ▪Enkephalinase |
| | | | | ▪Neprilysin |
| | | | | ▪Dipeptidyl peptidase III |
| | | | | ▪Cytosol alanyl aminopeptidase |

**[Table 78]**

| **Neprilysin inhibitor** | | | | |
|---|---|---|---|---|
| No. | Name | Structural formula | Molecul ar weight | Protease to be inhibited |
| 1 | Opiorphin | 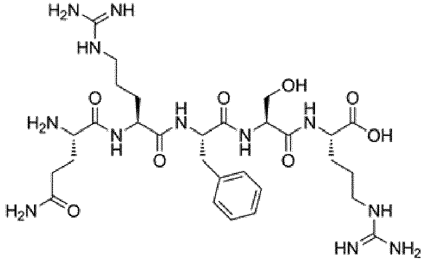 | 692.77 | ▪Enkephalinase |
| | | | | ▪Neprilysin |
| | | | | ▪Dipeptidyl peptidase III |
| | | | | ▪Cytosol alanyl aminopeptidase |

It is noted that in the above descriptions, proteasome inhibitors and protease inhibitors other than the proteasome inhibitors are separately discussed for convenience, but a compound is also known which can inhibit the activities of both a proteasome and a protease other than proteasomes. Therefore, a protein-degradation inducing tag having an affinity with both a proteasome and a protease other than proteasomes can be obtained when such a compound is used.

Examples of the compound which can inhibit the activities of both a proteasome and a protease other than proteasomes are shown in the following table 79. However, the compound which can inhibit the activities of both a proteasome and a protease other than proteasomes shall not be limited to these examples.

**[Table 79]**

| No. | Name | Structural formula | Molecul ar weight | Protease to be inhibited |
|---|---|---|---|---|
| 1 | Calpain Inhibitor I (ALLN, Ac-LLnL-CHO, MG-101 ) | 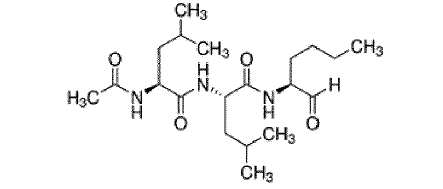 | 383.53 | ▪Proteasome |
| | | | | ▪Cathepsin B |
| | | | | ▪Cathepsin L |
| | | | | ▪Calpine |
| 2 | Calpain Inhibitor II | 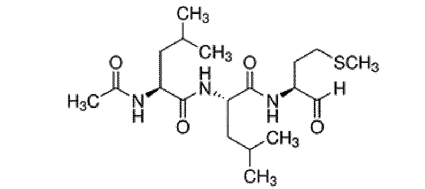 | 401.56 | ▪Proteasome |
| | | | | ▪Cathepsin B |
| | | | | ▪Calpine |
| 3 | Leupeptin | 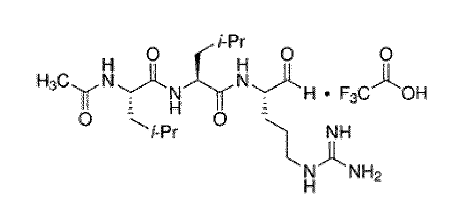 | 426.55 | ▪Plasmin |
| | | | | ▪Trypsin |
| | | | | ▪Papain |
| | | | | ▪Calpine |
| | | | | ▪Cathepsin B |
| | | | | ▪Thrombin |
| | | | | ▪Kallikrein |
| | | | | ▪Endoproteinase |
| | | | | ▪Chymotrypsin |
| | | | | ▪Proteasome (β2) |
| 4 | Chymostati n | 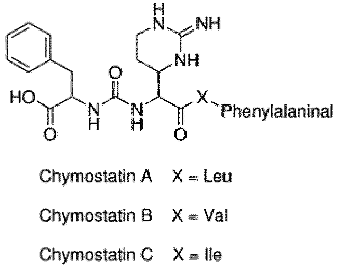 | A: MW = 607.7 | ▪Proteasome (β5) |
| | | | | ▪Chymotrypsin |
| | | | B: MW = 593.7 | ▪Papain |
| | | | | ▪Chymotrypsin-like serine proteinase |
| | | | C: MW = 607.7 | ▪Cathepsin A, B, C, B, H, L |
| 5 | clasto-Lactacysti n β-lactone | 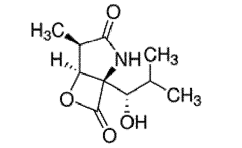 | 213.23 | ▪tripeptidyl peptidase II |
| | | | | ▪chlamydial protease-like activity factor |

In another embodiment, a proteasome activator can be used as a protein-degradation inducing tag. A proteasome activator is a compound having an affinity with a proteasome (a protease complex) without inhibiting degradation of a protein by the proteasome, and can be used as a protein-degradation inducing tag.

Examples of the proteasome activator are shown in the following Tables 80 to 82. However, the proteasome activator which can be used for producing a protein-degradation inducing tag shall not be limited to these examples.

**[Table 80]**

| **20S proteasome activator** | | | |
|---|---|---|---|
| No. | Generic name / Product name | Structural formula | Molecular weight |
| 1 | Oleuropein | 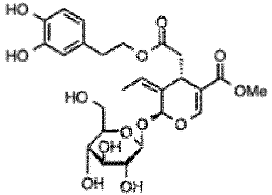 | 540.51 |
| 2 | Betulinic acid | 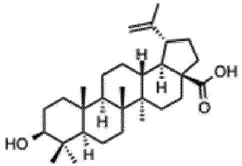 | 456.70 |

**[Table 81]**

| **19S/11S (PA28) proteasome activator** | | | |
|---|---|---|---|
| No. | Generic name / Product name | Structural formula | Molecular weight |
| 1 | IU1 (Usp 14 inhibitor) | 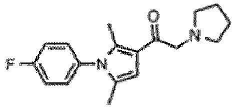 | 300.38 |
| 2 | b-AP-15 (Usp 14 and Uch-L5 inhibitor) | 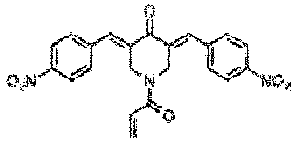 | 419.39 |
| 3 | 17-AAG | 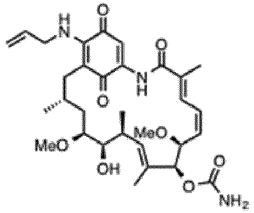 | 585.7 |
| 4 | PU3 | 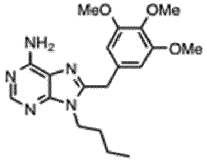 | 371.44 |
| 5 | PU-H71 | 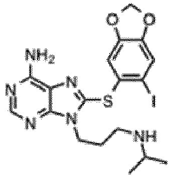 | 512.37 |
| 6 | NVP-AUY922 | 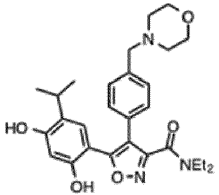 | 493.60 |

**[Table 82]**

| **19S/11S (PA28) proteasome activator (Continued)** | | | |
|---|---|---|---|
| No. | Generic name / Product name | Structural formula | Molecular weight |
| 7 | SNX-5422 | 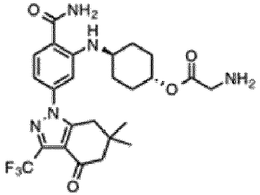 | 521.54 |
| 8 | HBX 19,818 | 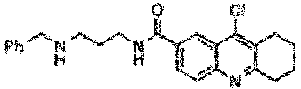 | 407.94 |
| 9 | LS1 | 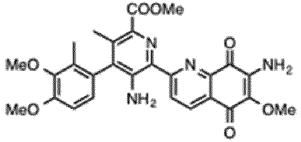 | 518.53 |
| 10 | LDN91946 | 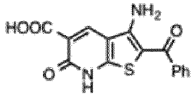 | 314.32 |
| 11 | P005091 | 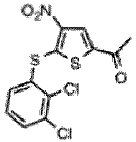 | 348.21 |
| 12 | P0040429 | 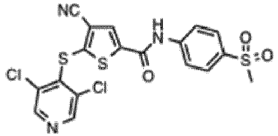 | 484.38 |

Among the protein-degradation inducing tags as mentioned above, in particular, the protein-degradation inducing tag having an affinity with a 26S proteasome is preferable. The intracellular proteasome is generally present in a state of the 26S proteasome in which two 19S proteasomes are bonded to a 20S proteasome. Therefore, use of the protein-degradation inducing tag having an affinity with the 26S proteasome can lead the intracellular specific protein to degradation more efficiently.

### (Specific protein affinity molecule)

A specific protein affinity molecule is a molecule having an affinity with a specific protein.

Examples of the specific protein include proteins residing inside a cell or on a cell membrane. The specific protein may be a mutant protein produced by mutation, or may be a fusion protein produced by translocation and the like. Further, the specific protein may be an endogenous protein, or may be an exogenous protein derived from viruses, bacteria, and the like. Moreover, the specific protein may be a protein which is not promptly degraded, and thus accumulated for some reason. In a certain embodiment, the specific protein is a protein involved in cell cycle, signal transduction, cell differentiation, cell dedifferentiation, cell proliferation, or production of a biologically active substance such as cytokine or the like.

Furthermore, the specific protein may be a complex including a plurality of proteins. Examples of the complex including a plurality of proteins include p53 complexes such as a p53/MDM2 complex (a complex of a p53 protein and an MDM2 protein. The same is true hereinafter), a p53/E6 complex, a p53/HDM2 complex, a p53/AICD complex, a p53/RUNX2 complex, and a p53/RUNX3 complex. However, the complex in the present disclosure is not necessarily limited to these examples.

When the specific protein is a complex including a plurality of proteins, the specific protein affinity molecule may be a protein having an affinity with a part of the proteins constituting the complex, or may be a protein having an affinity with the complex itself.

The below-mentioned molecular kinetics evaluation method of the present disclosure and the screening method of the present disclosure are very useful in being able to evaluate molecular kinetics of a test material (a specific protein affinity molecule or a protein-degradation inducing molecule), and to select a test material (a specific protein affinity molecule or a protein-degradation inducing molecule) showing specific molecular kinetics, using such a complex as mentioned above as a target and using the degradation of the complex as an indicator.

As used herein, the phrase "having an affinity with a specific protein" means the capability of binding to a specific protein, for example, via a covalent bond, a hydrogen bond, a hydrophobic bond, Van der Waals force, and the like. When the interaction between the other molecules that have been known to interact with the specific protein (proteins, peptides, DNA, RNA, metabolites, low molecular weight compounds, and the like) and the specific protein is influenced by a certain molecule in a concentration dependent manner, it can be determined that the molecule has an affinity with the specific protein.

Examples of the specific protein affinity molecule include medicines or medicine candidates such as low molecular weight compounds, antibodies, and peptides; endogenous biologically active substances such as cytokines, growth factors, and hormones; natural products; metabolites; plant ingredients; food ingredients; and the like. Binding molecules (inhibitors and the like) in some types of the specific protein are known (for example, see WO2008/123266), and thus these known molecules can be used as the specific protein affinity molecule. When a molecule capable of binding to a specific protein is unknown, binding molecules may be screened by high throughput screening (HTS). Alternatively, an antibody capable of binding to a specific protein may be produced, which may be used as the specific protein affinity molecule. (Form of conjugate of protein-degradation inducing tag and specific protein affinity molecule)

There is no particular limitation for the form of a conjugate of the protein-degradation inducing tag and the specific protein affinity molecule as long as the binding property for the protease of the protein-degradation inducing tag and the affinity of the specific protein affinity molecule with the specific protein are maintained. It is noted that when both the protein-degradation inducing tag and the specific protein affinity molecule are proteins, the both proteins can be fused to each other to synthesize a fusion protein, but such fusion proteins are not included in the "conjugate".

The protein-degradation inducing molecule may have, for example, a structure in which at least one protein-degradation inducing tag is linked to at least one specific protein affinity molecule. The protein-degradation inducing molecule may have a structure in which one protein-degradation inducing tag is linked to one specific protein affinity molecule, or may have a structure in which one protein-degradation inducing tag is linked to a plurality of specific protein affinity molecules, a structure in which a plurality of protein-degradation inducing tags are linked to one specific protein affinity molecule, or may have a structure in which a plurality of protein-degradation inducing tags are linked to a plurality of specific protein affinity molecule. In a certain embodiment, the protein-degradation inducing molecule has a structure in which one protein-degradation inducing tag is linked to one specific protein affinity molecule.

A position in the protein-degradation inducing tag at which the specific protein affinity molecule is linked to the protein-degradation inducing tag is not particularly limited as long as the affinity with a protease is maintained. For example, when the protein-degradation inducing tag has, as described above, a structure in which the active site of a protease inhibitor (for example, a proteasome inhibitor) is replaced with another structural moiety, the protein-degradation inducing tag can be linked to the specific protein affinity molecule at this replaced other structural moiety. Specifically, when the active site of the protease inhibitor is replaced with a carboxy group, the protein-degradation inducing tag can be linked to the specific protein affinity molecule via a carboxy group. Meanwhile, a position in the specific protein affinity molecule at which the protein-degradation inducing tag is linked to the specific protein affinity molecule is not particularly limited as long as the affinity with the specific protein is maintained.

It is noted that the protein-degradation inducing tag and the specific protein affinity molecule may have a structure in which they can be linked to each other. When it is difficult to directly link the protein-degradation inducing tag to the specific protein affinity molecule, it is considered that a structure capable of linking them to each other is introduced into at least one of the protein-degradation inducing tag and the specific protein affinity molecule. For example, as the specific protein affinity molecule, a well-known molecule having an affinity with the specific protein can be used, but it is assumed to be difficult to directly link this well-known molecule to the protein-degradation inducing tag. In such a case, a structure which can be linked to the protein-degradation inducing tag may be introduced into the well-known molecule, and used as the specific protein affinity molecule.

### <Molecular kinetics evaluation method>

The molecular kinetics evaluation method of the present disclosure includes a step of administering the protein-degradation inducing molecule to a human or a non-human animal, and inducing degradation of a specific protein in a living body of the human or the non-human animal (hereinafter, also referred to as a "degradation inducing step"); and a step of evaluating molecular kinetics of the specific protein affinity molecule or the protein-degradation inducing molecule by detecting degradation of the specific protein in a specimen being at least a portion of the human or the non-human animal (hereinafter, also referred to as a "molecular kinetics evaluation step"). The molecular kinetics evaluation method of the present disclosure enables evaluation of the molecular kinetics of the specific protein affinity molecule or the protein-degradation inducing molecule.

In the degradation inducing step, the above-described protein-degradation inducing molecule is administered to a human or a non-human animal. With this administration, the specific protein can be led to degradation (knockdown) by a protease (for example, a proteasome) in a living body of a human or a non-human animal without ubiquitination of the specific protein (that is, in a ubiquitin-independent manner).

The non-human animal is not particularly limited, and examples thereof include a primate such as a monkey; a mouse; a rat; a pig; a dog; and a cat. The non-human animal may be a genetically modified animal in which a gene is modified (for example, a disease model animal), or a wild-type animal in which a gene is not modified. Furthermore, the administration method of the protein-degradation inducing molecule is not particularly limited, and may be oral administration or parenteral administration (intravenous administration, intraarterial administration, portal administration, intradermal administration, subcutaneous administration, intraperitoneal administration, intrathoracic administration, intrathecal administration, intramuscular administration, and the like).

The molecular kinetics evaluation step evaluates molecular kinetics of a specific protein affinity molecule or a protein-degradation inducing molecule by detecting degradation of the specific protein in a specimen being at least a portion of the human or the non-human animal. In a specimen in which a protein-degradation inducing molecule is transferred, degradation of the specific protein is induced. Therefore, by detecting the degradation of the specific protein, the molecular kinetics of the specific protein affinity molecule or the protein-degradation inducing molecule can be evaluated.

The "specimen being at least a portion of the human or the non-human animal" means tissues, organs, cells, molecules, and the like, collected from the human or the non-human animal. Examples of the specimen include the whole of or a part of an organ, skin, blood, a cell included therein, a molecule included therein, or the like. A method for collecting the specimen is not particularly limited, and examples of specimen-collecting methods that have been usually used include a method for collecting specimens in biopsy (for example, a method for collecting a specimen using an endoscope or a forceps catheter), a method for collecting a specimen by surgical operation, and the like. In the case of non-human animals, the specimen may be collected by dissection or the like.

The method for detecting degradation of the specific protein in the specimen is not particularly limited. For example, an amount of the specific protein may be measured by Western blot analysis and the like. Alternatively, degradation of the specific protein may be indirectly detected by measuring the amount of another protein whose expression amount is changed by degradation of the specific protein, by Western blot analysis and the like. Furthermore, degradation of the specific protein may be indirectly detected by measuring mRNA whose expression amount is changed by degradation of the specific protein, by an RT-PCR method and the like. It is noted that when the specific protein is a complex, the degradation of the specific protein may be detected by detecting a part of proteins constituting the complex, or by detecting all of the proteins constituting the complex. Since it is sufficient in the molecular kinetics evaluation step to detect the degradation of the specific protein, it is not necessary to use HPLC, LC-MS/MS, autoradiography, and the like, which have conventionally been used.

A test material to be evaluated in terms of the molecular kinetics may be a specific protein affinity molecule or a protein-degradation inducing molecule.

When the molecular kinetics of the specific protein affinity molecule are evaluated, a specific protein affinity molecule is combined with a protein-degradation inducing tag to produce a protein-degradation inducing molecule, and the protein-degradation inducing molecule may be administered to a human or a non-human animal. Since the degradation of a specific protein is induced in a specimen in which the protein-degradation inducing molecule is transferred, the molecular kinetics of the specific protein affinity molecule can be evaluated by detecting the degradation. In a certain embodiment, drug candidate molecules such as a low molecular weight compound, an antibody, and a peptide can be used as the specific protein affinity molecule.

Furthermore, when the molecular kinetics of the protein-degradation inducing molecule are evaluated, the protein-degradation inducing molecule may be administered to a human or a non-human animal. Since the degradation of the specific protein is induced in a specimen in which the protein-degradation inducing molecule is transferred, the molecular kinetics of the protein-degradation inducing molecule can be evaluated by detecting the degradation.

Here, when the specific protein is a disease-related protein, the protein-degradation inducing molecule can be a drug candidate molecule for the disease. In this case, distribution of the protein-degradation inducing molecule in a living body of the human or the non-human animal represents distribution of places where a pharmacological action is expressed. Therefore, the target disease or patient can be narrowed down by evaluating the molecular kinetics of the protein-degradation inducing molecule. As an example, when the specific protein is a cancer-related protein it is possible to evaluate what cancer the drug candidate molecule is for by evaluating the molecular kinetics of the protein-degradation inducing molecule.

It is noted that when the specific protein is a disease-related protein, the molecular kinetics evaluation method of the present disclosure may further include a step of evaluating pharmacological action by inducing degradation of the specific protein in the living body of the human or the non-human animal (hereinafter, referred to as a "pharmacological action evaluation step"). This makes it possible to evaluate the molecular kinetics of the protein-degradation inducing molecule and the pharmacological action together. The order of the molecular kinetics evaluation step and the pharmacological action evaluation step is not particularly limited.

### <Screening method>

The screening method of the present disclosure includes a step of administering the protein-degradation inducing molecule to a human or a non-human animal and inducing degradation of the specific protein in a living body of the human or the non-human animal (hereinafter, also referred to as a "degradation inducing step"), and a step of selecting a specific protein affinity molecule or a protein-degradation inducing molecule showing specific molecular kinetics by detecting degradation of the specific protein in a specimen being at least a portion of the human or the non-human animal (hereinafter, also referred to as a "selecting step"). The screening method of the present disclosure enables selection of the specific protein affinity molecule or the protein-degradation inducing molecule showing specific molecular kinetics.

Since the degradation inducing step is the same as the above-described molecular kinetics evaluation method of the present disclosure, detailed description is omitted.

In the selecting step, a specific protein affinity molecule or a protein-degradation inducing molecule showing specific molecular kinetics is selected by detecting degradation of the specific protein in a specimen being at least a portion of the human or the non-human animal. In a specimen in which the protein-degradation inducing molecule is transferred, the degradation of the specific protein is induced. Therefore, by detecting the degradation, it is possible to select the specific protein affinity molecule or the protein-degradation inducing molecule showing specific molecular kinetics.

Since the method for detecting the degradation of specimen and specific protein in the specimen is the same as the above-described molecular kinetics evaluation method of the present disclosure, detailed description is omitted. Since it is sufficient in the selecting step to detect degradation of the specific protein, it is not necessary to use HPLC, LC-MS/MS, autoradiography, or the like, and therefore it is easy.

The test material to be selected may be a specific protein affinity molecule or may be a protein-degradation inducing molecule.

When a specific protein affinity molecule showing specific molecular kinetics is selected, a specific protein affinity molecule is combined with a protein-degradation inducing tag to produce a protein-degradation inducing molecule, and the protein-degradation inducing molecule may be administered to a human or a non-human animal. Since the degradation of a specific protein is induced in a specimen in which the protein-degradation inducing molecule is transferred, a specific protein affinity molecule showing specific molecular kinetics can be selected by detecting this degradation. In a certain embodiment, drug candidate molecules such as a low molecular weight compound, an antibody, and a peptide can be used as the specific protein affinity molecule.

Furthermore, when a protein-degradation inducing molecule showing specific molecular kinetics is selected, the protein-degradation inducing molecule is only required to be administered to a human or a non-human animal. Since the degradation of a specific protein is induced in a specimen in which the protein-degradation inducing molecule is transferred, a protein-degradation inducing molecule showing specific molecular kinetics can be selected by detecting this degradation.

### EXAMPLES

Below, the present invention will be described specifically with reference to Examples, but the present invention shall not be limited to these Examples. In the following Examples, Synthesis Examples, and Reference Examples, room temperature indicates temperatures in a range of 20ºC to 30ºC.

Abbreviations of compounds used in the following Examples, Synthesis Examples, and Reference Examples are as follows.
H-Gly-OtBu·HCl: L-Glycine t-butyl ester hydrochloride
DMF: N,N-Dimethylformamide
DIPEA: N,N-Diisopropylethylamine
PyBOP: 1H-Benzotriazol-1-yloxy-tri(pyrrolidino)phosphonium hexafluorophosphate
TFA: Trifluoroacetic acid
H-Leu-OtBu·HCl: L-Leucine t-butyl ester hydrochloride
HATU: 0-(7-Azabenzotriazol-1-yl)-N,N,N',N'-tetramethyluronium hexafluorophosphate
ec: Escherichia coli
DHFR: Dihydrofolate reductase
RF: Restriction-free
HA: Hemagglutinin
GFP: Green fluorescent protein
DsRed: Discosoma sp. red fluorescent protein
D-MEM: Dulbecco's modified eagle's medium
DMSO: Dimethyl sulfoxide
PBS: Phosphate buffered saline
EDTA: Ethylenediamine tetraacetic acid
FBS: Fetal bovine serum
SDS: Sodium dodecyl sulfate
PAGE: Polyacrylamide gel ectrophoresis
BPB: Bromophenol blue
PVDF: Polyvinylidene difluoride
TBS: Tris buffered saline
GAPDH: Glyceraldehyde 3-phosphate dehydrogenase
PMSF: Phenylmethylsulfonyl fluoride
DTT: Dithiothreitol
TMP: Trimethoprim
DMT-MM: 4-(4,6-Dimethoxy-1,3,5-triazin-2-yl)-4-methylmorpholinium chloride n-hydrate
AMC: 7-Amino-4-methylcoumarin
MTX: Methotrexate
DMA: N,N-Dimethylacetamide
BOP: (Benzotriazol-1-yloxy)-tris(dimetylamino)phosphonium hexafluorophosphate
DEPC: Diethylpyrocarbonate

### <Synthesis Example 1: Synthesis of TUS-007>

In Synthesis Example 1, a protein-degradation inducing tag and a specific protein affinity molecule having an affinity with a Ras protein were linked to each other to synthesize TUS-007 as a protein-degradation inducing molecule.

As the protein-degradation inducing tag, a compound (CANDDY_MLN) in which active sites of MLN9708 and MLN2238 as the proteasome inhibitors (a boronic acid ester moiety or a boronyl group) were replaced with a carboxy group was used.

Furthermore, Ras-SOS-NH₂ represented by the following formula was used as the specific protein affinity molecule. Ras-SOS-NH₂ is a compound obtained by reacting an amino group of Ras-SOS represented by the following formula with H₂N-(CH₂)₆-COOH.

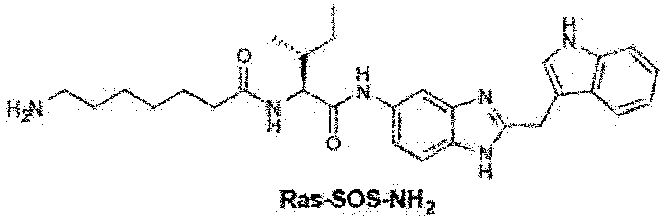

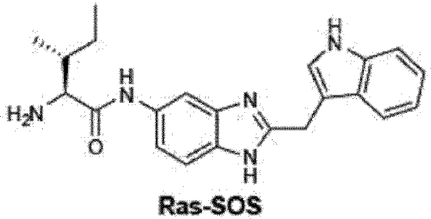

Ras-SOS is Compound 12 described in the document by Sun, Q. et al. (Sun, Q. et al., Angew. Chem. Int. Ed., 2012, 51, 6140-6143). When a SOS protein is bound to the Ras protein, GDP bound to the Ras protein is replaced with GTP, and the Ras protein is activated. It is known that Ras-SOS is bound to the Ras protein to inhibit the interaction between the Ras protein and the SOS protein, thus inhibiting the activation of the Ras protein.

It is noted that Ras-SOS and Ras-SOS-NH₂ were synthesized according to the method described in the document by Sun, Q. et.al.

The method of synthesizing TUS-007 is described in detail as follows.

### (Synthesis of CANDDY_MLN)

CANDDY_MLN was synthesized according to the following synthesis scheme.

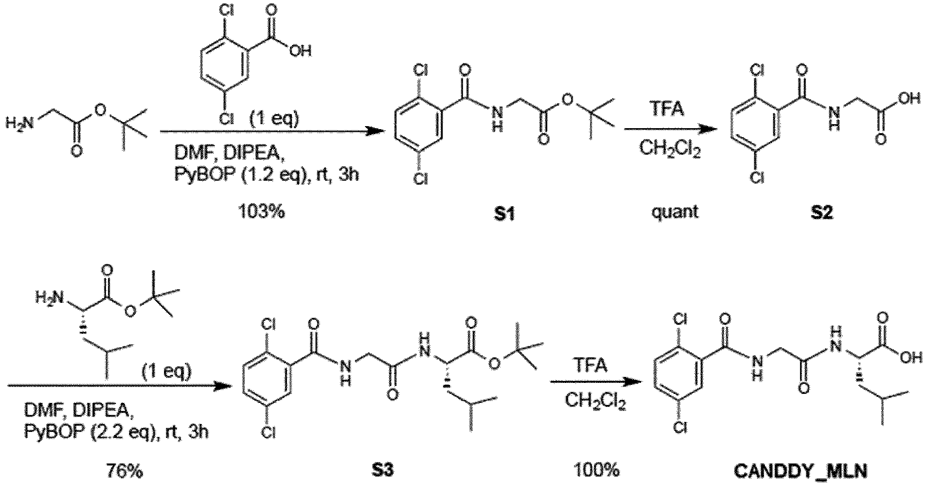

First, H-Gly-OtBu·HCl (286.8 mg, 1.69 mmol, 1 eq) was charged into a side-arm eggplant flask, and purged with nitrogen. Under nitrogen gas stream, 10 mL of dehydrate DMF and 5 mL of DIPEA were added, and stirred at room temperature. In 1 mL of dehydrate DMF and 1 mL of DIPEA, 2,5-dichlorobenzoic acid (309.3 mg, 1.62 mmol, 1 eq) was dissolved, which was then added to the reaction solution, and the resultant solution was stirred at room temperature for 20 minutes. PyBOP (1.02 g, 1.96 mmol, 1.2 eq) was dissolved in 1 mL of dehydrate DMF, then added to the reaction solution, and stirred at room temperature for 3 hours. The reaction solution was diluted with water and aqueous sodium hydrogen carbonate, and extracted twice with ethyl acetate/hexane (= 4/1). After being dried over anhydrous sodium sulfate, the solvent was evaporated under reduced pressure. Separation and purification treatment was performed by silica gel chromatography (hexane/chloroform = 1/1 to 0/1, gradient) to obtain a compound S1 (531.0 mg, 1.75 mmol, 103%).

Next, the compound S1 (212.4 mg, 0.70 mmol) was charged into an eggplant flask, and 5 mL of dichloromethane was then added. This was stirred at room temperature for 5 minutes, then 5 mL of TFA was added thereto, and the resultant solution was stirred at room temperature for one hour. After evaporating the solvent under reduced pressure, vacuum drying was performed to obtain a compound S2 (190.7 mg, quant.).

Next, the compound S2 (190.7 mg, 0.77 mmol, 1 eq) and H-Leu-OtBu·HCl (175.8 mg, 0.79 mmol, 1 eq) were charged into a side-arm eggplant flask, and purged with nitrogen. Under nitrogen gas stream, 5 mL of dehydrate DMF and 5 mL of DIPEA were added, and stirred at room temperature for 20 minutes. PyBOP (886.7 mg, 1.70 mmol, 2.2 eq) was dissolved in 1.5 mL of dehydrate DMF, then the resultant solution was added to the reaction solution and stirred at room temperature for 3 hours. The reaction solution was diluted with water and aqueous sodium hydrogen carbonate, and extracted twice with ethyl acetate/hexane (= 4/1). After being dried over anhydrous sodium sulfate, the solvent was evaporated under reduced pressure. Separation and purification treatment was performed by silica gel chromatography (hexane/chloroform = 1/1 to 0/1, gradient) to obtain a compound S3 (244.2 mg, 0.58 mmol, 76%).

Next, the compound S3 (240.8 mg, 0.58 mmol) was charged into an eggplant flask, and 5 mL of dichloromethane was added. This was stirred at room temperature for 5 minutes, and then 5 mL of TFA was added, and stirred at room temperature for 1 hour. After evaporating the solvent under reduced pressure, vacuum drying was performed to obtain CANDDY_MLN (214.7 mg, 0.59 mmol, 100%).

### (Synthesis of TUS-007)

TUS-007 was synthesized according to the following synthesis scheme.

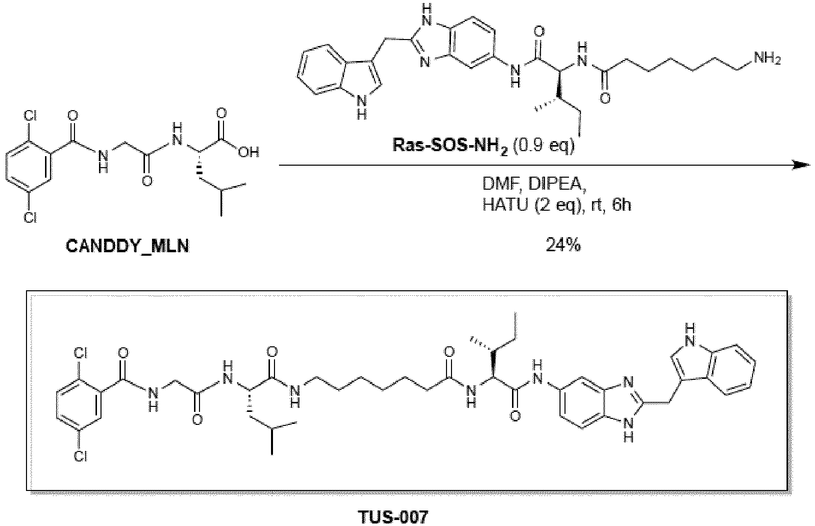

CANDDY_MLN (52.4 mg, 0.15 mmol, 1 eq) and separately synthesized Ras-SOS-NH₂ (62.4 mg, 0.12 mmol, 0.9 eq) were charged into an eggplant flask, and 4 mL of dehydrate DMF was then added. After the resultant solution was stirred at room temperature for 5 minutes, 4 mL of DIPEA was then added to neutralize the solution. After the resultant solution was stirred at room temperature for 5 minutes, HATU (114.1 mg, 0.30 mmol, 2 eq) was directly added to a reaction solution, and the reaction solution was stirred at room temperature for 6 hours. Under cooling, a saturated sodium hydrogen carbonate aqueous solution was added, an organic layer was separated, and then, a water layer was extracted with ethyl acetate. Organic layers were collected, and dried over anhydrous sodium sulfate. After the solvent was evaporated under reduced pressure, a separation refining process using silica gel chromatography (chloroform/methanol = 20/1 to 4/1, gradient) was performed to obtain TUS-007 (25.2 mg, 0.03 mmol, 24%, isolated yield). The obtained TUS-007 was further purified by preparative thin layer chromatography (chloroform/methanol = 10/1). The physical property data of TUS-007 are shown as follows. HRMS-FAB (m/z): [M+H]⁺ calcd for C₄₄H₅₅C₁₂N₈O₅, 845.3672; found, 845.3674.

### <Reference Example 1>

In Reference Example 1, degradation (knockdown) of a wild-type K-Ras protein forcibly expressed in HeLa cells (human cervical cancer cells) through TUS-007 was evaluated by FACS analysis.

### (Preparation of plasmid)

A plasmid expressing a wild-type K-Ras protein (K-Ras-WT) was prepared using a plasmid (pMIR-DsRed-IRES-ecDHFR-HA-GFP) expressing an ecDHFR protein by RF cloning. The full-length cDNA clone (Accession No. AK292510) of a human K-ras gene was purchased from Independent Administrative Institution, the National Institute of Technology and Evaluation. PCR amplification was performed using KOD-Plus-Neo (TOYOBO CO., LTD) as a PCR enzyme. Forward primers and reverse primers used for RF cloning are shown in Table 83.

**[Table 83]**

| Primer name | Sequence (5'->3') | SEQ ID No. |
|---|---|---|
| RFC_IRES-HsKras-HA_Fw | CACGATGATAATATGGCCACAACCATGACTGAATA TAAACTTGTGGTAG | 1 |
| RFC_IRES-HsKras-HA_Rv | GAACGTCGTACGGGTAATCGATCATAATTACACAC TTTGTCTTTGAC | 2 |

### (Introduction of plasmid into HeLa cells and cell seeding)

The plasmid was introduced into HeLa cells to transiently overexpress a wild-type K-Ras protein (specifically, a fusion protein of a wild-type K-Ras protein and GFP via a HA tag) or a DsRed protein for comparison in the cells.

ScreenFect™A (Wako Pure Chemical Industries, Ltd.) as a transfection reagent was used to introduce the plasmid into HeLa cells by a routine procedure. The HeLa cells into which the plasmid had been introduced were seeded in a 24-well plate at a cell density of 4 x 10⁴ cells/well, and then cultured under conditions of 37ºC and 5 vol% CO₂ for 40 hours.

### (Addition of TUS-007 to HeLa cells)

Culture was performed for 40 hours after introduction of the plasmid, and then TUS-007 was added to HeLa cells as follows. As a medium, a serum-free medium (37ºC) in which 1 mass% L-glutamine solution (Sigma-Aldrich) was added to D-MEM (high D-glucose, phenol red, sodium pyruvate (Wako Pure Chemical Industries, Ltd.)) was used. It is noted that the L-glutamine solution was added immediately before use. A DMSO solution containing TUS-007 was mixed with the medium so that the concentration of DMSO was 1 vol%, and added to each well at 500 µL/well, and cultured under conditions of 37ºC and 5 vol% CO₂. Furthermore, in addition to an experiment group in which a DMSO solution containing TUS-007 had been added, an experiment group in which a DMSO solution containing both TUS-007 and MLN2238, or Ras-SOS-NH₂ had been added was prepared. It is noted that DMSO was used as a control.

### (Evaluation of degradation (knockdown) of wild-type K-Ras protein through TUS-007 (FACS analysis))

The medium was removed 24 hours after addition of TUS-007, and then PBS was added to wash the cells. After removing PBS, trypsin (0.25 w/v% Trypsin-1 mmol/L EDTA·4 Na solution with phenol red) (Wako Pure Chemical Industries, Ltd.) at 37ºC was added to each well at 200 µL/well, and cultured under conditions of 37ºC and 5 vol% CO₂ for 1 minute. After culturing, a medium where 10 mass% FBS and 1 mass% PenStrep (100 U/mL sodium penicillin G and 100 µg/mL streptomycin sulfate) (Wako Pure Chemical Industries, Ltd.) were added to D-MEM (low D-glucose, L-glutamine, phenol red) (Wako Pure Chemical Industries, Ltd.) was added to each well at 300 µL/well, and suspended, and then a cell solution was collected in a 15 mL tube.

The cell solution collected was centrifuged (at 1000 rpm x 5 minutes, 4ºC), and the supernatant was removed, and then suspended in 2 ml of PBS (37ºC). The cell solution after suspension was centrifuged (at 1000 rpm x 5 minutes, 4ºC), and the supernatant was removed, and then 500 µL of an FACS buffer (1 mass% FBS/PBS) at 4ºC was added, and allowed to stand on ice.

A BD FACSCanto™ II (BD Biosciences) was used for flow cytometry, and the expression levels of GFP and DsRed protein in the cells were quantified. The cell solution was passed through a mesh with a pore size of 32 µm, and transferred to an FACS tube immediately before FACS analysis. The GFP/DsRed ratio per cell was computed using an analysis software FlowJo™ (TOMY Digital Biology Co., Ltd.), and degradation (knockdown) of the wild-type K-Ras protein by TUS-007 was determined from a shift in a graph.

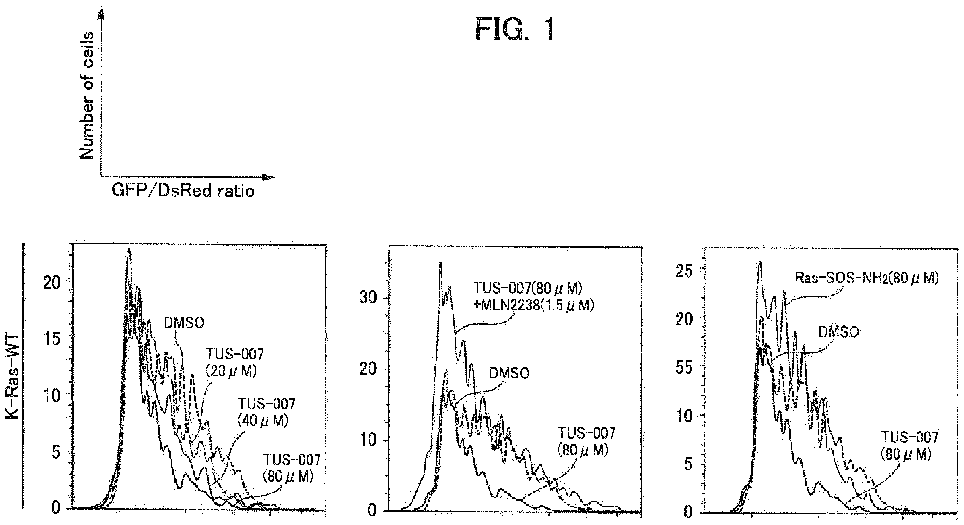

The results of the FACS analysis are shown in Fig. 1. As shown in Fig. 1, when TUS-007 was added, the graph is shifted toward the left in a concentration-dependent manner, demonstrating that degradation of the wild-type K-Ras protein was induced by TUS-007. On the other hand, when Ras-SOS-NH₂ was added, the graph is overlapped to that of a control (DMSO), demonstrating that the wild-type K-Ras protein was not degraded. From this result, it is found that the degradation of the wild-type K-Ras protein is induced by linking CANDDY_MLN as a protein-degradation inducing tag to Ras-SOS-NH₂. Furthermore, when both TUS-007 and MLN2238 were added, degradation of the wild-type K-Ras protein was inhibited as compared with the case where TUS-007 was added. This result supports that TUS-007 leads the wild-type K-Ras protein to the degradation by a proteasome.

### <Reference Example 2>

In Reference Example 2, degradation (knockdown) of a forcibly expressed wild-type K-Ras protein in HeLa cells through TUS-007 was evaluated by Western blot analysis.

### (Preparation of plasmid)

A plasmid expressing the wild-type K-Ras protein (K-Ras-WT) was prepared, as in Reference Example 1.

### (Introduction of plasmid into HeLa cells and cell seeding)

As in Reference Example 1, the plasmid was introduced into HeLa cells to transiently overexpress the wild-type K-Ras protein (specifically, a fusion protein of the wild-type K-Ras protein and GFP through a HA tag) or a DsRed protein for comparison in the cells. HeLa cells into which the plasmid had been introduced were seeded in a 24-well plate at a cell density of 4 x 10⁴ cells/well, and then cultured under conditions of 37ºC and 5 vol% CO₂ for 40 hours.

### (Addition of TUS-007 to HeLa cells)

Culture was performed for 40 hours after introduction of the plasmid, and then TUS-007 was added to HeLa cells as follows. As a medium, a serum-free medium (37ºC) in which 1 mass% L-glutamine solution (Sigma-Aldrich) was added to D-MEM (high D-glucose, phenol red, sodium pyruvate (Wako Pure Chemical Industries, Ltd.)) was used. It is noted that the L-glutamine solution was added immediately before use. A DMSO solution containing TUS-007 was mixed with the medium so that the concentration of DMSO was 1 vol%, and added to each well at 500 µL/well, and cultured under conditions of 37ºC and 5 vol% CO₂. Furthermore, in addition to an experiment group in which a DMSO solution containing TUS-007 had been added, an experiment group in which a DMSO solution containing both TUS-007 and MLN2238, MLN2238 or Ras-SOS-NH₂ had been added was prepared. It is noted that DMSO was used as a control.

### (Evaluation of degradation (knockdown) of Wild-type K-Ras protein through TUS-007 (Western blot analysis))

The medium was removed 24 hours after addition of TUS-007, and then PBS was added to wash the cells. After removing PBS, a mixed solution of a cell lysis buffer (CelLytic™ M, Sigma) and a protease inhibitor (cOmplete™ Mini, EDTA-free, Roche) was added to each well at 27 µL/well. After being allowed to stand at 4ºC for 15 minutes, cells were detached with a pipette tip on ice. A cell solution was collected in a 1.5 mL tube, and flash frozen in liquid nitrogen, and then thawed on ice. After repeating this freeze-thaw cycle for three times, the solution was centrifuged (at 13800 rpm x 20 minutes, 4ºC), and the supernatant (cell extract) was collected.

The cell extract collected was subjected to Western blot analysis. An SDS-PAGE gel was prepared using TGX™ FastCast™ Acrylamide Kit, 12% (Bio-Rad). Electrophoresis samples were prepared in a 6x SDS-PAGE sample buffer (62.5 mM Tris-HCl pH 6.8, 2% SDS, 5% 2-mercaptoethanol, 10% glycerol, 0.25% BPB), and placed on a heat block at 95ºC for 4 minutes. Electrophoresis was performed at 150 V for 50 minutes (electrophoresis buffer; 195 mM glycine, 25 mM Tris).

After electrophoresis, proteins were transferred to a PVDF membrane (Immobion™-P, Millipore) under conditions of 100 V and 120 minutes using a tank-type blotting device and a transfer buffer (25 mM Tris-HCl, 195 mM glycine, 0.01% SDS, 15% methanol). The membrane after transfer was shaken and blocked at room temperature for 30 minutes in 5% skim milk/high-salt TBS-T (100 mM Tris-HCl, 500 mM NaCl, 0.2% Tween-20, pH 7.6). After blocking, the membrane was rinsed with high-salt TBS-T, and an antibody reaction was performed in 1% skim milk/high-salt TBS-T. As the antibody, anti-HA-peroxidase, high-affinity (3F10) Rat monoclonal antibody (25 U/mL) (Roche) diluted 1000 times was used. The membrane was shaken at room temperature for one hour, and then washed with high-salt TBS-T for 5 minutes. It is noted that washing was performed three times. Further, the membrane was washed with high-salt TBS (100 mM Tris-HCl, 500 mM NaCl, pH 7.6) for 5 minutes. Subsequently, the membrane was treated with a chemiluminescence reagent Immobilon™ Western (Millipore), and then chemiluminescence was detected using a lumino image analyzer LAS-3000 (FUJIFILM Corporation).

Next, a reaction for detecting GAPDH as a control was performed using the same membrane. The membrane was washed with TBS-T (100 mM Tris-HCl, 150 mM NaCl, 0.1% Tween-20, pH 7.6), and shaken and blocked in 5% skim milk/TBS-T at room temperature for 30 minutes. After blocking, a primary antibody reaction was performed in 5% skim milk/TBS-T. As the primary antibody, anti-GAPDH antibody (6C5, SantaCruz, diluted 20000 times). The membrane was shaken at room temperature for 60 minutes, and then washed with TBS-T for 5 minutes. It is noted that washing was performed three times. After the primary antibody reaction, a secondary antibody reaction was performed in 2% skim milk/TBS-T. As the secondary antibody, an anti-mouse IgG (H+L) antibody (A90-116P-33, Bethyl) diluted 20000 times was used. The membrane was shaken at room temperature for 30 minutes, and then washed with TBS-T for 5 minutes. It is noted that washing was performed three times. Further, the membrane was washed with TBS (100 mM Tris-HCl, 150 mM NaCl, pH 7.6) for 5 minutes. Subsequently, the membrane was treated with a chemiluminescence reagent Immobilon™ Western (Millipore), and then chemiluminescence was detected using a lumino image analyzer LAS-3000 (FUJIFILM Corporation). Detected bands were quantified with an image processing software ImageJ (NIH).

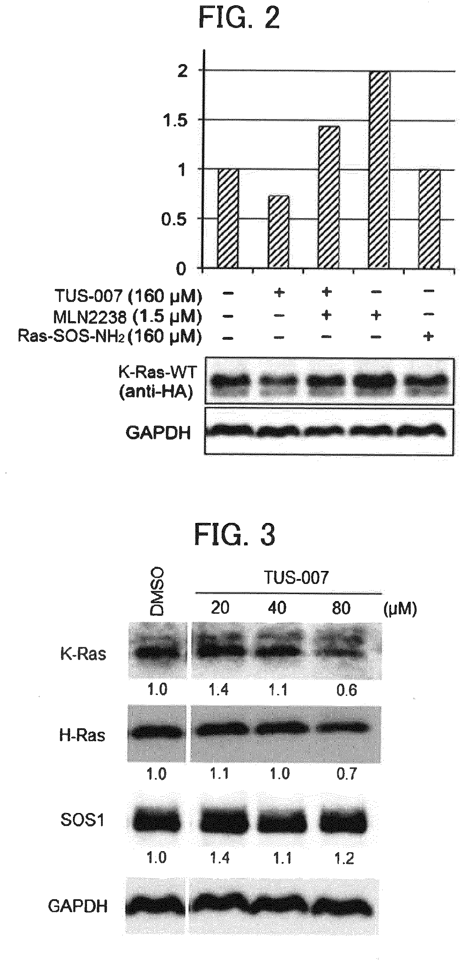

The results of the Western blot analysis are shown in Fig. 2. The graph in Fig. 2 shows the quantification result of the wild-type K-Ras protein detected by the Western blot analysis as a relative value when the value of the control (DMSO) was defined as 1. As shown in Fig. 2, when TUS-007 was added, the amount of the wild-type K-Ras protein was reduced, but when Ras-SOS-NH₂ was added, the amount of the wild-type K-Ras protein was not reduced. From this result, it is found that the wild-type K-Ras protein degradation was induced by linking CANDDY_MLN as a protein-degradation inducing tag to Ras-SOS-NH₂. Furthermore, when both TUS-007 and MLN2238 were added, the amount of the wild-type K-Ras protein was increased as compared with the amount of the control (DMSO). This result supports that TUS-007 leads the wild-type K-Ras protein to the degradation by a proteasome.

### <Reference Example 3>

In Reference Example 3, degradation (knockdown) of an endogenous wild-type K-Ras protein and wild-type H-Ras protein in HeLa cells to which TUS-007 had been added was evaluated by Western blot analysis.

### (Cell seeding)

HeLa cells were seeded in a 24-well plate at a cell density of 8 x 10⁴ cells/well, and then cultured under conditions of 37ºC and 5 vol% CO₂ for 16 hours.

### (Addition of TUS-007 to HeLa cells)

After 16 hours from cell seeding, TUS-007 was added to HeLa cells as follows. As a medium, a serum-free medium (37ºC) in which 1 mass% L-glutamine solution (Sigma-Aldrich) was added to D-MEM (high D-glucose, phenol red, sodium pyruvate (Wako Pure Chemical Industries, Ltd.)) was used. It is noted that the L-glutamine solution was added immediately before use. A DMSO solution containing TUS-007 was mixed with the medium so that the concentration of DMSO was 1 vol%, and added to each well at 500 µL/well, and cultured under conditions of 37ºC and 5 vol% CO₂. As a control, DMSO was used.

### (Evaluation of degradation (knockdown) of endogenous wild-type K-Ras protein and wild-type H-Ras protein through TUS-007 (Western blot analysis))

The medium was removed 48 hours after addition of TUS-007, and then PBS was added to wash the cells. After removing PBS, a mixed solution of a cell lysis buffer (CelLytic™ M, Sigma) and a protease inhibitor (cOmplete™ Mini, EDTA-free, Roche) was added to each well at 27 µL/well. After being allowed to stand at 4ºC for 15 minutes, cells were detached with a pipette tip on ice. A cell solution was collected in a 1.5 mL tube, and flash frozen in liquid nitrogen, and then thawed on ice. After thawing, the solution was centrifuged (at 13800 rpm x 20 minutes, 4ºC), and the supernatant (cell extract) was collected.

The cell extract collected was subjected to Western blot analysis. An SDS-PAGE gel was prepared using TGX™ FastCast™ Acrylamide Kit, 12% (Bio-Rad). Electrophoresis samples were prepared in a 6x SDS-PAGE sample buffer (62.5 mM Tris-HCl pH 6.8, 2% SDS, 5% 2-mercaptoethanol, 10% glycerol, 0.25% BPB), and placed on a heat block at 95ºC for 4 minutes. Electrophoresis was performed at 150 V for 50 minutes (electrophoresis buffer; 195 mM glycine, 25 mM Tris).

After electrophoresis, proteins were transferred to a PVDF membrane (Immobion™-P, Millipore) under conditions of 100 V and 2 hours using a tank-type blotting device and a transfer buffer (25 mM Tris-HCl, 195 mM glycine, 0.01% SDS, 15% methanol). The membrane after transfer was shaken and blocked at room temperature for 30 minutes in 5% skim milk/TBS-T (100 mM Tris-HCl, 150 mM NaCl, 0.1% Tween-20, pH 7.6). After blocking, a primary antibody reaction was performed in 5% skim milk/ TBS-T. As the primary antibody, an anti-K-Ras antibody (C-17, SantaCruz, diluted 500 times), an anti-H-Ras antibody (C-20, SantaCruz, diluted 1000 times), and an anti-SOS1 antibody (C-23, SantaCruz, diluted 1000 times) were used. The membrane was shaken at 4ºC for 16 hours, and then washed with TBS-T for 5 minutes. It is noted that washing was performed three times. Further, the membrane was washed with TBS-T for 5 minutes. Subsequently, the membrane was treated with a chemiluminescence reagent Immobilon™ Western (Millipore), and then chemiluminescence was detected using a lumino image analyzer LAS-3000 (FUJIFILM Corporation).

Next, a reaction for detecting GAPDH as a control was performed using the same membrane. The membrane was washed with TBS-T, and shaken and blocked in 5% skim milk/TBS-T at room temperature for 30 minutes. After blocking, a primary antibody reaction was performed in 5% skim milk/TBS-T. As the primary antibody, anti-GAPDH antibody (6C5, SantaCruz, diluted 20000 times) was used. The membrane was shaken at room temperature for 60 minutes, and then washed with TBS-T for 5 minutes. It is noted that washing was performed three times. After the primary antibody reaction, a secondary antibody reaction was performed in 2% skim milk/TBS-T. As the secondary antibody, anti-mouse IgG (H+L) antibody (A90-116P-33, Bethyl) diluted 20000 times was used. The membrane was shaken at room temperature for 30 minutes, and then washed with TBS-T for 5 minutes. It is noted that washing was performed three times. Further, the membrane was washed with TBS (100 mM Tris-HCl, 150 mM NaCl, pH 7.6) for 5 minutes. Subsequently, the membrane was treated with a chemiluminescence reagent Immobilon™ Western (Millipore), and then chemiluminescence was detected using a lumino image analyzer LAS-3000 (FUJIFILM Corporation). Detected bands were quantified with an image processing software ImageJ (NIH).

The results of the Western blot analysis are shown in Fig. 3. Numeric values below each band in Fig. 3 show the quantification result of each protein detected by the Western blot analysis as a relative value when the value of the control (DMSO) was defined as 1.0. As shown in Fig. 3, when TUS-007 was added, the amount of the endogenous wild-type K-Ras protein and wild-type H-Ras protein was reduced, but the amount of the SOS1 protein was not reduced. This result matches the results of the protein affinity of Ras-SOS reported in the document by Sun, Q. et al. (Sun, Q. et al., Angew. Chem. Int. Ed., 2012, 51, 6140-6143).

### <Example 1>

In Example 1, TUS-007 was administered to mouse individuals, and then degradation (knockdown) of the wild-type K-Ras protein in each tissue of the mouse was detected to evaluate the molecular kinetics of TUS-007.

### (Administration of TUS-007 to mice)

TUS-007 was dissolved in DMSO, and then dissolved in corn oil so that the concentration of DMSO was 10 vol%, and then intraperitoneally administered to C57BL/6J wild-type mice (8 to 9 weeks old, male) (CLEA Japan, Inc.) in an amount of 40 mg/kg body weight or 80 mg/kg body weight (n = 3 to 4). Furthermore, in addition to a group in which TUS-007 was administered, a group in which Ras-SOS had been administered in a dose of 80 mg/kg body weight was prepared. As a control, an injection carrier (corn oil containing 10 vol% DMSO) was used. The mice were kept under an environment of ad libitum access to food and water. The mice were dissected under deep anesthesia by Somnopentyl (Kyoritsu Seiyaku Corporation) 48 hours after administration. Abdominal section was performed, and then the spleen, pancreas, liver, kidneys, colon, lungs, and heart were sequentially extracted and flash frozen in liquid nitrogen. Each tissue frozen in liquid nitrogen was stored in a deep freezer at -80ºC.

### (Western blot analysis of mouse tissues)

The frozen tissues (spleen: 0.02 g, other tissues: 0.04 g) were each triturated, and then 500 µL of TKM tissue lysis buffer (50 mM triethanolamine (pH 7.8), 50 mM KCl, 5 mM MgCl₂, 0.25 M sucrose, 1 mM PMSF, Protein Inhibitors Cocktail-EDTA free (Nacalai Tesque, Inc.), 1 mM DTT, and a recombinant RNase inhibitor (0.2 U/µL, Takara Bio) were added, and dissolved by rotation for 15 minutes (1 rpm, 25ºC). Then, the resultant product was subjected to centrifugation (at 13800 rpm x 30 minutes, 4ºC), and the supernatants (each tissue extract) were collected. The concentration of the extracted proteins was quantified by a spectrophotometer.

Each tissue extract collected was subjected to Western blot analysis. An SDS-PAGE gel was prepared using TGX™ FastCast™ Acrylamide Kit, 12% (Bio-Rad). Electrophoresis samples were prepared in a 6x SDS-PAGE sample buffer (62.5 mM Tris-HCl pH 6.8, 2% SDS, 5% 2-mercaptoethanol, 10% glycerol, 0.25% BPB), and placed on a heat block at 95ºC for 5 minutes. Electrophoresis was performed at 160 V for 60 minutes (electrophoresis buffer; 195 mM glycine, 25 mM Tris).

After electrophoresis, proteins were transferred to a PVDF membrane (Immobion™-P, Millipore) under conditions of 100 V and 1.5 hours using a tank-type blotting device and a transfer buffer (25 mM Tris-HCl, 195 mM glycine, 0.01% SDS, 15% methanol). The membrane was split into two at the position of a 25 kDa marker. The membrane after transfer was shaken and blocked in 5% skim milk/TBS-T (100 mM Tris-HCl, 150 mM NaCl, 0.1% Tween-20, pH 7.6) at room temperature for 30 minutes. After blocking, a primary antibody reaction was performed in 5% skim milk/TBS-T. As the primary antibody, an anti-K-Ras antibody (sc-30, SantaCruz, diluted 500 times) and an anti-GAPDH antibody (sc-32233, SantaCruz, diluted 20000 times) were used. The membrane was shaken at room temperature for 60 minutes (anti-K-Ras antibody) or at 4ºC overnight (anti-GAPDH antibody), and then the membrane was washed with TBS-T for 5 minutes. It is noted that washing was performed four times. After the primary antibody reaction, a secondary antibody reaction was performed in 1% skim milk/TBS-T. The membrane was shaken at room temperature for 30 minutes, and then the membrane was washed with TBS-T for 5 minutes. It is noted that washing was performed four times. Furthermore, the membrane was washed with TBS (100 mM Tris-HCl, 150 mM NaCl, pH 7.6) for 10 minutes. Subsequently, the membrane was treated with a chemiluminescence reagent Immobilon™ Western (Millipore), and then chemiluminescence was detected using a lumino image analyzer LAS-3000 (FUJIFILM Corporation). Detected bands were quantified with an image processing software ImageJ (NIH).

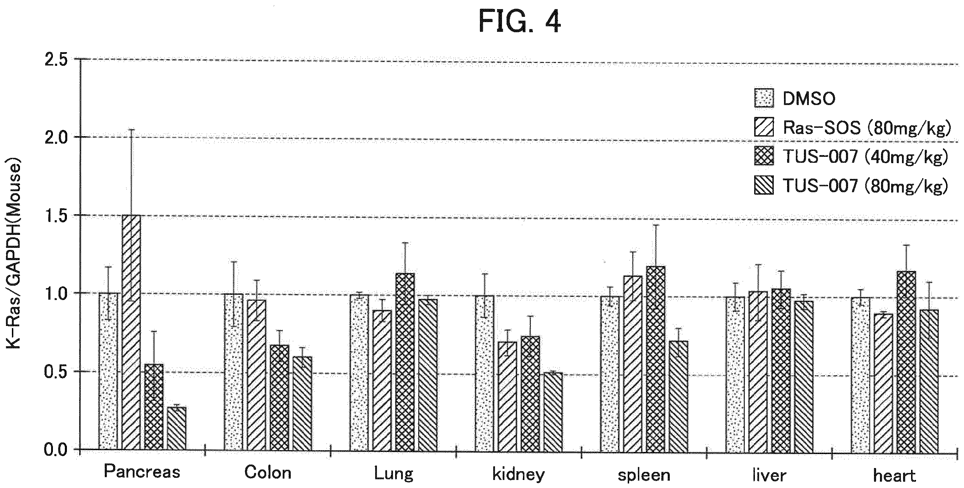

The results of the Western blot analysis are shown in Fig. 4. As shown in Fig. 4, when TUS-007 was administered to mice, the amount of the wild-type K-Ras protein was reduced in a concentration dependent manner in the pancreas, colon, kidneys, and spleen, and in particular, the reduction amount was greater in the pancreas. On the other hand, the amount of the wild-type K-Ras protein was not reduced in the lungs, liver, and heart. From the result, it was suggested that the TUS-007 showed a higher transfer property in the pancreas, colon, kidneys, and spleen as compared with the lungs, liver, and heart.

### <Synthesis Example 2>

In Synthesis Example 2, a protein-degradation inducing tag and a specific protein affinity molecule having an affinity with an ecDHFR protein were linked to each other to synthesize TMP-CANDDY_DMT as a protein-degradation inducing molecule.

As the protein-degradation inducing tag, a compound (DMT) in which R¹ and R² in the aforementioned formula (I) are each a methoxy group was used. DMT is a compound which is not derived from a proteasome inhibitor, but has an affinity with a proteasome. Furthermore, as the specific protein affinity molecule, a TMP derivative (TMP-NH₂) was used. The TMP derivative was obtained by introducing a functional group including an amino group into TMP that is a dihydrofolate reductase inhibitor to be bonded to an ecDHFR protein.

The method of synthesizing TMP-CANDDY_DMT is described in detail as the following synthesis scheme.

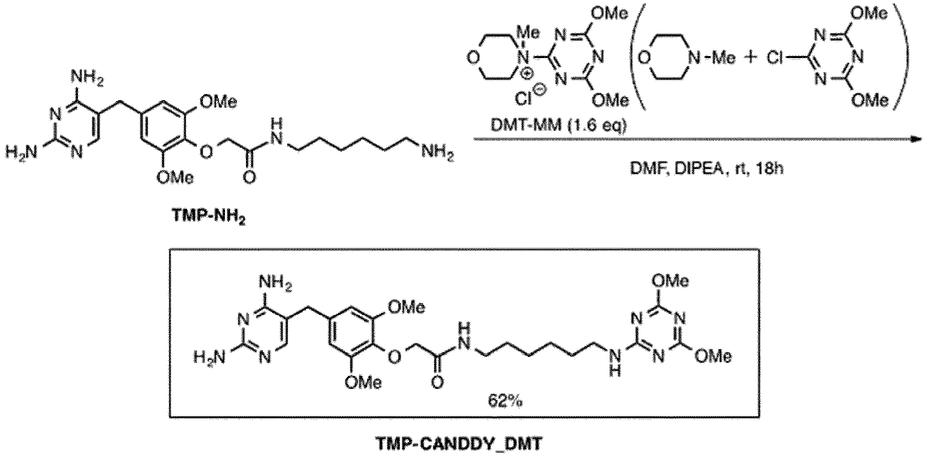

TMP-NH₂ (Long, M. J. et al., Chem. Biol., 2012, 19 (5), 629-637) (31.7 mg, 0.073 mmol) was charged into an eggplant flask, and 0.3 mL of dehydrate DMF was added. After the resultant solution was stirred at room temperature for 10 minutes, 0.1 mL of DIPEA was added, and stirred at room temperature for 10 minutes. DMT-MM (33.6 mg, 0.12 mmol, 1.6 eq, Wako Pure Chemical Industries, Ltd.) was directly added to the reaction solution, and stirred at room temperature for 18 hours. The reaction solution was diluted with water and aqueous sodium hydrogen carbonate, and extracted with chloroform for five times. After being dried over anhydrous sodium sulfate, the solvent was evaporated under reduced pressure. Separation and purification treatment was performed by silica gel chromatography (chloroform/methanol = 92/8) to obtain TMP-CANDDY_DMT (25.8 mg, 0.045 mmol, 62%, isolated yield).

### <Reference Example 4>

In Reference Example 2, the proteasome inhibitory activity of TMP-CANDDY_DMT and the affinity of TMP-CANDDY_DMT with a proteasome were evaluated. As a positive control, MG-132 as a proteasome inhibitor was used.

For evaluation, 20S Proteasome StressXpress™ Assay Kit Gold (Bioscience) was used. AMC was measured by using Multi-Detection Microplate Reader (Synergy HT, BIO-TEK). The AMC was produced by cleaving the C-terminus of an AMC-binding proteasome fluorescence substrate specific to β subunits of a 20S proteasome, including β5 (chymotrypsin-like activity), β2 (trypsin-like activity), and β1 (caspase-like activity). The measuring wavelengths were 360 nm for excitation light (Ex.), and 460 nm for fluorescence (Em.).

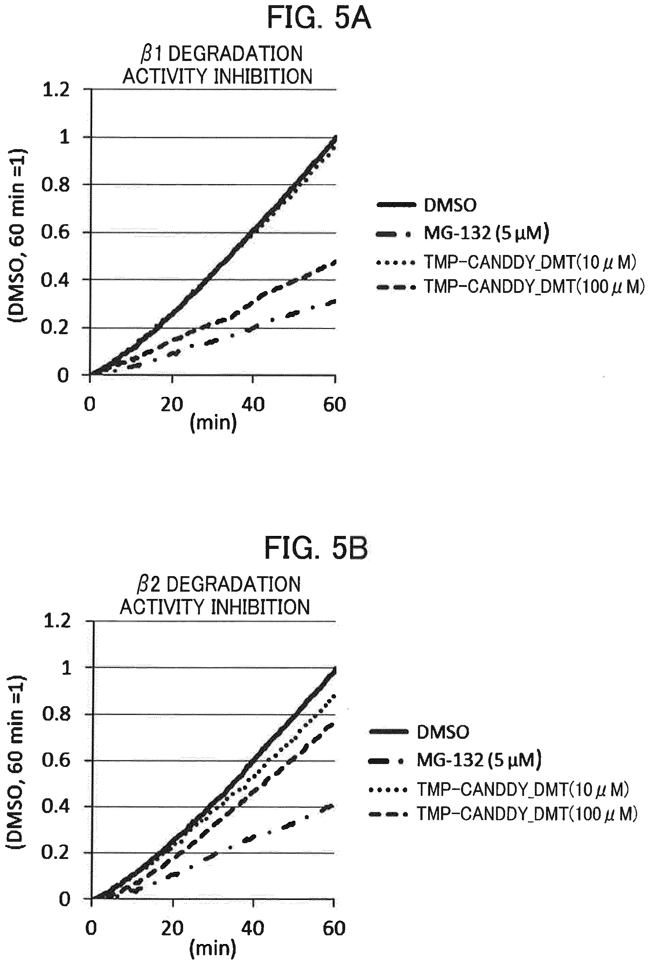

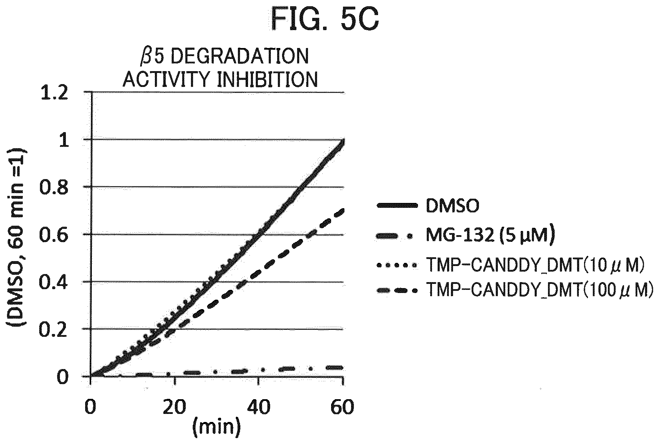

Figs. 5A to 5C show the proteasome activities against β1 (caspase-like activity), β2 (trypsin-like activity), and β5 (chymotrypsin-like activity), respectively. As can be seen in Figs. 5A to 5C, TMP-CANDDY_DMT was found to have a significantly lower proteasome inhibitory activity as compared with MG-132. Moreover, the inhibitory activity of TMP-CANDDY_DMT was increased in a concentration dependent manner against any of β1, β2, and β5, suggesting that TMP-CANDDY_DMT has a moderate affinity with a proteasome. That is, it was evaluated that DMT has an affinity with a proteasome, and does not inhibit degradation.

### <Reference Example 5>

In Reference Example 5, degradation (knockdown) of a forcibly expressed ecDHFR protein in HeLa cells through TMP-CANDDY_DMT was evaluated by FACS analysis.

### (Preparation of plasmid)

A plasmid (pMIR-DsRed-IRES-ecDHFR-HA-GFP) expressing an ecDHFR protein was amplified in E. coli, and then purified with Miniprep Kit (QIAGEN).

### (Introduction of plasmid into HeLa cells and cell seeding)

As in Reference Example 1, the plasmid was introduced into HeLa cells to transiently overexpress an ecDHFR protein (specifically, a fusion protein of an ecDHFR protein and GFP through a HA tag) or a DsRed protein for comparison in the cells. HeLa cells into which the plasmid had been introduced were seeded in a 24-well plate at a cell density of 6 x 10⁴ cells/well, and then cultured under conditions of 37ºC and 5 vol% CO₂ for 40 hours.

### (Addition of TMP-CANDDY_DMT to HeLa cells)

Culture was performed for 40 hours after introduction of the plasmid, and then TMP-CANDDY_DMT was added to HeLa cells as follows. As a medium, a serum-free medium (37ºC) in which 1 mass% L-glutamine solution (Sigma-Aldrich) was added to D-MEM (high D-glucose, phenol red, sodium pyruvate (Wako Pure Chemical Industries, Ltd.)) was used, and 297 µL of the medium was added to each well. It is noted that the L-glutamine solution was added immediately before use. A DMSO solution containing TMP-CANDDY_DMT was added to each well at 3 µL/well, and cultured under conditions of 37ºC and 5 vol% CO₂. As a control, a TMP-containing DMSO solution or DMSO was used.

### (Evaluation of degradation (knockdown) of ecDHFR protein through TMP-CANDDY_DMT (FACS analysis))

The medium was removed 24 hours after addition of TMP-CANDDY_DMT, and then PBS was added to wash the cells. After removing PBS, trypsin (0.25 w/v% trypsin-1 mmol/L EDTA·4 Na solution with phenol red) (Wako Pure Chemical Industries, Ltd.) at 37ºC was added to each well at 300 µL/well, and cultured under conditions of 37ºC and 5 vol% CO₂ for 1 minute. After culturing, a medium, in which 10 mass% FBS and 1 mass% PenStrep (100 U/mL sodium penicillin G and 100 µg/mL streptomycin sulfate) (Wako Pure Chemical Industries, Ltd.) had been added to D-MEM (low D-glucose, L-glutamine, phenol red) (Wako Pure Chemical Industries, Ltd.), was added to each well at 500 µL/well, and suspended, and then a cell solution was collected in a 15 mL tube.

The cell solution collected was centrifuged (at 1000 rpm x 5 minutes, 4ºC), and the supernatant was removed, and then suspended in 2 mL of PBS (37ºC). The cell solution after suspension was centrifuged (at 1000 rpm x 5 minutes, 4ºC), and the supernatant was removed, and then 500 µL of an FACS buffer (1 mass% FBS/PBS) at 4ºC was added, and allowed to stand on ice.

A BD FACSCanto™ II (BD Biosciences) was used for flow cytometry, and the expression levels of GFP and the DsRed protein in the cells were quantified. The cell solution was passed through a mesh with a pore size of 32 µm, and transferred to an FACS tube immediately before FACS analysis. The GFP/DsRed ratio per cell was computed using an analysis software FlowJo™ (TOMY Digital Biology Co., Ltd.), and the degradation (knockdown) of the ecDHFR protein by TMP-CANDDY_DMT was determined from a shift in a graph.

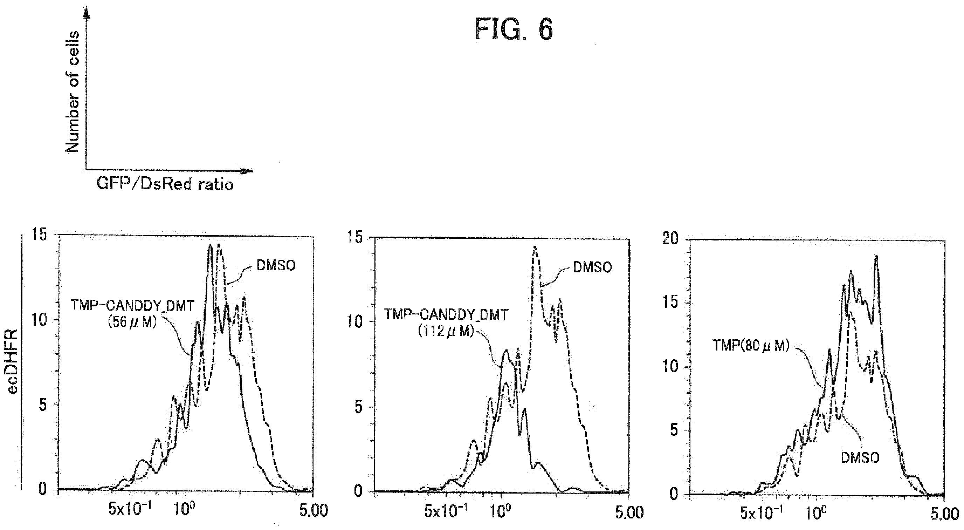

The results of the FACS analysis are shown in Fig. 6. As shown in Fig. 6, when TMP-CANDDY_DMT was added, the graph is shifted toward the left in a concentration-dependent manner, demonstrating that degradation of the ecDHFR protein was induced by TMP-CANDDY_DMT. On the other hand, when TMP was added, the graph is overlapped to that of the control (DMSO), demonstrating that the ecDHFR protein was not degraded.

### <Reference Example 6>

In Reference Example 6, degradation (knockdown) of a forcibly expressed ecDHFR protein in HeLa cells through TMP-CANDDY_DMT was evaluated by Western blot analysis.

### (Preparation of plasmid)

A plasmid expressing an ecDHFR protein was prepared, as in Reference Example 5.

### (Introduction of plasmid into HeLa cells and cell seeding)

As in Reference Example 5, the plasmid was introduced into HeLa cells to transiently overexpress an ecDHFR protein or a DsRed protein for comparison in the cells. HeLa cells into which a plasmid had been introduced were seeded in a 24-well plate at a cell density of 4 x 10⁴ cells/well, and then cultured under conditions of 37ºC and 5 vol% CO₂ for 40 hours.

### (Addition of TMP-CANDDY_DMT to HeLa cells)

Culture was performed for 40 hours after introduction of the plasmid, and then TMP-CANDDY_DMT was added to HeLa cells as follows. As a medium, a serum-free medium (37ºC) in which 1 mass% L-glutamine solution (Sigma-Aldrich) was added to D-MEM (high D-glucose, phenol red, sodium pyruvate (Wako Pure Chemical Industries, Ltd.)) was used. It is noted that the L-glutamine solution was added immediately before use. A DMSO solution containing TMP-CANDDY_DMT was mixed with the medium so that the concentration of DMSO was 1 vol%, and added to each well at 300 µL/well, and cultured under conditions of 37ºC and 5 vol% CO₂. Furthermore, in addition to an experiment group in which a DMSO solution containing TMP-CANDDY_DMT had been added, an experiment group in which a DMSO solution containing both TMP-CANDDY_DMT and bortezomib had been added was prepared. Cycloheximide as a protein synthesis inhibitor was added to the medium so as to give a concentration of 50 µg/mL 12 hours after addition of TMP-CANDDY_DMT. It is noted that as a control, a TMP-containing DMSO solution or DMSO was used.

### (Evaluation of degradation (knockdown) of ecDHFR protein through TMP-CANDDY_DMT (Western blot analysis))

The medium was removed 24 hours after addition of TMP-CANDDY_DMT, and PBS was added to wash the cells. After removing PBS, a mixed solution of a cell lysis buffer (CelLytic™ M, Sigma) and a protease inhibitor (cOmplete™ Mini, EDTA-free, Roche) was added to each well at 55 µL/well. After being allowed to stand at 4ºC for 15 minutes, cells were detached with a pipette tip on ice. A cell solution was collected in a 1.5 mL tube, and flash frozen in liquid nitrogen, and then thawed on ice. After repeating this freeze-thaw cycle three times, the solution was centrifuged (at 13000 rpm x 20 minutes, 4ºC), and the supernatant (cell extract) was collected.

The cell extract collected was subjected to Western blot analysis. An SDS-PAGE gel was prepared using TGX™ FastCast™ Acrylamide Kit, 12% (Bio-Rad). Electrophoresis samples were prepared in a 6x SDS-PAGE sample buffer (62.5 mM Tris-HCl pH 6.8, 2% SDS, 5% 2-mercaptoethanol, 10% glycerol, 0.25% BPB), and placed on a heat block at 95ºC for 4 minutes. Electrophoresis was performed at 150 V for 50 minutes (electrophoresis buffer; 195 mM glycine, 25 mM Tris).

After electrophoresis, proteins were transferred to a PVDF membrane (Immobion™-P, Millipore) under conditions of 100 V and 40 minutes using a tank-type blotting device and a transfer buffer (25 mM Tris-HCl, 195 mM glycine, 0.01% SDS, 15% methanol). The membrane after transfer was shaken and blocked at room temperature for 30 minutes in 5% skim milk/high-salt TBS-T (100 mM Tris-HCl, 500 mM NaCl, 0.2% Tween-20, pH 7.6). After blocking, the membrane was rinsed with High-Salt TBS-T, and an antibody reaction was performed in 1% skim milk/high-salt TBS-T. As the antibody, anti-HA-peroxidase and high-affinity (3F10) rat monoclonal antibody (25 U/mL) (Roche) diluted 1000 times was used. The membrane was shaken at room temperature for 1 hour, and then washed with high-salt TBS-T for 5 minutes. It is noted that washing was performed three times. Further, the membrane was washed with high-salt TBS (100 mM Tris-HCl, 500 mM NaCl, pH 7.6) for 5 minutes. Subsequently, the membrane was treated with a chemiluminescence reagent Immobilon™ Western (Millipore), and then chemiluminescence was detected using a lumino image analyzer LAS-3000 (FUJIFILM Corporation).

Next, a reaction for detecting GAPDH as a control was performed using the same membrane. The membrane was washed with TBS-T (100 mM Tris-HCl, 150 mM NaCl, 0.1% Tween-20, pH 7.6), and blocked by shaking at room temperature for 30 minutes in 5% skim milk/TBS-T. After blocking, a primary antibody reaction was performed in 5% skim milk/TBS-T. As the primary antibody, an anti-GAPDH antibody (6C5, SantaCruz, diluted 20000 times) was used. The membrane was shaken at room temperature for 60 minutes, and then washed with TBS-T for 5 minutes. It is noted that washing was performed three times. After the primary antibody reaction, a secondary antibody reaction was performed in 2% skim milk/TBS-T. As the secondary antibody, anti-mouse IgG (H+L) antibody (A90-116P-33, Bethyl) diluted 20000 times was used. The membrane was shaken at room temperature for 30 minutes, and then washed with TBS-T for 5 minutes. It is noted that washing was performed three times. Further, the membrane was washed with TBS (100 mM Tris-HCl, 150 mM NaCl, pH 7.6) for 5 minutes. Subsequently, the membrane was treated with a chemiluminescence reagent Immobilon™ Western (Millipore), and then chemiluminescence was detected using a lumino image analyzer LAS-3000 (FUJIFILM Corporation). Detected bands were quantified with an image processing software ImageJ (NIH).

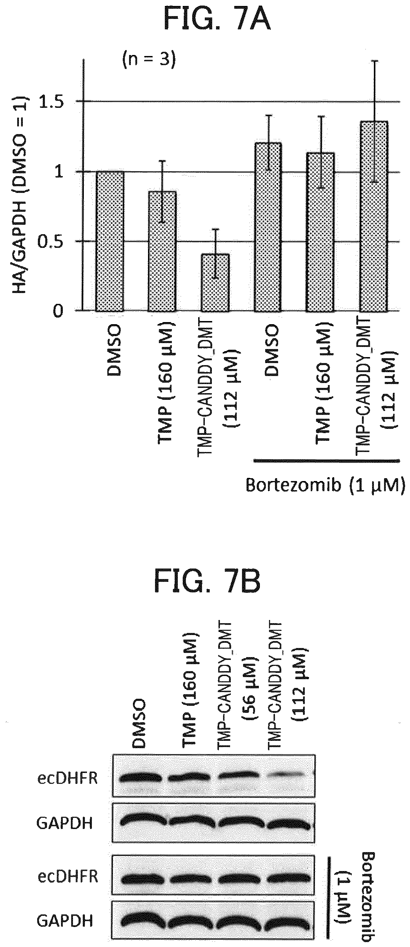

The results of the Western blot analysis are shown in Figs. 7A and 7B. As shown in Figs. 7A and 7B, when TMP-CANDDY_DMT was added, the amount of the ecDHFR protein was reduced, but when TMP was added, the amount of the ecDHFR protein was not reduced. Furthermore, when both TMP-CANDDY_DMT and bortezomib were added, as compared with the addition of TMP-CANDDY_DMT, degradation of the ecDHFR protein was inhibited. This result supports that TMP-CANDDY_DMT leads the ecDHFR protein to the degradation by a proteasome.

### <Synthesis Example 3>

In Synthesis Example 3, a protein-degradation inducing tag and a specific protein affinity molecule having an affinity with a DHFR protein were linked to each other to synthesize MTX-CANDDY_MLN as a protein-degradation inducing molecule.

As the protein-degradation inducing tag, the above-described CANDDY_MLN was used. Furthermore, as the specific protein affinity molecule, an MTX derivative (MTX-NH₂) was used. The MTX derivative was obtained by introducing a functional group including an amino group into MTX that is a dihydrofolate reductase inhibitor to be bonded to a DHFR protein.

The method of synthesizing TMP-CANDDY_DMT is described in detail as the following synthesis scheme.

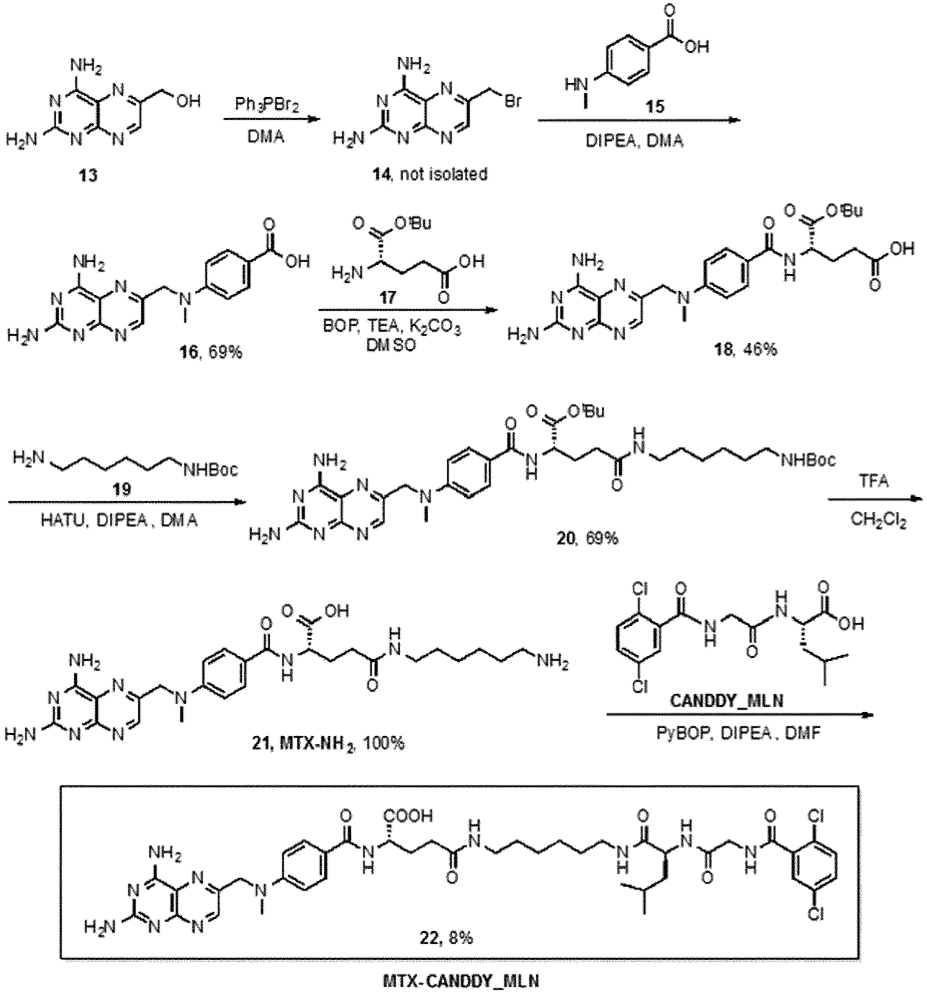

### (Synthesis of compound 21 (MTX-NH₂))

A compound 13 was reacted with triphenylphosphine dibromide in DMA to obtain a compound 14. The compound 14 was dissolved in DMA under a stream of nitrogen, and then a compound 15 and DIPEA were added and reacted to obtain a compound 16 (yield: 69%). Subsequently, the compound 16 and a compound 17 were dissolved in DMSO under a stream of nitrogen, and subjected to condensation reaction with a BOP reagent to obtain a compound 18 (yield: 46%). Subsequently, the compound 18 and a compound 19 were dissolved in DMA under a stream of nitrogen, and subjected to condensation reaction with HATU to obtain a compound 20 (yield: 69%). Then, the compound 20 was dissolved in dichloromethane, and subjected to deprotection with TFA to obtain a compound 21 (MTX-NH₂).

### (Synthesis of compound 22 (MTX-CANDDY_MLN))

The compound 21 (MTX-NH₂) and CANDDY_MLN were dissolved in DMA under a stream of nitrogen, and condensation reaction was performed with PyBOP (at room temperature, 3 hours). The reaction solution was diluted with water and aqueous sodium hydrogen carbonate, and extracted with ethyl acetate three times. After being dried over anhydrous sodium sulfate, the solvent was evaporated under reduced pressure. Separation and purification treatment was performed by silica gel chromatography (chloroform/methanol = 20/1 to 4/1, gradient). Subsequently, separation and purification treatment was performed by preparative thin layer chromatography (chloroform/methanol = 85/15) to obtain a compound 22 (MTX-CANDDY_MLN) (isolated yield: 8%).

### <Reference Example 7>

In Reference Example 7, degradation (knockdown) of an endogenous DHFR protein in HeLa cells to which MTX-CANDDY_MLN had been added was evaluated by Western blot analysis.

### (Preparation of cultured cells and cell seeding)

HeLa cells were prepared, and seeded in a 24-well plate as in Reference Example 3.

### (Addition of MTX-CANDDY_MLN to HeLa cells)

MTX-CANDDY_MLN was added to HeLa cells, as in Reference Example 5. As a control, instead of an MTX-CANDDY_MLN-containing DMSO solution, an MTX-containing DMSO solution or DMSO was used.

### (Evaluation of degradation (knockdown) of DHFR protein through MTX-CANDDY_MLN (Western blot analysis))

The medium was removed 16 hours after addition of MTX-CANDDY_MLN (50 µM, 100 µM, or 200 µM) or MTX (50 µM, 100 µM or 200 µM), and then 1 mL/well of PBS (Wako Pure Chemical Industries, Ltd.) at 4ºC was added to wash the cells. After removing PBS, a mixed solution of a cell lysis buffer (CelLytic™ M, Sigma) and a protease inhibitor (cOmplete™ Mini, EDTA-free (REF 11 836 170 001), Roche) was added to each well at 27 µL/well. After being allowed to stand at 4ºC for 15 minutes, cells were detached with a pipette tip (P1000) on ice. A cell solution was collected in a 1.5 mL tube, and flash frozen in liquid nitrogen, and then thawed on ice. After thawing, the solution was centrifuged (at 12000 rpm × 15 minutes, 4ºC), and the supernatant (cell extract) was collected.

The cell extract collected was subjected to Western blot analysis. An SDS-PAGE gel (14 wells) was prepared using TGX™ FastCast™ Acrylamide Kit, 12% (Bio-Rad). Electrophoresis samples were prepared in a 6x SDS-PAGE sample buffer (62.5 mM Tris-HCl pH 6.8, 2% SDS, 5% 2-mercaptoethanol, 10% glycerol, 0.25% BPB), and placed on a heat block at 95ºC for 4 minutes. The electrophoresis samples prepared were applied to each well at 20 µL/well. Precision Plus Protein™ Dual Color Standards (Bio-Rad) were used for an electrophoresis marker. Electrophoresis was performed at 160 V for 65 minutes (electrophoresis buffer; 195 mM glycine, 25 mM Tris).

After electrophoresis, proteins were transferred to a PVDF membrane (Immobion™-P, Millipore) under conditions of 100 V and 2 hours using a tank-type blotting device and a transfer buffer (25 mM Tris-HCl, 195 mM glycine, 0.01% SDS, 15% methanol). The membrane was split into two at the position of a 25 kDa marker. The membrane after transfer was shaken and blocked in 5% skim milk/TBS-T (100 mM Tris-HCl, 150 mM NaCl, 0.1% Tween-20, pH 7.6) at room temperature for 30 minutes. After blocking, a primary antibody reaction was performed in 5% skim milk/TBS-T. As the primary antibody, anti-DHFR antibody (sc-14780, SantaCruz, diluted 500 times) and anti-GAPDH antibody (sc-32233, SantaCruz, diluted 20000 times) were used. The membranes were shaken at room temperature for 90 minutes (anti-DHFR antibody) or for 45 minutes (anti-GAPDH antibody), and then washed with TBS-T for 5 minutes. It is noted that washing was performed three times. After the primary antibody reaction, a secondary antibody reaction was performed in 2% skim milk/TBS-T. The membranes were shaken at room temperature for 30 minutes, and then washed with TBS-T for 5 minutes. It is noted that washing was performed three times. Further, the membranes were washed with TBS (100 mM Tris-HCl, 150 mM NaCl, pH 7.6) for 5 minutes. Subsequently, the membranes were treated with a chemiluminescence reagent Immobilon™ Western (Millipore), and then chemiluminescence was detected using a lumino image analyzer LAS-3000 (FUJIFILM Corporation). Detected bands were quantified with an image processing software ImageJ (NIH).

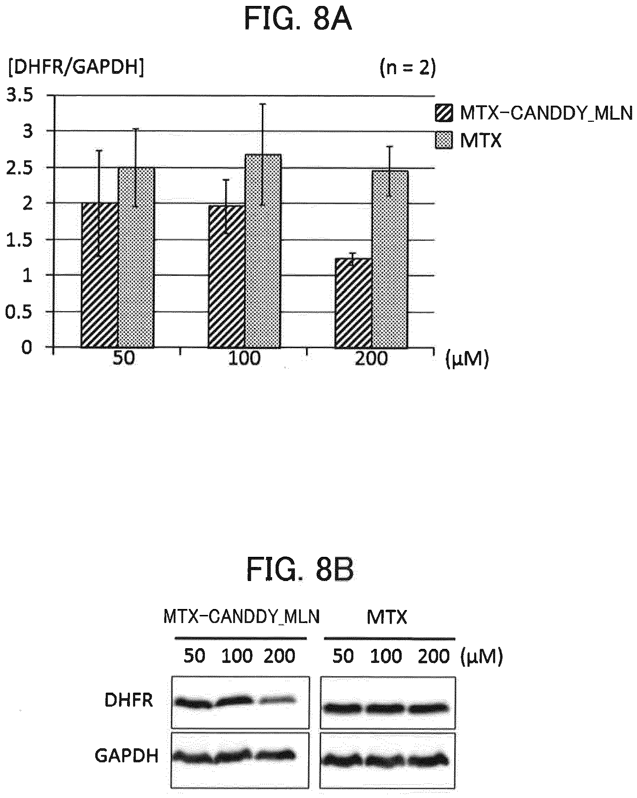

Fig. 8A shows the results from quantification of bands detected in the Western blot analysis of the endogenous DHFR protein in HeLa cells to which MTX-CANDDY_MLN or MTX was added. Fig. 8B shows the detected bands. As can be seen in Figs. 8A and 8B, the amount of the DHFR protein was decreased in a concentration dependent manner when MTX-CANDDY_MLN was added. In contrast, when MTX was added, a decrease in the amount of the DHFR protein was not observed even at a concentration of 200 µM.

### <Example 2>

In Example 2, MTX-CANDDY_MLN was administered to mouse individuals, and then degradation (knockdown) of the DHFR protein in each tissue of the mouse was detected to evaluate the molecular kinetics of MTX-CANDDY_MLN.

### (Administration of MTX-CANDDY_MLN to mice)

MTX-CANDDY_MLN was dissolved in DMSO immediately before administration, and then dissolved in corn oil (Code No. 25606-55, Nacalai Tesque) so that the concentration of DMSO was 10 vol%, and then a dose of 50 mg/kg body weight or 100 mg/kg body weight was administered intraperitoneally to C57BL/6J wild-type mice (7 weeks old, male) (CLEA Japan, Inc.) (n = 3). Furthermore, in addition to a group in which MTX-CANDDY_MLN was administered, a group in which MTX had been administered in a dose of 100 mg/kg body weight was prepared. As a control, an injection carrier (corn oil containing 10 vol% DMSO) was used. The mice were kept under an environment of ad libitum access to food and water. The mice were dissected under deep anesthesia by Somnopentyl (Kyoritsu Seiyaku Corporation) 24 hours after administration. Abdominal section was performed, and then the liver, kidneys, and heart were sequentially extracted and flash frozen in liquid nitrogen. Each tissue frozen in liquid nitrogen was stored in a deep freezer at -80ºC.

### (Western blot analysis of mouse tissues)

The frozen tissues (0.04 g) were each frozen and triturated, and then 980 µL of 1x TKM tissue lysis buffer (50 mM triethanolamine (pH 7.8), 50 mM KCl, 5 mM MgCl₂, 0.25 M sucrose, 1 mM PMSF, protein inhibitors cocktail-EDTA free (Code No. 03969-21, Nacalai Tesque, Inc.), 1 mM DTT, and 5 µL/mL recombinant RNase inhibitor (40 U/µL, Cat No. 2313A, Lot No. K8402DA, TAKARA Bio)) were added, and dissolved by rotation for 15 minutes (1 rpm, 25ºC). Then, the resultant product was subjected to centrifugation (at 3000 rpm x 15 minutes, 4ºC), and the supernatants (each tissue extract) were collected. The concentration of proteins in each tissue extract was quantified with a spectrophotometer using each tissue extract that had been diluted 20 times using DEPC-treated water.

Each tissue extract collected was subjected to Western blot analysis. An SDS-PAGE gel was prepared using TGX™ FastCast™ Acrylamide Kit, 12% (Bio-Rad). Electrophoresis samples were prepared in a 6x SDS-PAGE sample buffer (62.5 mM Tris-HCl pH 6.8, 2% SDS, 5% 2-mercaptoethanol, 10% glycerol, 0.25% BPB), and placed on a heat block at 95ºC for 5 minutes. The electrophoresis samples prepared were applied at 50 µg/well for detection of GAPDH, and applied at 100 µg/well for other detection. Electrophoresis was performed at 160 V for 60 minutes (electrophoresis buffer; 195 mM glycine, 25 mM Tris).

After electrophoresis, proteins were transferred to a PVDF membrane (Immobion™-P, Millipore) under conditions of 100 V and 1.5 hours using a tank-type blotting device and a transfer buffer (25 mM Tris-HCl, 195 mM glycine, 0.01% SDS, 15% methanol). The membrane was split into two at the position of a 25 kDa marker. The membrane after transfer was shaken and blocked in 5% skim milk/TBS-T (100 mM Tris-HCl, 150 mM NaCl, 0.1% Tween-20, pH 7.6) at room temperature for 30 minutes. After blocking, a primary antibody reaction was performed in 5% skim milk/TBS-T. As the primary antibody, an anti-DHFR antibody (sc-14780, SantaCruz, diluted 500 times) and an anti-GAPDH antibody (sc-32233, SantaCruz, diluted 20000 times) were used. The membrane was shaken at room temperature for 60 minutes, and then the membrane was washed with TBS-T for 5 minutes. It is noted that washing was performed three times. After the primary antibody reaction, a secondary antibody reaction was performed in 1% skim milk/TBS-T. The membrane was shaken at room temperature for 30 minutes, and then the membrane was washed with TBS-T for 5 minutes. It is noted that washing was performed three times. Furthermore, the membrane was washed with TBS (100 mM Tris-HCl, 150 mM NaCl, pH 7.6) for 10 minutes. Subsequently, the membrane was treated with a chemiluminescence reagent Immobilon™ Western (Millipore), and then chemiluminescence was detected using a lumino image analyzer LAS-3000 (FUJIFILM Corporation). Detected bands were quantified with an image processing software ImageJ (NIH).

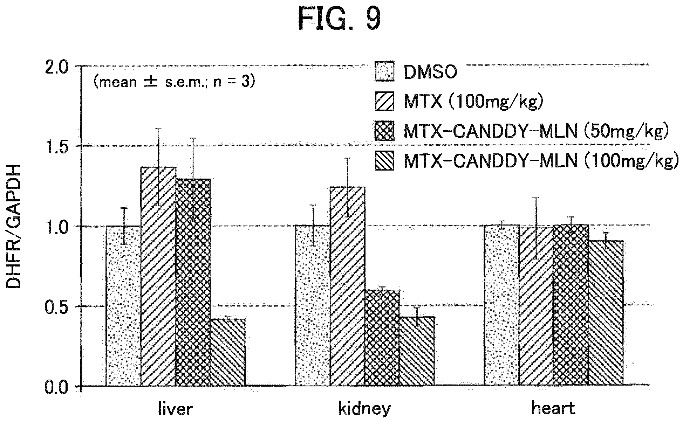

The results of the Western blot analysis are shown in Fig. 9. As shown in Fig. 9, when MTX-CANDDY_MLN was administered to mice, the amount of the DHFR protein was reduced in a concentration dependent manner in the liver and kidneys. On the other hand, reduction in the amount of the DHFR protein was not found in the heart. From the result, it was suggested that the MTX-CANDDY_MLN showed a higher transfer property in the liver and kidneys, as compared with the heart.

### <Synthesis Example 4: Synthesis of TIBC-CANDDY_MLN>

In Synthesis Example 4, a protein-degradation inducing tag and a specific protein affinity molecule having an affinity with a p53/MDM2 complex were linked to each other to synthesize TIBC-CANDDY_MLN as a protein-degradation inducing molecule.

As the protein-degradation inducing tag, the above-described CANDDY_MLN was used. Furthermore, as the specific protein affinity molecule, TIBC-NH₂ represented by the following formula was used. TIBC-NH₂ is a compound obtained by adding H₂N-(CH₂)₆-COOH to TIBC represented by the following formula. TIBC has an affinity with the p53/MDM2 complex.

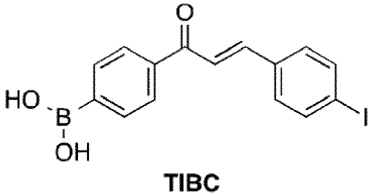

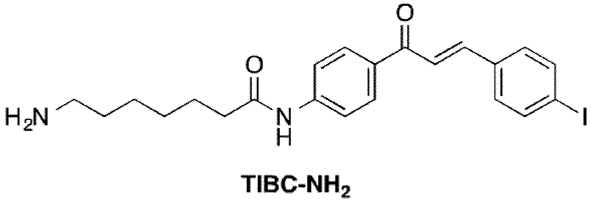

The method of synthesizing TIBC-CANDDY_MLN is described in detail as follows.

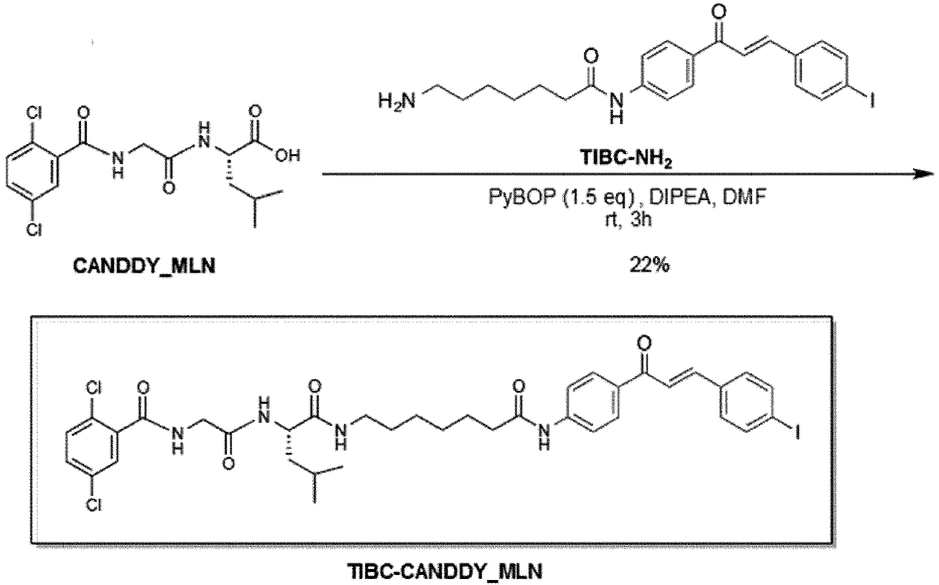

CANDDY_MLN (21.7 mg, 0.06 mmol, 1 eq) and separately synthesized TIBC-NH₂ (29.3 mg, 0.06 mmol, 1 eq) were charged into an eggplant flask, and 5 mL of dehydrate DMF was then added. After the resultant solution was stirred at room temperature for 5 minutes, 5 mL of DIPEA was then added to neutralize the solution. After the resultant product was stirred at room temperature for 20 minutes, PyBOP (46.8 mg, 0.09 mmol, 1.5 eq) was directly added to a reaction solution, and the reaction solution was stirred at room temperature for 16 hours. Under cooling, a saturated sodium hydrogen carbonate aqueous solution was added, an organic layer was separated, and then a water layer was extracted with ethyl acetate. Organic layers were collected, and dried over anhydrous sodium sulfate. After the solvent was evaporated under reduced pressure, a separation refining process using silica gel chromatography (chloroform/methanol = 20/1 to 4/1, gradient) was performed to obtain TIBC-CANDDY_MLN (10.8 mg, 0.013 mmol, 22%, isolated yield). The obtained TIBC-CANDDY_MLN was further purified by preparative thin layer chromatography (chloroform/methanol = 10/1). The physical property data of TIBC-CANDDY_MLN are shown as follows. HRMS-FAB (m/z): [M+H]+ calcd for C₃₇H₄₂C₁₂N₄O₅I, 819.1577; found, 819.1577

### <Reference Example 8>

In Reference Example 8, degradation (knockdown) of an endogenous wild-type p53 protein and MDM2 protein in HCT116 cells (human large intestine cancer cells) to which TIBC-CANDDY_MLN had been added was evaluated by Western blot analysis.

### (Cell seeding)

HCT116 cells were seeded in a 24-well plate at a cell density of 8 x 10⁴ cells/well, and then cultured under conditions of 37ºC and 5 vol% CO₂ for 16 hours.

### (Addition of TIBC-CANDDY_MLN or TIBC to HCT116 cells)

After 16 hours from cell seeding, TIBC-CANDDY_MLN or TIBC was added to HCT116 cells as follows. As a medium, a serum-free medium (37ºC) in which 1 mass% L-glutamine solution (Sigma-Aldrich) was added to D-MEM (high D-glucose, phenol red, sodium pyruvate (Wako Pure Chemical Industries, Ltd.)) was used. It is noted that the L-glutamine solution was added immediately before use. A DMSO solution containing TIBC-CANDDY_MLN or TIBC was mixed with the medium so that the concentration of DMSO was 1 vol%, and added to each well at 500 µL/well, and cultured under conditions of 37ºC and 5 vol% CO₂. As a control, DMSO was used.

### (Evaluation of degradation (knockdown) of endogenous wild-type p53 protein and MDM2 protein through TIBC-CANDDY_MLN (Western blot analysis))

The medium was removed 48 hours after addition of TIBC-CANDDY_MLN or TIBC, and then PBS was added to wash the cells. After removing PBS, a mixed solution of a cell lysis buffer (CelLytic™ M, Sigma) and a protease inhibitor (cOmplete™ Mini, EDTA-free (REF 11 836 170 001), Roche) was added to each well at 27 µL/well. After being allowed to stand at 4ºC for 15 minutes, cells were detached with a pipette tip on ice. A cell solution was collected in a 1.5 mL tube, and flash frozen in liquid nitrogen, and then thawed on ice. After thawing, the solution was centrifuged (at 13800 rpm x 20 minutes, 4ºC), and the supernatant (cell extract) was collected.

The cell extract collected was subjected to Western blot analysis. An SDS-PAGE gel was prepared using TGX™ FastCast™ Acrylamide Kit, 12% (Bio-Rad). Electrophoresis samples were prepared in a 6x SDS-PAGE sample buffer (62.5 mM Tris-HCl pH 6.8, 2% SDS, 5% 2-mercaptoethanol, 10% glycerol, 0.25% BPB), and placed on a heat block at 95ºC for 4 minutes. Electrophoresis was performed at 160 V for 65 minutes (electrophoresis buffer; 195 mM glycine, 25 mM Tris).

After electrophoresis, proteins were transferred to a PVDF membrane (Immobion™-P, Millipore) under conditions of 100 V and 2 hours using a tank-type blotting device and a transfer buffer (25 mM Tris-HCl, 195 mM glycine, 0.01% SDS, 15% methanol). The membrane after transfer was shaken and blocked at room temperature for 30 minutes in 5% skim milk/TBS-T (100 mM Tris-HCl, 150 mM NaCl, 0.1% Tween-20, pH 7.6). After blocking, a primary antibody reaction was performed in 5% skim milk/TBS-T. As the primary antibody, an anti-p53 antibody (DO-1, SantaCruz, diluted 1500 times), an anti-MDM2 antibody (SMP14, SantaCruz, diluted 500 times), and an anti-GAPDH antibody (6C5, SantaCruz, diluted 20000 times) were used. The membrane was shaken at 4ºC overnight, and then washed with TBS-T for 5 minutes. It is noted that washing was performed three times. After the primary antibody reaction, a secondary antibody reaction was performed in 2% skim milk/TBS-T. As the secondary antibody, anti-mouse IgG (H+L) antibody (A90-116P-33, Bethyl, diluted 20000 times) was used. The membrane was shaken at room temperature for 45 minutes, and then washed with TBS-T for 5 minutes. It is noted that washing was performed three times. Further, the membrane was washed with TBS (100 mM Tris-HCl, 150 mM NaCl, pH 7.6) for 5 minutes. Subsequently, the membrane was treated with a chemiluminescence reagent Immobilon™ Western (Millipore), and then chemiluminescence was detected using a lumino image analyzer LAS-3000 (FUJIFILM Corporation).

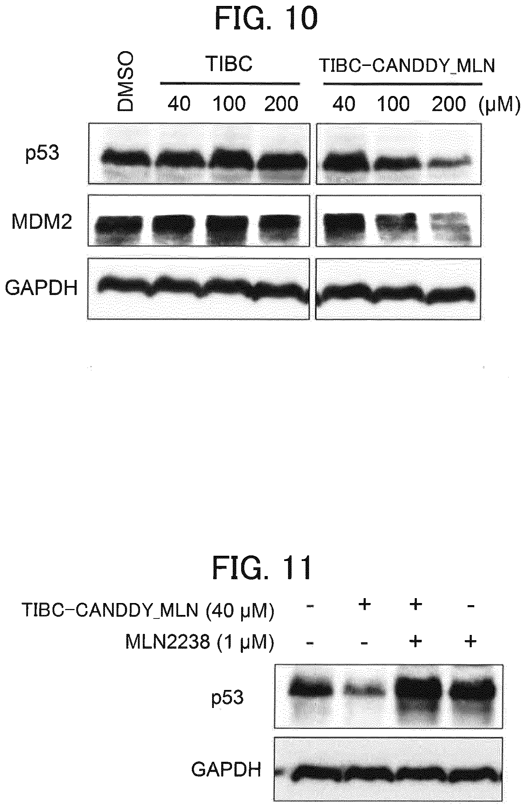

The results of the Western blot analysis are shown in Fig. 10. As shown in Fig. 10, when TIBC-CANDDY_MLN was added, the amount of the endogenous wild-type p53 protein and MDM2 protein was reduced. On the contrary, when TIBC was added, the amount of the endogenous wild-type p53 protein and MDM2 protein was not reduced.

### <Reference Example 9>

In Reference Example 9, degradation (knockdown) of an endogenous wild-type p53 protein in HeLa cells through TIBC-CANDDY_MLN was evaluated by Western blot analysis. At the same time, a rescue of degradation of the p53 protein by a proteasome inhibitor was evaluated.

### (Cell seeding)

HeLa cells were seeded in a 24-well plate at a cell density of 4 x 10⁴ cells/well, and then cultured under conditions of 37ºC and 5 vol% CO₂ for 16 hours.

### (Addition of TIBC-CANDDY_MLN to Hela cells)

After 16 hours from cell seeding, TIBC-CANDDY_MLN was added to HeLa cells as follows. As a medium, a serum-free medium (37ºC) in which 1 mass% L-glutamine solution (Sigma-Aldrich) was added to D-MEM (high D-glucose, phenol red, sodium pyruvate (Wako Pure Chemical Industries, Ltd.) was used. It is noted that the L-glutamine solution was added immediately before use. A DMSO solution containing TIBC-CANDDY_MLN was mixed with the medium so that the concentration of DMSO was 1 vol%, and added to each well at 500 µL/well, and cultured under conditions of 37ºC and 5 vol% CO₂. As a control, DMSO was used. Furthermore, in addition to an experiment group in which a DMSO solution containing TIBC-CANDDY_MLN was added, an experiment group in which a DMSO solution containing both of TIBC-CANDDY_MLN and MLN2238, or MLN2238 has been added was prepared.

### (Evaluation of degradation (knockdown) of endogenous wild-type p53 protein through TIBC-CANDDY_MLN (Western blot analysis))

The medium was removed 24 hours after addition of TIBC-CANDDY_MLN or MLN2238, and PBS was added to wash the cells. After removing PBS, a mixed solution of a cell lysis buffer (CelLytic™ M, Sigma) and a protease inhibitor (cOmplete™ Mini, EDTA-free, Roche) was added to each well at 27 µL/well. After being allowed to stand at 4ºC for 15 minutes, cells were detached with a pipette tip on ice. A cell solution was collected in a 1.5 mL tube, and flash frozen in liquid nitrogen, and then thawed on ice. After thawing, the solution was centrifuged (at 13800 rpm x 20 minutes, 4ºC), and the supernatant (cell extract) was collected.

The cell extract collected was subjected to Western blot analysis. An SDS-PAGE gel was prepared using TGX™ FastCast™ Acrylamide Kit, 12% (Bio-Rad). Electrophoresis samples were prepared in a 6x SDS-PAGE sample buffer (62.5 mM Tris-HCl pH 6.8, 2% SDS, 5% 2-mercaptoethanol, 10% glycerol, 0.25% BPB), and placed on a heat block at 95ºC for 4 minutes. Electrophoresis was performed at 160 V for 65 minutes (electrophoresis buffer; 195 mM glycine, 25 mM Tris).

After electrophoresis, proteins were transferred to a PVDF membrane (Immobion™-P, Millipore) under conditions of 100 V and 2 hours using a tank-type blotting device and a transfer buffer (25 mM Tris-HCl, 195 mM glycine, 0.01% SDS, 15% methanol). The membrane after transfer was blocked by shaking at room temperature for 30 minutes in 5% skim milk/TBS-T (100 mM Tris-HCl, 150 mM NaCl, 0.1% Tween-20, pH 7.6). After blocking, a primary antibody reaction was performed in 5% skim milk/TBS-T. As the primary antibody, an anti-p53 antibody (DO-1, SantaCruz, diluted 1000 times), and an anti-GAPDH antibody (6C5, SantaCruz, diluted 10000 times) were used. The membrane was shaken at 4ºC overnight, and then washed with TBS-T for 5 minutes. It is noted that washing was performed three times. After the primary antibody reaction, a secondary antibody reaction was performed in 2% skim milk/TBS-T. As the secondary antibody, anti-mouse IgG (H+L) antibody (A90-116P-33, Bethyl, diluted 10000 times) was used. The membrane was shaken at room temperature for 30 minutes, and then washed with TBS-T for 5 minutes. It is noted that washing was performed three times. Further, the membrane was washed with TBS (100 mM Tris-HCl, 150 mM NaCl, pH 7.6) for 5 minutes. Subsequently, the membrane was treated with a chemiluminescence reagent Immobilon™ Western (Millipore), and then chemiluminescence was detected using a lumino image analyzer LAS-3000 (FUJIFILM Corporation).

The results of the Western blot analysis are shown in Fig. 11. As shown in Fig. 11, when TIBC-CANDDY_MLN was added, the amount of the endogenous wild-type p53 protein was reduced. Furthermore, when both TIBC-CANDDY_MLN and MLN2238 were added, the amount of the wild-type p53 protein was increased as compared with the control (DMSO). The results support that TIBC-CANDDY_MLN leads the wild-type p53 protein to the degradation by a proteasome.

### <Example 3>

In Example 3, TIBC-CANDDY_MLN was administered to mouse individuals, and then degradation (knockdown) of the wild-type p53 protein and MDM2 protein in each tissue of the mouse was detected to evaluate the molecular kinetics of TIBC-CANDDY_MLN.

### (Administration of TIBC-CANDDY_MLN to mice)

TIBC-CANDDY_MLN was dissolved in DMSO immediately before administration, and then dissolved in corn oil (Code No. 25606-55, Nacalai Tesque) so that the concentration of DMSO was 10 vol%, and then a dose of 50 mg/kg body weight or 100 mg/kg body weight was administered intraperitoneally to C57BL/6J wild-type mice (7 to 8 weeks old, male) (CLEA Japan, Inc.) (n = 3). As a control, an injection carrier (corn oil containing 10 vol% DMSO) was used. The mice were kept under an environment of ad libitum access to food and water. The mice were dissected under deep anesthesia by Somnopentyl (Kyoritsu Seiyaku Corporation) 48 hours after administration. Abdominal section was performed, and then the liver, kidneys, spleen, and heart were sequentially extracted and flash frozen in liquid nitrogen. Each tissue frozen in liquid nitrogen was stored in a deep freezer at -80ºC.

The frozen tissues (spleen: 0.02 g, other tissues: 0.04 g) were each frozen and triturated, and then 1x TKM tissue lysis buffer (50 mM triethanolamine (pH 7.8), 50 mM KCl, 5 mM MgCl₂, 0.25 M sucrose, 1 mM PMSF, protein inhibitors cocktail-EDTA free (Code No. 03969-21, Nacalai Tesque), 1 mM DTT, and 5 µL/mL recombinant RNase inhibitor (40 U/µL, Cat No. 2313A, Lot No. K8402DA, TAKARA Bio)) were added in an amount of 490 µL for the spleen and 980 µL for the others, and dissolved by rotation for 15 minutes (1 rpm, 25ºC). Then, the resultant product was subjected to centrifugation (at 3000 rpm x 15 minutes, 4ºC), and the supernatants (each tissue extract) was collected. The concentration of proteins in each tissue extract was quantified with a spectrophotometer using each tissue extract that had been diluted 20 times using DEPC-treated water.

Each tissue extract collected was subjected to Western blot analysis. An SDS-PAGE gel was prepared using TGX™ FastCast™ Acrylamide Kit, 12% (Bio-Rad). Electrophoresis samples were prepared in a 6x SDS-PAGE sample buffer (62.5 mM Tris-HCl pH 6.8, 2% SDS, 5% 2-mercaptoethanol, 10% glycerol, 0.25% BPB), and placed on a heat block at 95ºC for 5 minutes. The electrophoresis samples prepared were applied at 50 µg/well for detecting GAPDH, and at 100 µg/well for other detection. Electrophoresis was performed at 160 V for 60 minutes (electrophoresis buffer; 195 mM glycine, 25 mM Tris).

After electrophoresis, proteins were transferred to a PVDF membrane (Immobion™-P, Millipore) under conditions of 100 V and 1.5 hours using a tank-type blotting device and a transfer buffer (25 mM Tris-HCl, 195 mM glycine, 0.01% SDS, 15% methanol). The membrane was split into two at the position of a 25 kDa marker. The membrane after transfer was shaken and blocked in 5% skim milk/TBS-T (100 mM Tris-HCl, 150 mM NaCl, 0.1% Tween-20, pH 7.6) at room temperature for 30 minutes. After blocking, a primary antibody reaction was performed in 5% skim milk/TBS-T. As the primary antibody, an anti-p53 antibody (MAB1355, R&D Systems, Inc., diluted 500 times), an anti-MDM2 antibody (sc-965, SantaCruz, diluted 500 times), and an anti-GAPDH antibody (sc-32233, SantaCruz, diluted 20000 times) were used. The membrane was shaken at room temperature for 60 minutes, and then the membrane was washed with TBS-T for 5 minutes. It is noted that washing was performed three times. After the primary antibody reaction, a secondary antibody reaction was performed in 1% skim milk/TBS-T. The membrane was shaken at room temperature for 30 minutes, and then the membrane was washed with TBS-T for 5 minutes. It is noted that washing was performed three times. Furthermore, the membrane was washed with TBS (100 mM Tris-HCl, 150 mM NaCl, pH 7.6) for 10 minutes. Subsequently, the membrane was treated with a chemiluminescence reagent Immobilon™ Western (Millipore), and then chemiluminescence was detected using a lumino image analyzer LAS-3000 (FUJIFILM Corporation). Detected bands were quantified with an image processing software ImageJ (NIH).

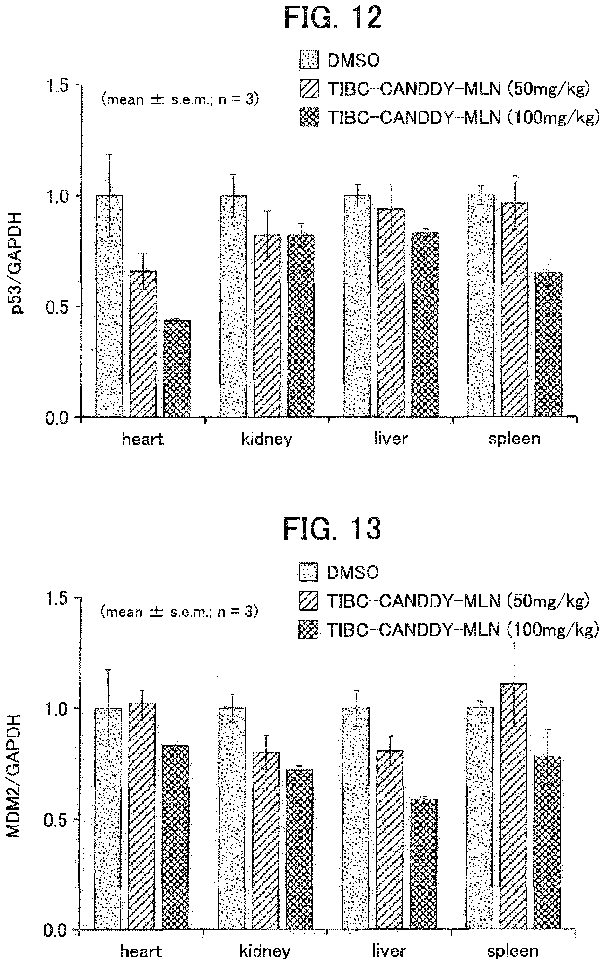

The results of the Western blot analysis are shown in Figs. 12 and 13. As shown in Figs. 12 and 13, when TIBC-CANDDY_MLN was administered to mice, the amounts of the wild-type p53 protein and the MDM2 protein were found to be reduced in a concentration dependent manner in the heart and spleen. Furthermore, in the liver, only when administration was performed a dose of 100 mg/kg body weight, the amounts of the wild-type p53 protein and the MDM2 protein were found to be reduced slightly. On the other hand, in the kidneys, reduction of the target was not found. From the result, it was suggested that the TIBC-CANDDY_MLN showed a higher transfer property to the heart, liver, and spleen as compared with the kidneys.

### <Reference Example 10>

In Reference Example 10, a protein-degradation inducing tag and a specific protein affinity molecule having an affinity with an ecDHFR protein were linked to each other to synthesize TMP-CANDDY_ALLN as a protein-degradation inducing molecule.

As the protein-degradation inducing tag, a compound (CANDDY_ALLN) in which an active site (formyl group) of ALLN as a proteasome inhibitor was substituted with a carboxy group was used. Furthermore, as the specific protein affinity molecule, the above-described TMP-NH₂ was used.

The method of synthesizing TMP-CANDDY_ALLN is described in detail as the following synthesis scheme.

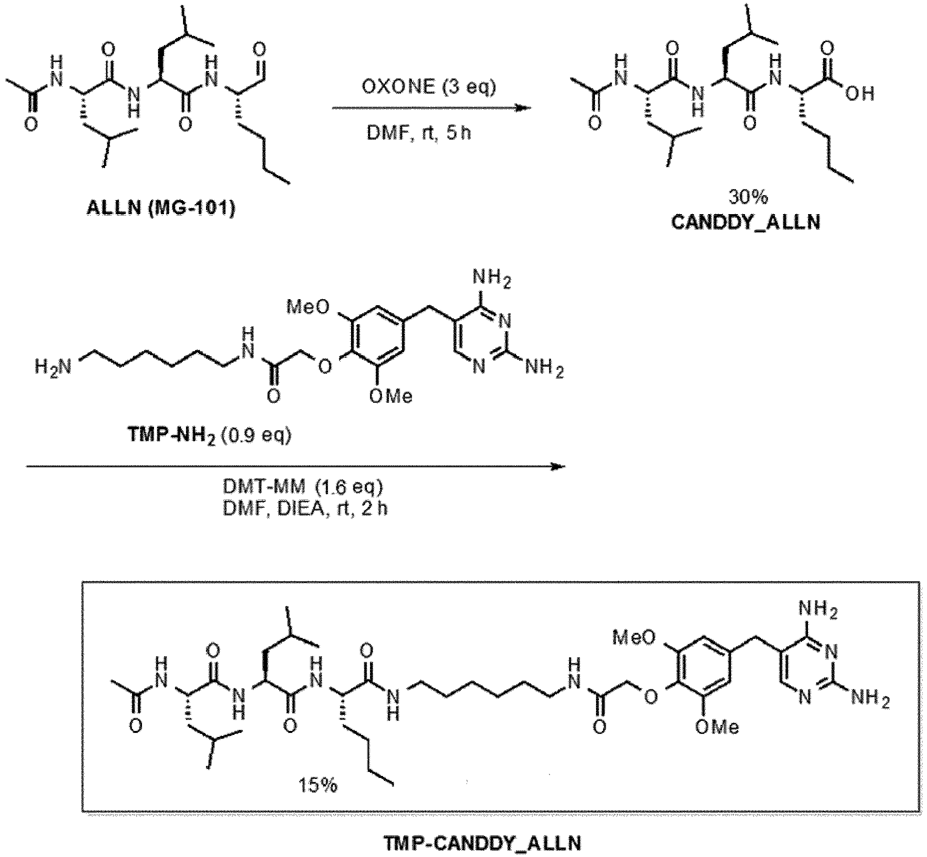

### (Synthesis of CANDDY_ALLN)

ALLN (87.2 mg, 0.23 mmol, 1 eq, Code No. 07036-24, Nacalai Tesque, Inc.) was charged into an eggplant flask, and 2 mL of dehydrate DMF was added. After the resultant product was stirred at room temperature for 5 minutes, Oxone (212.1 mg, 0.69 mmol, 3 eq, Code No. 228036, Sigma-Aldrich) was directly added to a reaction solution, and the reaction solution was stirred at room temperature for 5 hours. The reaction solution was diluted with water, and extracted with chloroform three times. After being dried over anhydrous sodium sulfate, the solvent was evaporated under reduced pressure. Separation and purification treatment was performed by silica gel chromatography (Code No. 30511-35, Nacalai Tesque, Inc.) (chloroform/methanol = 20/1 to 10/1, gradient) to obtain CANDDY_ALLN (27.0 mg, 0.068 mmol, 30%).

### (Synthesis of TMP-CANDDY_ALLN)

CANDDY_ALLN (26.8 mg, 0.067 mmol, 1 eq) and separately synthesized TMP-NH₂ (Long, M.J. et al., Chem. Biol., 2012, 19(5), 629-637) (26.0 mg, 0.060 mmol, 0.9 eq) were charged into an eggplant flask, and 2 mL of dehydrate DMF was added. After the resultant solution was stirred at room temperature for 5 minutes, 0.1 mL of DIPEA was added to neutralize the solution. The obtained product was stirred for 5 minutes at room temperature, then DMT-MM (30.0 mg, 0.11 mmol, 1.6 eq, Code No. 329-53751, Wako Pure Chemical Industries, Ltd.) was directly added to a reaction solution, and stirred at room temperature for 2 hours. Under cooling conditions, 10 mL of 10 mass% brine/0.1 N aqueous hydrochloric acid was added, and extracted with ethyl acetate three times. This was washed with 0.5 N aqueous hydrochloric acid and then brine, and then dried over anhydrous sodium sulfate. After evaporating the solvent under reduced pressure, separation and purification treatment was performed by silica gel chromatography (Code No. 30511-35, Nacalai Tesque, Inc.) (chloroform/methanol = 10/1) to obtain TMP-CANDDY_ALLN (8.2 mg, 0.010 mmol, 15%, isolated yield).

### <Reference Example 11>

In Reference Example 11, as in Reference Example 4, a proteasome inhibitory activity of TMP-CANDDY_ALLN and an affinity of TMP-CANDDY_ALLN with a proteasome were evaluated.

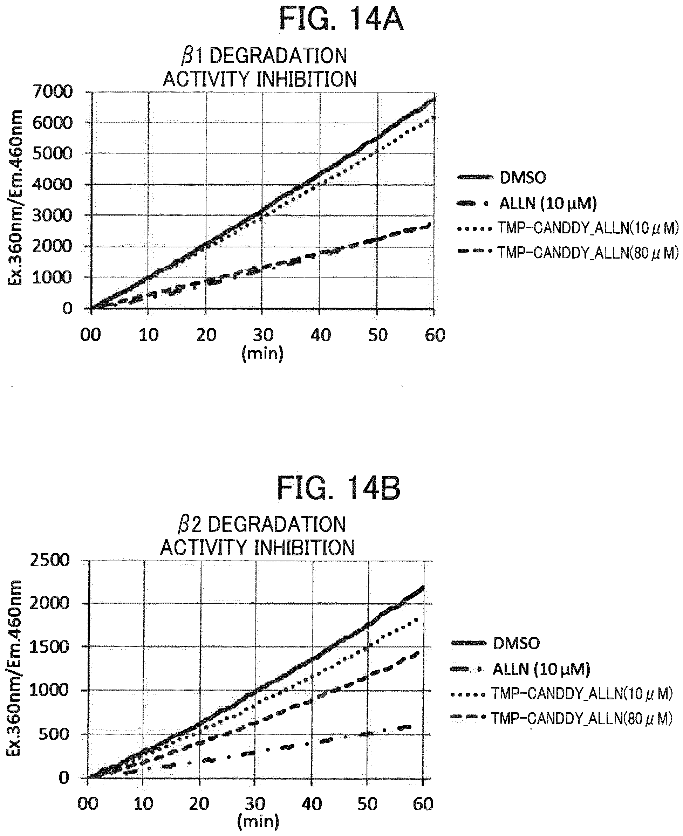

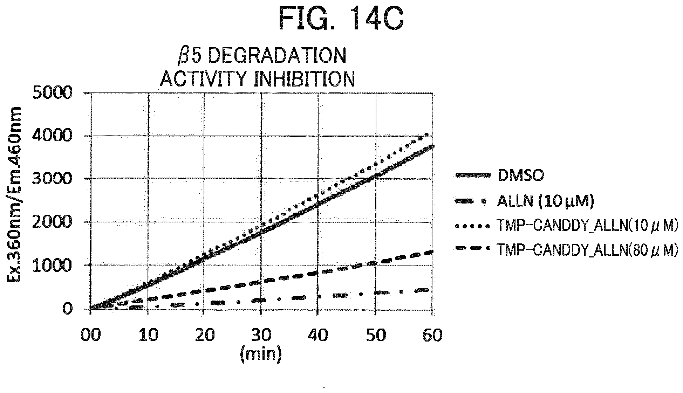

Figs. 14A to 14C show the proteasome activities against β1 (caspase-like activity), β2 (trypsin-like activity), and β5 (chymotrypsin-like activity), respectively. As can be seen in Figs. 14A to 14C, it was demonstrated that with respect to the activities of β2 and β5, in TMP-CANDDY_ALLN, as compared with single use of ALLN, the inhibitory activity was weakened, and the inhibitory activity of ALLN was inactivated. It was reported that β1 was not inhibited by ALLN (Kaiser, M. et al., Chem. Bio. Chem., 2004, 5, 1256-1266). The result was consistent with this report. Further, the inhibitory activity of TMP-CANDDY_ALLN was found to be increased against any of β1, β2, and β5 in a concentration dependent manner, indicating that TMP-CANDDY_ALLN had an affinity with a proteasome.

### <Reference Example 12>

In Reference Example 12, degradation (knockdown) of a forcibly expressed ecDHFR protein in HeLa cells through TMP-CANDDY_ALLN was evaluated by FACS analysis.

### (Preparation of plasmid)

A plasmid (pMIR-DsRed-IRES-ecDHFR-HA-GFP) expressing the ecDHFR protein was prepared, as in Reference Example 5.

### (Introduction of plasmid into HeLa cells and cell seeding)

As in Reference Example 5, the plasmid was introduced into HeLa cells to transiently overexpress an ecDHFR protein or a DsRed protein for comparison in the cells. HeLa cells into which the plasmid had been introduced were seeded in a 24-well plate at a cell density of 4 x 10⁴ cells/well, and then cultured under conditions of 37ºC and 5 vol% CO₂ for 40 hours.

### (Addition of TMP-CANDDY_ALLN to HeLa cells)

Culture was performed for 40 hours after introduction of the plasmid, and then TMP-CANDDY_ALLN was added to HeLa cells as follows. As a medium, a serum-free medium (37ºC) in which 1 mass% L-glutamine solution (Sigma-Aldrich) was added to D-MEM (high D-glucose, phenol red, sodium pyruvate (Wako Pure Chemical Industries, Ltd.)) was used, and added to each well at 300 µL/well. It is noted that the L-glutamine solution was added immediately before use. A DMSO solution containing TMP-CANDDY_ALLN was added to each well at 3 µL/well, and cultured under conditions of 37ºC and 5 vol% CO₂. As a control, a TMP-containing DMSO solution or DMSO was used.

### (Evaluation of degradation of protein (knockdown) of ecDHFR protein through TMP-CANDDY_ALLN (FACS analysis))

As in Reference Example 1, degradation of the ecDHFR protein through TMP-CANDDY_ALLN was evaluated by FACS analysis.

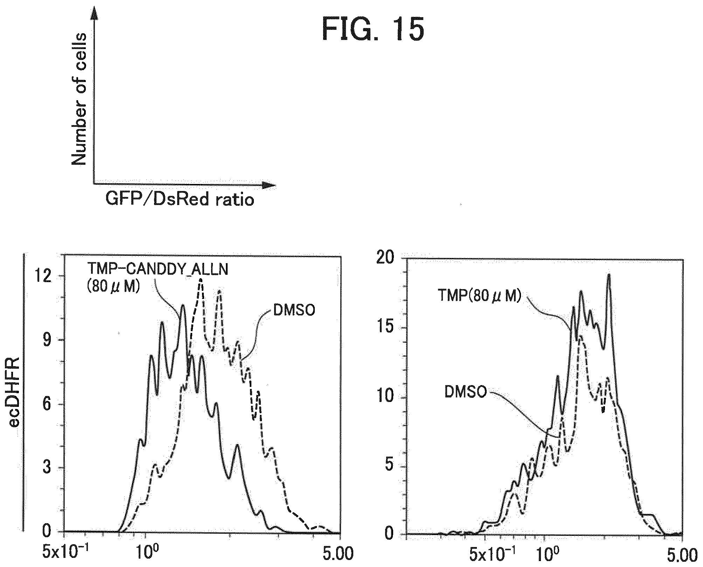

The results of the FACS analysis are shown in Fig. 15. As shown in Fig. 15, when TMP-CANDDY_ALLN was added, a graph is largely shifted toward the left as compared with the case where the control (DMSO) was added, demonstrating that degradation of the ecDHFR protein was induced by TMP-CANDDY_ALLN. On the other hand, when TMP was added, the graph is overlapped to that of the control (DMSO), demonstrating that the ecDHFR protein was not degraded.

The disclosure of Japanese Patent Application No. 2016-222682 filed on November 15, 2016 is entirely incorporated herein by reference. All documents, patent applications, and technical standards cited herein are incorporated herein by reference to the same extent as if each of the documents, patent applications, and technical standards was specifically and individually indicated to be incorporated by reference.

## Claims

1. A molecular kinetics evaluation method comprising:
a step of administering a protein-degradation inducing molecule to a human or a non-human animal, the protein-degradation inducing molecule being a conjugate of a protein-degradation inducing tag as a molecule which has an affinity with a protease and does not inhibit degradation of a protein by the protease and a specific protein affinity molecule which has an affinity with a specific protein, and inducing degradation of the specific protein in a living body of the human or the non-human animal; and
a step of evaluating molecular kinetics of the specific protein affinity molecule or the protein-degradation inducing molecule by detecting degradation of the specific protein in a specimen being at least a portion of the human or the non-human animal.

2. The molecular kinetics evaluation method according to claim 1, wherein in the step of inducing degradation of the specific protein, the degradation of the specific protein is induced in a ubiquitin-independent manner.

3. The molecular kinetics evaluation method according to claim 1 or 2, wherein the protein-degradation inducing tag has a structure where a protease inhibitory activity of a protease inhibitor is inactivated.

4. The molecular kinetics evaluation method according to any one of claims 1 to 3, wherein the protease is proteasome.

5. The molecular kinetics evaluation method according to claim 4, wherein the protein-degradation inducing tag has a structure where a proteasome inhibitory activity of a proteasome inhibitor is inactivated.

6. The molecular kinetics evaluation method according to claim 5, wherein the proteasome inhibitory activity is an inhibitory activity against at least one selected from a caspase-like activity, a trypsin-like activity, and a chymotrypsin-like activity.

7. The molecular kinetics evaluation method according to any one of claims 1 to 6, wherein the protein-degradation inducing molecule is a drug candidate molecule, the method further comprising a step of evaluating a pharmacological action by inducing the degradation of the specific protein in a living body of the human or the non-human animal.

8. The molecular kinetics evaluation method according to any one of claims 1 to 7, wherein the step of inducing degradation of the specific protein includes administering the protein-degradation inducing molecule to a non-human animal, and inducing degradation of the specific protein in a living body of the non-human animal, and the step of evaluating molecular kinetics of the specific protein affinity molecule or the protein-degradation inducing molecule includes detecting degradation of the specific protein in a specimen being at least a portion of the non-human animal.

9. The molecular kinetics evaluation method according to any one of claims 1 to 8, wherein the molecular kinetics is specificity to a tissue, an organ, a cell, or a molecule.

10. A screening method comprising:
a step of administering a protein-degradation inducing molecule to a human or a non-human animal, the protein-degradation inducing molecule being a conjugate of a protein-degradation inducing tag that is a molecule which has an affinity with a protease and does not inhibit degradation of a protein by the protease, and a specific protein affinity molecule which has an affinity with a specific protein, and inducing degradation of the specific protein in a living body of the human or the non-human animal; and
a step of selecting the specific protein affinity molecule or the protein-degradation inducing molecule showing specific molecular kinetics by detecting degradation of the specific protein in a specimen being at least a portion of the human or the non-human animal.

11. The screening method according to claim 10, wherein in the step of inducing degradation of the specific protein, the degradation of the specific protein is induced in a ubiquitin-independent manner.

12. The screening method according to claim 10 or 11, wherein the step of inducing degradation of the specific protein includes administering the protein-degradation inducing molecule to a non-human animal, and inducing degradation of the specific protein in a living body of the non-human animal, and the step of selecting the specific protein affinity molecule or the protein-degradation inducing molecule includes detecting degradation of the specific protein in a specimen being at least a portion of the non-human animal.

13. The screening method according to any one of claims 10 to 12, wherein the molecular kinetics is specificity to a tissue, an organ, a cell, or a molecule.
